# EUROPEAN PATENT APPLICATION

(11) **EP 4 717 704 A1**
(43) Date of publication of application: **01.04.2026**
(21) Application number: 24203009.6
(22) Date of filing: 26.09.2024
(51) Int. Cl.: C07K 14/31, C07K 1/22

(54) **NOVEL PROTEIN A LIGAND THAT ENABLES MILDER ELUTION**

(71) Applicant: Cytiva BioProcess R&D AB, 751 84 Uppsala (SE)
(72) Inventor: ANDER, Mats, 75184 Uppsala (SE); JONSSON, Andreas, 75184 Uppsala (SE)
(74) Representative: Démoulin, Eva Lotta

(57) **Abstract**

The present disclosure relates to a class of polypeptides derived from a Staphylococcus Protein A (SpA) or a domain thereof that enables mild elution, such as elution within a pH range of pH 4.4-6.0, for the efficient isolation of immunoglobulins. Methods for isolating immunoglobulins using mild elution as well as related products, such as separation matrices coupled to the herein disclosed polypeptides or the multimers thereof, are also disclosed.

## Description

### TECHNICAL FIELD

The present disclosure relates to a class of polypeptides derived from a Staphylococcus Protein A (SpA) or a domain thereof that enables mild elution, such as elution within a pH range of pH 4.4-6.0, for the efficient isolation of immunoglobulins. Methods for isolating immunoglobulins using mild elution as well as related products, such as separation matrices coupled to the herein disclosed polypeptides or the multimers thereof, are also disclosed.

### BACKGROUND

Immunoglobulins represent the most prevalent biopharmaceutical products in either manufacture or development worldwide. The high commercial demand for and hence value of this particular therapeutic market has led to the emphasis being placed on pharmaceutical companies to maximize the productivity of their respective monoclonal antibodies (mAb) manufacturing processes whilst controlling the associated costs. Antibody therapies have been extremely successful, with more than 100 approved therapies, and many of the antibody therapies are on the top 10 selling drug list.

Affinity chromatography is used in most cases as one of the key steps in the purification of immunoglobulin molecules, such as monoclonal or polyclonal antibodies. A particularly interesting class of affinity reagents is proteins capable of specific binding to invariable parts of an immunoglobulin molecule, such interaction being independent of the antigen-binding specificity of the antibody. Such reagents can be widely used for affinity chromatography recovery of immunoglobulins from different samples, such as but not limited to serum or plasma preparations or cell culture derived feed stocks. An example of such a protein is staphylococcal protein A (SpA), containing domains capable of binding to the Fc (fragment crystallizable) and Fab (fragment antigen-binding) portions of IgG immunoglobulins from different species. These domains are commonly denoted as the E-, D-, A-, B- and C domains.

SpA-based proteins have due to their high affinity and selectivity found a widespread use in the field of biotechnology, e.g. as ligands in affinity chromatography for capture and purification of antibodies, as well as for detection or quantification. At present, SpA-based affinity medium is probably the most widely used affinity medium for isolation of monoclonal antibodies and their fragments from different samples, including industrial cell culture supernatants. Accordingly, various matrices comprising protein A or protein A-derived ligands are commercially available, for example, in the form of MabSelect^{™} SuRe, MabSelect^{™} SuRe LX, MabSelect^{™} PrismA and HiScreen Fibro^{™} PrismA from Cytiva^{™}, Uppsala, Sweden.

In fact, SpA chromatography is the most well-established method for capture of antibodies from Chinese hamster ovary cell (CHO) supernatant. The typical antibody downstream purification process starts with clarification of CHO feed with depth filters, followed by loading the feed at neutral pH onto a Protein A chromatography resin. The protein is then eluted by lowering pH to 3-4. For example, MabSelect^{™} PrismA has acetate pH 3.5 as recommended elution buffer. After elution, the antibody is typically transferred to a hold tank for virus inactivation at pH < 4 for an hour. Some antibodies are not sensitive to low pH, and some standard antibodies are continued through the process after low pH viral inactivation with only 1-5 % aggregation which can be removed in subsequent process steps. However, other types of antibodies and antibody-inspired versions of proteins, for example symmetric bispecific antibodies with VHH (the antigen binding fragment of the heavy chain) or scFv (single-chain variable fragment) fragments attached to different domains, Fc fusions with other proteins or antibody fusion proteins, are sensitive to low pH and tend to aggregate under low pH conditions (Krishnamurthy R., Manning M.C., The stability factor: importance in formulation development. Curr Pharm Biotechnol, Dec 3(4), 361-371, 2002). The main disadvantages with the sensitivity to low pH are that these molecules typically show lower solubility and stability and have challenging impurity profiles with high aggregate and fragment levels. One example of a symmetric bispecific antibody with inherited instability and aggregation issues is the Morrison format, developed in the Morrison laboratory at UCLA, 1997 (Coloma MJ., Morrison SL., Design and production of novel tetravalent bispecific antibodies. Nat Biotechnol, 15:159-163, 1997). Indeed, about 50% of the pharmaceutically relevant monoclonal antibodies and a Fc fusion proteins were found to have aggregation issues following SpA-based purification (Shukla A., Hubbard B., Tressel T., Guhan S., Low D., Downstream processing of monoclonal antibodies-application of platform approaches. J Chromatogr B Analyt Technol Biomed Life Sci, Mar 15;848(1):28-39, 2007).

Various ideas have been tested to circumvent the above described acid-induced aggregation of antibodies and antibody inspired versions of proteins, to maximize the productivity of immunoglobulin manufacturing processes. These include for example the elution of immunoglobulin into a neutralizing buffer. However, this method is generally difficult to scale up and even though the approach is thought to reduce the aggregate content, it still remains at 15-20% (Shukla et al., 2007). This leads to the conclusion that the product tends to aggregate significantly under low pH conditions even in the short time it takes to exit the column. Another option is the addition of different excipients to the elution buffer to reduce acid induced aggregation. Commonly used excipients are amino acids, such as Histidine or Arginine, high salt concentration, such as 0.5-1 M NaCl or 1-2 M Urea, or sugars such as glucose or mannitol. While this approach might lead to reduced aggregation (Liu, B., Guo H., Xu,J., Qin, T., Xu, L., Zhang, J., Guo, Q., Zhang D., Qian, W., Li, B., Dai, J., Hou, S., Guo, Y., Wang H., Acid-induced aggregation propensity of nivolumab is dependent on the Fc, MABS, Vol. 8, No. 6, 1107-1117, 2016 and Shukla et al., 2007), the addition of excipients is complicated, expensive and time consuming. Furthermore, the excipient must be cleared in subsequent chromatography steps. Taken together, acid-induced aggregation of immunoglobulins and other immunoglobulin-inspired target molecules hampers and hinders the development of immunoglobulin-based therapeutics. Current methods for preventing aggregation are limited and associated with certain drawbacks that impede maximized productivity and efficient manufacturing processes.

### SUMMARY

It is an object of the present disclosure to provide SpA-derived ligands that allow for efficient isolation of target molecules while alleviating, at least in part, the abovementioned and other drawbacks of the prior art. The target molecules include immunoglobulins, such as immunoglobulin G (IgG), and fragments thereof that comprise the Fc (fragment crystallizable) region of the given immunoglobulin. Target molecules may for example be antibodies of IgG isotype. As it will be appreciated, the target molecules also include any polypeptide construct, such as a fusion protein, which comprises at least the Fc region and has maintained its binding property to the SpA-derived ligand.

It is an object of the present disclosure to provide SpA-derived ligands which allow for preventing or decreasing acid-induced aggregation of the target molecules during isolation.

It is also an object of the present disclosure to provide SpA derived ligands with optimal binding affinity to the target molecules.

It is another object of the present disclosure to provide SpA-derived ligands that are alkaline stable.

Another object of the present disclosure is to provide SpA-derived ligands that are optimized for industrial production in recombinant systems.

These and other objects which are evident to the skilled person from the present disclosure are met by different aspects of the invention as defined in the appended claims and as generally disclosed herein. Accordingly, the present disclosure provides SpA-derived ligands that enable mild elution for efficient isolation of the target molecules, such as immunoglobulins, thereby preventing or decreasing acid-induced aggregation of the target molecules during isolation. Polypeptides and polypeptide multimers of the present disclosure, as it will now be described in more detail, exhibit improved dissociation properties for the release of bound target molecules at an increased pH in comparison to the pH at which known polypeptides (and multimers thereof) operate. Advantageously, these improved dissociation properties can be obtained together with favourable stability and capacity properties.

Thus, in a first aspect of the disclosure, there is provided a polypeptide derived from an SpA or any domain thereof, wherein said polypeptide has binding affinity to an Fc region of an immunoglobulin at pH 7.0, which binding affinity for said Fc region is essentially lost at and below pH 4.4, or at and below pH 4.5, or at and below pH 4.6, or at and below pH 4.7, or at and below pH 4.8, or at and below pH 4.9, or at and below pH 5.0, or at and below pH 5.5, or at and below pH 6.0. In some embodiments, the polypeptide has a higher binding affinity than SEQ ID NO:10 for adalimumab. In some embodiments, the polypeptide has lower binding affinity for a VH3 region of trastuzumab compared to the binding affinity of SEQ ID NO:6 and/or SEQ ID NO:7 for the same VH3 region.

Without wishing to be bound by any particular theory, the inventors have surprisingly found that the beneficial loss of the binding affinity for said Fc region at slightly higher, but still weakly acidic pH values may be induced by a modified electrostatic repulsion, which may be mediated by at least one amino acid selected from the group consisting of amino acids in positions in the amino acid sequence of the polypeptide corresponding to positions X₁₈, X₂₄ and X₂₈ in SEQ ID NO:7.

In a second aspect of the disclosure, there is thus provided a polypeptide derived from an SpA or any domain thereof comprising an amino acid sequence, wherein said polypeptide comprises an amino acid selected from R and K in a position in the amino acid sequence thereof, which position corresponds to position X₁₈ in SEQ ID NO:7; an amino acid selected from H, D, Q and E, such as from H and D, in a position in said amino acid sequence, which position corresponds to position X₂₄ in SEQ ID NO:7; and an amino acid selected from H, T, K, S and G, such as from H, K and G, such as from H and G, in a position in said amino acid sequence, which position corresponds to position X₂₈ in SEQ ID NO:7.

Moreover, in a third aspect, there is provided a polypeptide comprising an amino acid sequence comprising Sequence A, which Sequence A consists of an amino acid sequence selected from i), ii) and iii), wherein i), ii) and iii) are defined as follows:
i) X₁₈LPNLTX₂₄EQRX₂₈ (SEQ ID NO:12);
ii) an amino acid sequence which has at least 70% identity to a sequence defined by i);
iii) an amino acid sequence which has at least 70% identity to any sequence selected from the group consisting of: residues 18-28 in SEQ ID NO:6, residues 18-28 in SEQ ID NO:9, residues 18-28 in SEQ ID NO:8, residues 18-28 in SEQ ID NO:1, residues 18-28 in SEQ ID NO:2, residues 18-28 in SEQ ID NO:3, residues 21-31 in SEQ ID NO:4 and residues 16-26 in SEQ ID NO:5;
wherein additionally, in each of i), ii) and iii), independently from each other,
X₁₈ is selected from R and K,
X₂₄ is selected from H, D, Q and E, such as from H and D, and
X₂₈ is selected from H, T, K, S and G, such as from H, K and G, such as
from H and G.

In some embodiments of the first, second and third aspects of the disclosure, the polypeptide comprises an amino acid selected from T, S, G, Q, A, E, H, R, P, D, K and N, such as from S, A and E, in a position in the amino acid sequence thereof, which position corresponds to position X₃₃ in SEQ ID NO:7; an amino acid selected from V, E, G, R, D, K, Q, N, H and S, such as from E, G, R, D, K, Q, N, H and S, such as from G, R, E and D, in a position in the amino acid sequence thereof, which position corresponds to position X₄₀ in SEQ ID NO:7; and an amino acid selected from L, V, S, I, R and G, such as from V, L, I and R, in a position in the amino acid sequence thereof, which position corresponds to position X₅₁ in SEQ ID NO:7.

In some embodiments, of the first, second and third aspects of the disclosure, the polypeptide comprises an amino acid selected from E, N, H, R and S, such as from N, E, H and R, in a position in the amino acid sequence thereof, which position corresponds to position X₁₁ in SEQ ID NO:7; and/or an amino acid selected from A and Q in a position in the amino acid sequence thereof, which position corresponds to position X₉ in SEQ ID NO:7; preferably with the proviso that the amino acids in the positions corresponding to X₉X₁₁ in SEQ ID NO:7 are not AE.

In a fourth aspect, a polypeptide multimer is provided, wherein each monomer of the multimer comprises a polypeptide which is independently selected from any polypeptide defined in any one of the embodiments of the first, second and third aspects of the disclosure.

In a fifth aspect, there is provided a polynucleotide encoding the polypeptide as defined in any one of the embodiments of the first, second and third aspects of the disclosure, or the polypeptide multimer as defined in any one of the embodiments of the fourth aspect of the disclosure.

In a sixth aspect, there is provided an expression vector comprising the polynucleotide as defined in the fifth aspect of the disclosure.

In a seventh aspect, there is provided a host cell comprising the expression vector as defined in the sixth aspect of the disclosure.

In an eighth aspect, there is provided an adsorbent material comprising the polypeptide as defined in any one of the embodiments of the first, second and third aspects of the disclosure, or the polypeptide multimer as defined in any one of the embodiments of the fourth aspect of the disclosure, coupled to a solid support. In some embodiments, the solid support is a separation matrix.

In an ninth aspect, there is provided a method of isolating an immunoglobulin or a fragment thereof comprising a) contacting a liquid sample comprising said immunoglobulin or a fragment thereof with the adsorbent material as defined in any one of the embodiments of the eighth aspect of the present disclosure.

### DETAILED DESCRIPTION

Aspects of the present disclosure will be described more fully hereinafter with reference to the accompanying drawings and Examples. The terminology used herein is for the purpose of describing particular aspects of the disclosure only, and is not intended to limit the disclosure.

As used herein, the singular forms "a", "an" and "the" are intended to include the plural forms as well, unless the context clearly indicates otherwise.

For the sake of clarity, in embodiments, wherein several alternatives are listed, the relation between these alternatives (such as "and/or", "or" and "and") is indicated only once, but should be understood between each alternative (for example A, B, C and/or D is to be understood as A and/or B and/or C and/or D).

The term "% identity", as used throughout the disclosure, may for example be calculated as follows. The query sequence is aligned to the target sequence using the CLUSTAL W algorithm (Thompson et al., Nucleic Acids Research, 22: 4673-4680 (1994)). A comparison is made over the window corresponding to the shortest of the aligned sequences. The shortest of the aligned sequences may in some instances be the target sequence. In other instances, the query sequence may constitute the shortest of the aligned sequences. The amino acid residues at each position are compared and the percentage of positions in the query sequence that have identical correspondences in the target sequence is reported as % identity.

Herein, "Xₙ" and "Xₘ" are used to indicate amino acid residues in positions n and m corresponding to positions n and m in e.g. SEQ ID NO:7 as defined herein, wherein n and m are integers which indicate the position of an amino acid residue within said sequence as counted from the N-terminal end of said sequence. For example, X₂₄ and X₁₃ indicate the amino acid residue in positions that corresponds to positions twenty-four and thirteen, respectively, from the N-terminal end of e.g. SEQ ID NO:3 as defined herein, in an alignment. To clarify, the X numbering as used herein is based on the full-length SpA scaffold comprising 58 amino acid residues. Hence X₂₄ is to be understood as being the 24th amino acid residue in the 58-mer. The skilled person is able to make alignments of sequences to determine the position of an amino acid in accordance with the above.

It has been shown that the highly conserved Histidine residues H₄₃₃, H₄₃₅ and H₃₁₀ (according to Kabat numbering scheme, 1969) on SpA target molecules, such as immunoglobulins, face positive amino acid residues, Lysine, Arginine, and Histidine in known SpA-derived molecules. Some of the most important positively charged amino acids in known SpA-derived molecules are thought to be Histidine in X₁₈, Arginine in X₂₇ and Lysine in X₃₅ (corresponding to positions X₁₈, X₂₇ and X₃₅ in SEQ ID NO:7). These residues take on a positive charge at low pH, thus repelling each other and weakening the hydrophobic association between the SpA-derived molecule and its target. As a result, operating conditions for SpA-based columns typically require the use of low pH conditions, typically between pH 3-4 for column elution (Huang, Bo., Liu F., Dong X., Sun Y., Molecular mechanism of the effects of salt and pH on the affinity between protein A and human immunoglobulin G1 revealed by molecular simulations, J Phys Chem B, Jan 12;116(1): 424-33, 2012). As discussed above, however, such pH range is associated with acid-induced aggregation of the target molecules during isolation. The present disclosure provides SpA-derived ligands that enable mild elution for efficient isolation of the target molecules, such as immunoglobulins, thereby preventing or decreasing acid-induced aggregation during isolation. Polypeptides and polypeptide multimers of the present disclosure, as it will now be described in more detail, exhibit improved dissociation properties for the release of bound target molecules at an increased pH in comparison to the pH at which, known polypeptides (and multimers thereof) operate. Without wishing to be bound by any particular theory, the inventors have surprisingly found that mild elution, or in other words, the loss of the binding affinity for the target molecule at a mild pH, may be induced by a modified electrostatic repulsion, which may be mediated by at least one amino acid selected from the group consisting of amino acids in positions in the amino acid sequence of the polypeptide corresponding to positions X₁₈, X₂₄ and X₂₈ in SEQ ID NO:7. In some embodiments discussed below, the electrostatic repulsion is mediated by one amino acid in the position in the amino acid sequence of the polypeptide corresponding to position X₁₈ SEQ ID NO:7; by two amino acids in the positions in the amino acid sequence of the polypeptide corresponding to positions X₁₈ and X₂₄ SEQ ID NO:7; or by all three amino acids in positions in the amino acid sequence of the polypeptide corresponding to positions X₁₈, X₂₄ and X₂₈ in SEQ ID NO:7. As it will be discussed in further details, certain amino acid residues in these positions are particularly beneficial in the context of the present disclosure. The inventors envision that one or more of these amino acid residues in these positions of the amino acid sequence of the herein disclosed polypeptides and multimers thereof create a stronger net repulsion due to more negatively charged groups. This allows for mild elution, or in other words, the loss of the binding affinity for the target molecule already at a mild pH, thereby preventing or decreasing acid-induced aggregation during isolation.

The target molecules include immunoglobulins, such as IgG, and fragments thereof that comprise the Fc region of the given immunoglobulin. The target may be a human or a humanized protein. The target may be antibodies of IgG isotype or derivatives thereof. When the target molecule is IgG, it may for example be IgG1, IgG2 and/or IgG4. Polypeptides and polypeptide multimers of the present disclosure are also beneficial for the isolation of further target molecules (such as immunoglobulin/antibody inspired proteins and other derivatives). As it will be appreciated, the target molecules also include any polypeptide construct, such as a fusion protein, which comprises at least the Fc region the given immunoglobulin and has maintained the binding property thereof to polypeptides and polypeptide multimers of the present disclosure. For example, immunoglobulin/antibody inspired proteins and other derivatives are symmetric bispecific antibodies with VHH or scFv fragments attached to different domains, Fc fusions with other proteins or antibody fusion proteins, which are often known to be sensitive to low pH and tend to aggregate under low pH conditions.

The term "mild elution" as used herein refers to an elution at and below pH 4.4. In some embodiments, the term refers to an elution at and below pH 4.5, or at and below pH 4.6, or at and below pH 4.7, or at and below pH 4.8, or at and below pH 4.9, or at and below pH 5.0, or at and below pH 5.5, or at and below pH 6.0. It should be understood that the target molecule is eluted at said pH in a yield of at least 80%, such as at least 85%, such as at least 90%, such as at least 95% from a column that comprises resin coupled to a polypeptide or a multimer thereof according to the present disclosure. This results in gentler elution conditions which minimize the risk for aggregation or inactivation of the target molecule.

The term "mild elution" relates to the term "essentially lost binding affinity" in the context of the present disclosure. In particular, essential loss of the binding affinity for the Fc region refers to that the polypeptide or the polypeptide multimer has substantially reduced or abolished binding to its target molecule at the recited pH or pH range. This corresponds to "essentially no binding affinity" for the target molecule at the given pH or pH range, which may be measured in correspondence with the binding affinity between the polypeptide or the polypeptide multimer and the same target molecule at neutral pH, such as at pH 7. This may be measured using the techniques described below for assessing binding affinity, such as surface plasmon resonance (SPR), and using an immunoglobulin, such as IgG, such as IgG selected from the group consisting of IgG1, IgG2 and IgG4, as analyte. For assessing essentially lost binding affinity, the analyte may be IgG1, such as trastuzumab and/or adalimumab, as demonstrated in the appended Examples.

In light of the above, in a first aspect of the disclosure, there is thus provided a polypeptide derived from a SpA or any domain thereof, wherein said polypeptide has binding affinity to an Fc region of an immunoglobulin at pH 7.0, which binding affinity for said Fc region is essentially lost at and below pH 4.4. In some embodiments, the binding affinity for said Fc region is essentially lost at and below pH 4.4, or at and below pH 4.5, or at and below pH 4.6, or at and below pH 4.7, or at and below pH 4.8, or at and below pH 4.9, or at and below pH 5.0, or at and below pH 5.5, or at and below pH 6.0. In some embodiments, the binding affinity for said Fc region is essentially lost at and below pH 4.5, or at and below pH 4.6, or at and below pH 4.7, or at and below pH 4.8, or at and below pH 4.9, or at and below pH 5.0, or at and below pH 5.5, or at and below pH 6.0. Said binding affinity, in some embodiments, is essentially lost at and below pH 4.6, or at and below pH 4.7, or at and below pH 4.8, or at and below pH 4.9, or at and below pH 5.0, or at and below pH 5.5, or at and below pH 6.0. In other embodiments, said binding affinity is essentially lost at and below pH 4.7, or at and below pH 4.8, or at and below pH 4.9, or at and below pH 5.0, or at and below pH 5.5, or at and below pH 6.0. In some embodiments, said binding affinity is essentially lost at and below pH 4.8, or at and below pH 4.9, or at and below pH 5.0, or at and below pH 5.5, or at and below pH 6.0. In some embodiments, the binding affinity is essentially lost at and below pH 4.9, or at and below pH 5.0, or at and below pH 5.5, or at and below pH 6.0. In some embodiments, the binding affinity is essentially lost at and below pH 5.0, or at and below pH 5.5, or at and below pH 6.0. In some embodiments, the binding affinity is essentially lost at and below pH 5.5, or at and below pH 6.0. In some embodiments, the binding affinity is essentially lost at and below pH 6.0. In particular embodiments, the binding affinity is essentially lost at and below pH 5.0, or at and below pH 5.5. In one embodiment, the binding affinity is essentially lost at and below pH 4.5. In one embodiment, the binding affinity is essentially lost at and below pH 4.6. In one embodiment, the binding affinity is essentially lost at and below pH 4.7. In one embodiment, the binding affinity is essentially lost at and below pH 4.8. In one embodiment, the binding affinity is essentially lost at and below pH 4.9. In one embodiment, the binding affinity is essentially lost at and below pH 5.0. In one embodiment, the binding affinity is essentially lost at and below pH 5.5. In one embodiment, the binding affinity is essentially lost at and below pH 5.6. In one embodiment, the binding affinity is essentially lost at and below pH 5.8. In particular embodiments, the binding affinity for the Fc region is essentially lost at and below a pH range of pH 4.4-6.0, such as a pH range of pH 4.5-6.0, such as a pH range of pH 4.7-6.0, such as a pH range of pH 4.8-6.0, such as a pH range of pH 4.9-6.0, such as a pH range of pH 5.0-6.0, such as a pH range of pH 5.1-6.0, such as a pH range of pH 5.2-6.0, such as a pH range of pH 5.3-6.0, such as a pH range of pH 5.4-6.0, such as a pH range of pH 5.5-6.0, such as a pH range of pH 5.6-6.0, such as a pH range of pH 5.7-6.0, such as a pH range of pH 5.8-6.0, such as a pH range of pH 5.9-6.0. In particular embodiments, the binding affinity for the Fc region is essentially lost at and below a pH range of pH 4.4-6.0, such as a pH range of pH 4.4-5.9, such as a pH range of pH 4.4-5.8, such as a pH range of pH 4.4-5.7, such as a pH range of pH 4.4-5.6, such as a pH range of pH 4.4-5.5, such as a pH range of pH 4.4-5.4, such as a pH range of pH 4.4-5.3, such as a pH range of pH 4.4-5.2, such as a pH range of pH 4.4-5.1, such as a pH range of pH 4.5-5.0, such as a pH range of pH 4.6-4.9, such as a pH range of pH 4.7-4.9. The inventor in the appended Examples demonstrate the multimerization state of the polypeptide may affect the pH at which the binding affinity is lost. Thus, the above discussed pH values may be understood for the polypeptide in a monomeric form, i.e. the polypeptide may be a polypeptide monomer. As demonstrated in the Examples and as further discussed in the fourth aspect, the above discussed pH values may, in some instances, be understood for the polypeptide in a multimeric form, e.g. for the polypeptide multimer of the fourth aspect. Moreover, it is to be understood that if a comparison is made herein between a polypeptide of the present disclosure and any reference (e.g. known) polypeptide, the compared polypeptides share the same multimerization state. Such comparison may for example include elution pH, binding characteristics to target and/or non-target molecules and alkali stability. In some embodiments, the pH (or the maximum of the pH range) at which said binding affinity for said Fc region is essentially lost is higher than the pH (or the maximum of the pH range) at which a binding affinity of a reference polypeptide for the same Fc region is essentially lost. Such reference polypeptide may be selected from the group consisting of SEQ ID NO:6, SEQ ID NO:7 and SEQ ID NO:8. As demonstrated in e.g. Examples 1 and 2, the pH at which the binding affinity is essentially lost may be assessed using SPR (Biacore) as well as using chromatography columns packed with resins coupled to monomeric polypeptides of the present disclosure. As discussed above in more detail, in some embodiments, the immunoglobulin is IgG, such as IgG selected from the group consisting of IgG1, IgG2 and IgG4. The immunoglobulin may be a human immunoglobulin. The IgG may be IgG1, such as trastuzumab and/or adalimumab.

Beside the above discussed advantageous properties, the present inventors have surprisingly found that the hereby disclosed amino acid substitutions in positions in the amino acid sequence of the polypeptide corresponding to positions X₁₈, X₂₄ and X₂₈ in SEQ ID NO:7 may also be advantageous for improved binding affinity of the polypeptides to their target(s), while at the same time enabling mild elution. This is demonstrated in e.g. Example 1, using Biacore with adalimumab as analyte in comparison to a known reference polypeptide, SEQ ID NO:10, as ligand. Thus, in some embodiments, polypeptides of the present disclosure have a higher binding affinity than SEQ ID NO:10 for adalimumab. In some embodiments, said polypeptides have a higher binding affinity than SEQ ID NO:10 for an Fc region of adalimumab.

The term "having binding affinity" as used in this disclosure refers to a property of a polypeptide which may be tested by ligand binding assays and comparative binding experiments, for example by ELISA or SPR technology, as demonstrated in the appended Examples. Binding affinity may be tested in an experiment in which a target molecule, such as an immunoglobulin, such as IgG, such as IgG selected from the group consisting of IgG1, IgG2 and IgG4 (or e.g. an Fc fragment thereof) is immobilized on a sensor chip of the SPR instrument, and the sample containing the polypeptide to be tested is passed over the chip. Trastuzumab and/or adalimumab may for example be used. Alternatively, the polypeptide to be tested is immobilized on a sensor chip of the instrument, and a sample containing target molecule (as listed above) is passed over the chip. The skilled person may then interpret the results obtained by such experiments to establish at least a qualitative measure of the binding affinity of the polypeptide for the target. For example, as demonstrated in Example 1 below, when the polypeptide is immobilized on the sensor chip and IgG (such as trastuzumab and/or adalimumab) is used as analyte, 1:1 binding affinity cannot be calculated as the analyte has two binding sites. Accordingly, binding affinity in the context of the present disclosure is to be understood as apparent binding affinity. Characteristics of polypeptides of the disclosure as well as comparison to known polypeptides are discussed with reference to apparent binding affinity. If a quantitative measure is desired, for example to determine a KD value for the interaction, SPR methods may be used, wherein the polypeptides (in a monomeric form) are used as analyte. Binding values may for example be defined in a Biacore (Cytiva) or ProteOn XPR 36 (Bio-Rad) instrument. For example IgG is suitably immobilized on a sensor chip of the instrument, and samples of the polypeptide whose affinity is to be determined are prepared by serial dilution and injected in random order. KD values may then be calculated from the results using for example the 1:1 Langmuir binding model of the BIAevaluation 4.1 software, or other suitable software, provided by the instrument manufacturer.

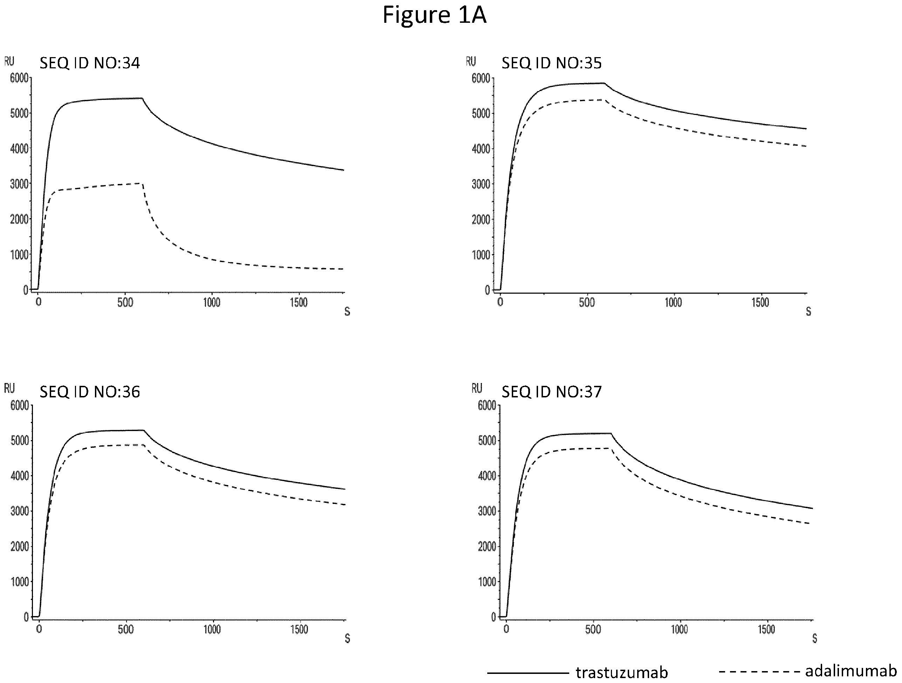

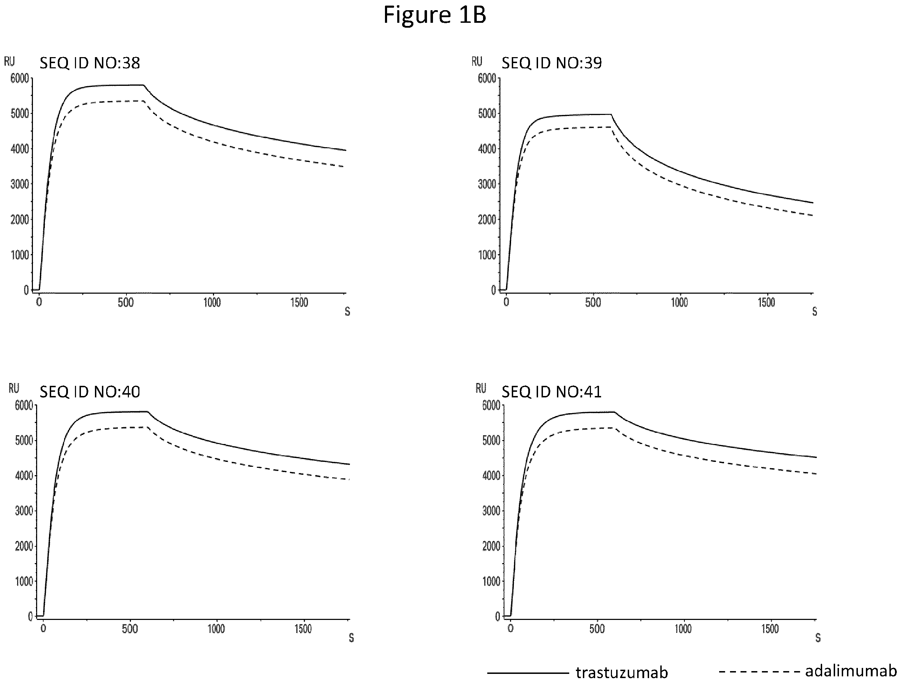

As shown in Example 1, polypeptides of the present disclosure are able to bind to adalimumab with improved binding characteristics, such as binding affinity and binding capacity, as measured in Biacore. Thus, adalimumab may be used in SPR experiments to further characterize the interactions. For comparison of relative binding affinities, for example SEQ ID NO:10 can be used as control under the same experimental conditions. The skilled person will appreciate that it may be useful to compare binding affinity (e.g. apparent binding affinity and/or KD values) obtained using the same assay and while some variation may occur between different assays, intra-assay comparisons generally demonstrate the same trends independent of the assay employed. In some embodiments, the binding affinity of a polypeptide according to the present disclosure for adalimumab (e.g. as measured in Biacore) is considered higher than the binding affinity of SEQ ID NO:10 for the same, if said binding affinity of the evaluated polypeptide is more than 100%, such as more than 110%, such as more than 120%, of the corresponding binding affinity of SEQ ID NO:10. In such comparisons, the binding affinity of the polypeptides for a target may be measured at pH 7.0. As discussed above, said binding affinity may be apparent binding affinity. The skilled person will understand higher binding affinity in the present context, at least in light of the data shown in Example 1, and Fig. 1. In some embodiments, the binding capacity of a polypeptide according to the present disclosure for adalimumab (e.g. as measured in Biacore) is considered higher than the binding capacity of SEQ ID NO:10 for the same, if said binding capacity of the evaluated polypeptide is more than 100%, such as more than 110%, such as more than 120%, such as more than 130%, such as more than 140%, such as more than 150%, such as more than 160%, such as more than 170%, such as more than 180%, such as more than 190%, such as more than 200%, of the corresponding binding capacity of SEQ ID NO:10. In such comparisons, the binding capacity of the polypeptides may be measured at pH 7.0. The skilled person will understand higher binding capacity in the present context, at least in light of the data shown in Example 1, and Fig. 1. Thus, it will also be appreciated that, in some embodiments, polypeptides of the present disclosure have a higher binding capacity for adalimumab compared to the binding capacity of SEQ ID NO:10 for the same.

In addition to the above discussed amino acid substitutions, the present inventors have surprisingly found that further introducing amino acid mutations in positions X₃₃, X₄₀ and X₅₁ of the polypeptides derived from SpA or domains thereof improves selectivity of the polypeptides. In some embodiments, the polypeptide has lower binding affinity for a VH3 region of trastuzumab compared to the binding affinity of SEQ ID NO:6 and/or SEQ ID NO:7 for the same VH3 region. It is to be understood that in certain embodiments, said polypeptide has essentially no binding affinity for said VH3 region. The binding affinity for the VH3 region of trastuzumab may be measured at pH 7.0. These amino acid mutations, which improve selectivity of the polypeptides of the present disclosure for their Fc target molecules lead to substantially reduced or abolished ability of the polypeptides to bind the VH3 region of trastuzumab, and are described in detail in WO2023/046886. For the sake of brevity, WO2023/046886 is included hereby by reference in its entirety, and amino acid mutations as described in WO2023/046886 are only briefly mentioned herein in the context of the present disclosure. It is to be understood that any amino acid mutation or a combination of amino acid mutations that leads to substantially reduced or abolished ability of the polypeptides to bind the VH3 region of trastuzumab is beneficial for improved selectivity of the polypeptides according to the present disclosure.

The term "essentially no binding affinity" to a non-target molecule means substantially reduced or abolished binding to a non-target molecule, such as immunoglobulin A (IgA), immunoglobulin D (IgD), immunoglobulin E (IgE) and immunoglobulin M (IgM), and/or the Fc region thereof, in the context of the present disclosure. In some embodiments, said polypeptide has thus essentially no binding affinity for an immunoglobulin selected from the group consisting of IgM, IgD, IgE and IgA; and/or the Fc region thereof. It is to be understood that polypeptides of the present disclosure have essentially no binding affinity for the Fc region of IgM, IgD, IgE and/or IgA. Additionally, in particular embodiments, polypeptides of the present disclosure have essentially no binding affinity for the VH3 region of any immunoglobulin, such as trastuzumab. This particular context thus includes substantially reduced or abolished binding to VH3 regions of a VH3-type immunoglobulins (or immunoglobulin derivatives), such as trastuzumab. As explained above, binding affinity in the present context refers to apparent binding affinity. This may be measured using the techniques described above for assessing binding affinity and may be evaluated in comparison to the binding affinity of one or more control polypeptide(s), such as SEQ ID NO:6, SEQ ID NO:7 and/or SEQ ID NO:9, for the same non-target molecule. The most suitable control polypeptide(s) may be selected based on which non-target molecule is tested for binding. For example, the binding of SEQ ID NO:8 to a VH3 region of an immunoglobulin, such as trastuzumab, is considered substantially reduced or abolished in comparison to the binding of SEQ ID NO:6 and/or SEQ ID NO:7 to the same VH3 region. The binding is considered substantially reduced or abolished according to the present disclosure if the binding affinity of the evaluated polypeptide or polypeptide multimer for said non-target molecule is at most 1 % of the binding affinity of one or more of said control polypeptides for the same non-target molecule.

The skilled person will appreciate that the polypeptides derived from SpA or any domain thereof as disclosed herein may for example, but not necessarily, be derived from any one of domains A (SEQ ID NO:3), B (SEQ ID NO:2), C (SEQ ID NO:1), D (SEQ ID NO:4) and E (SEQ ID NO:5) of SpA or derivatives thereof, such as domain Z (SEQ ID NO:6) or variants thereof, such as SEQ ID NO:7, SEQ ID NO:8 or SEQ ID NO:9. The polypeptide derived from an SpA domain may thus be a derivative, a mutant, a variant or a fragment of an SpA domain as defined above. In certain embodiments, the polypeptide comprises an amino acid sequence that has at least 70% identity to a sequence selected from the group consisting of SEQ ID NO:1-9, such as the group consisting of SEQ ID NO:1-8. In some embodiments, the amino acid sequence has at least 75%, such as at least 80%, such as at least 85%, such as at least 90%, such as at least 91%, such as at least 92%, such as at least 93%, such as at least 94%, such as at least 95%, identity to a sequence selected from the group consisting of SEQ ID NO:1-9, such as the group consisting of SEQ ID NO:1-8. In some embodiments, the amino acid sequence has at least 70%, such as at least 75%, such as at least 80%, such as at least 85%, such as at least 90%, such as at least 91%, such as at least 92%, such as at least 93%, such as at least 94%, such as at least 95%, identity to a sequence according to SEQ ID NO:7 and/or SEQ ID NO:8. For the purpose of determining said sequence identity, positions in the amino acid sequence corresponding to positions X₁₈, X₂₄, X₂₈; positions X₁₈, X₂₄, X₂₈, X₃₃, X₄₀, X₅₁; positions X₉, X₁₁, X₁₈, X₂₄, X₂₈; positions X₉, X₁₁, X₁₅, X₁₈, X₂₄, X₂₈, positions X₉, X₁₁, X₁₈, X₂₄, X₂₈, X₃₃, X₄₀, X₅₁; and/or positions X₉, X₁₁, X₁₅, X₁₈, X₂₄, X₂₈, X₃₃, X₄₀, X₅₁; in SEQ ID NO:7 are, in some embodiments, omitted.

As described above in detail, the loss of the binding affinity for the Fc region of the immunoglobulin may be induced by a modified electrostatic repulsion, which may be mediated by at least one amino acid selected from the group consisting of amino acids in positions in the amino acid sequence of the polypeptide corresponding to positions X₁₈, X₂₄ and X₂₈ in SEQ ID NO:7. In one embodiment, said at least one amino acid is an amino acid in the position in the amino acid sequence of the polypeptide corresponding to position X₁₈ SEQ ID NO:7. In another embodiment, said at least one amino acid comprises amino acids in the positions in the amino acid sequence of the polypeptide corresponding to positions X₁₈ and X₂₄ in SEQ ID NO:7. In a particular embodiment, said at least one amino acid comprises all three amino acids in the positions in the amino acid sequence of the polypeptide corresponding to positions X₁₈, X₂₄ and X₂₈ in SEQ ID NO:7. The inventors demonstrate in the appended Examples that certain amino acid variants in these positions are particularly beneficial for mediating the loss of the binding affinity for said Fc region at a mild pH, i.e. at slightly higher, but still weakly acidic pH values. Thus, in some embodiments, the polypeptide comprises an amino acid selected from R and K in a position in the amino acid sequence thereof, which position corresponds to position X₁₈ in SEQ ID NO:7. In some embodiments, the polypeptide comprises an amino acid selected from H, D, Q and E, such as from H and D, in a position in the amino acid sequence thereof, which position corresponds to position X₂₄ in SEQ ID NO:7. In some embodiments, the polypeptide comprises an amino acid selected from H, T, K, S and G, such as from H, K and G, such as from H and G, in a position in the amino acid sequence thereof, which position corresponds to position X₂₈ in SEQ ID NO:7.

In line with the beneficial role of certain amino acid variants in positions in the amino acid sequence of the polypeptide corresponding to positions X₁₈, X₂₄ and/or X₂₈ in SEQ ID NO:7, in a second aspect of the disclosure, there is provided a polypeptide derived from a SpA or any domain thereof comprising an amino acid sequence, wherein said polypeptide comprises an amino acid selected from R and K in a position in the amino acid sequence thereof, which position corresponds to position X₁₈ in SEQ ID NO:7; an amino acid selected from H, D, Q and E, such as from H and D, in a position in said amino acid sequence, which position corresponds to position X₂₄ in SEQ ID NO:7; and an amino acid selected from H, T, K, S and G, such as from H, K and G, such as from H and G, in a position in said amino acid sequence, which position corresponds to position X₂₈ in SEQ ID NO:7. It is to be understood that embodiments and definitions discussed above in the first aspect of the disclosure in relation to the features of the herein disclosed polypeptides are equally relevant for the second aspect. For the sake of brevity, these are not repeated herein or just briefly mentioned: According to the beneficial role of the above listed amino acid variants for mild elution, in some embodiments of the second aspect of the disclosure, the polypeptide has binding affinity to an Fc region of an immunoglobulin, such as binding affinity to said Fc region of the immunoglobulin at pH 7.0. In some embodiments, the polypeptide has binding affinity to said Fc region of the immunoglobulin at pH 7.0, which binding affinity for said Fc region is essentially lost at and below pH 4.4. In some embodiments, the binding affinity for said Fc region is essentially lost at and below pH 4.4, or at and below pH 4.5, or at and below pH 4.6, or at and below pH 4.7, or at and below pH 4.8, or at and below pH 4.9, or at and below pH 5.0, or at and below pH 5.5, or at and below pH 6.0. In some embodiments, the binding affinity for said Fc region is essentially lost at and below pH 4.5, or at and below pH 4.6, or at and below pH 4.7, or at and below pH 4.8, or at and below pH 4.9, or at and below pH 5.0, or at and below pH 5.5, or at and below pH 6.0. Said binding affinity, in some embodiments, is essentially lost at and below pH 4.6, or at and below pH 4.7, or at and below pH 4.8, or at and below pH 4.9, or at and below pH 5.0, or at and below pH 5.5, or at and below pH 6.0. In other embodiments, said binding affinity is essentially lost at and below pH 4.7, or at and below pH 4.8, or at and below pH 4.9, or at and below pH 5.0, or at and below pH 5.5, or at and below pH 6.0. In some embodiments, said binding affinity is essentially lost at and below pH 4.8, or at and below pH 4.9, or at and below pH 5.0, or at and below pH 5.5, or at and below pH 6.0. In some embodiments, the binding affinity is essentially lost at and below pH 4.9, or at and below pH 5.0, or at and below pH 5.5, or at and below pH 6.0. In some embodiments, the binding affinity is essentially lost at and below pH 5.0, or at and below pH 5.5, or at and below pH 6.0. In some embodiments, the binding affinity is essentially lost at and below pH 5.5, or at and below pH 6.0. In some embodiments, the binding affinity is essentially lost at and below pH 6.0. In particular embodiments, the binding affinity is essentially lost at and below pH 5.0, or at and below pH 5.5. In one embodiment, the binding affinity is essentially lost at and below pH 4.5. In one embodiment, the binding affinity is essentially lost at and below pH 4.6. In one embodiment, the binding affinity is essentially lost at and below pH 4.7. In one embodiment, the binding affinity is essentially lost at and below pH 4.8. In one embodiment, the binding affinity is essentially lost at and below pH 4.9. In one embodiment, the binding affinity is essentially lost at and below pH 5.0. In one embodiment, the binding affinity is essentially lost at and below pH 5.5. In one embodiment, the binding affinity is essentially lost at and below pH 5.6. In one embodiment, the binding affinity is essentially lost at and below pH 5.8. In particular embodiments, the binding affinity for the Fc region is essentially lost at and below a pH range of pH 4.4-6.0, such as a pH range of pH 4.5-6.0, such as a pH range of pH 4.7-6.0, such as a pH range of pH 4.8-6.0, such as a pH range of pH 4.9-6.0, such as a pH range of pH 5.0-6.0, such as a pH range of pH 5.1-6.0, such as a pH range of pH 5.2-6.0, such as a pH range of pH 5.3-6.0, such as a pH range of pH 5.4-6.0, such as a pH range of pH 5.5-6.0, such as a pH range of pH 5.6-6.0, such as a pH range of pH 5.7-6.0, such as a pH range of pH 5.8-6.0, such as a pH range of pH 5.9-6.0. In particular embodiments, the binding affinity for the Fc region is essentially lost at and below a pH range of pH 4.4-6.0, such as a pH range of pH 4.4-5.9, such as a pH range of pH 4.4-5.8, such as a pH range of pH 4.4-5.7, such as a pH range of pH 4.4-5.6, such as a pH range of pH 4.4-5.5, such as a pH range of pH 4.4-5.4, such as a pH range of pH 4.4-5.3, such as a pH range of pH 4.4-5.2, such as a pH range of pH 4.4-5.1, such as a pH range of pH 4.5-5.0, such as a pH range of pH 4.6-4.9, such as a pH range of pH 4.7-4.9. The loss of said binding affinity for the Fc region may be induced by electrostatic repulsion. The electrostatic repulsion may be mediated by at least one amino acid selected from the group consisting of amino acids in positions in the amino acid sequence of the polypeptide corresponding to positions X₁₈, X₂₄ and X₂₈ in SEQ ID NO:7. The immunoglobulin may be IgG, such as IgG selected from the group consisting of IgG1, IgG2 and IgG4. The IgG may be IgG1, such as trastuzumab and/or adalimumab. In some embodiments, the polypeptide has a higher binding affinity than SEQ ID NO:10 for adalimumab. In some embodiments, the polypeptide has lower binding affinity for a VH3 region of trastuzumab compared to the binding affinity of SEQ ID NO:6 and/or SEQ ID NO:7 for the same VH3 region. The binding affinity for the VH3 region of trastuzumab may be measured at pH 7.0. In some embodiments, the amino acid sequence of the polypeptide has at least 70% identity to a sequence selected from the group consisting of SEQ ID NO:1-9, such as the group consisting of SEQ ID NO:1-8. For the purpose of determining said sequence identity, positions in the amino acid sequence corresponding to positions X₁₈, X₂₄, X₂₈; positions X₁₈, X₂₄, X₂₈, X₃₃, X₄₀, X₅₁; positions X₉, X₁₁, X₁₈, X₂₄, X₂₈; positions X₉, X₁₁, X₁₅, X₁₈, X₂₄, X₂₈; positions X₉, X₁₁, X₁₈, X₂₄, X₂₈, X₃₃, X₄₀, X₅₁; and/or positions X₉, X₁₁, X₁₅, X₁₈, X₂₄, X₂₈, X₃₃, X₄₀, X₅₁; in SEQ ID NO:7 are, in some embodiments, omitted.

For the sake of brevity, the following section describes embodiments that are equally relevant for the first and second aspects of the disclosure, as presented in the Itemized List of Embodiments below. Accordingly, in some embodiments of the first and the second aspect of the disclosure, the amino acid in said position corresponding to position X₁₈ in SEQ ID NO:7 is R. In some embodiments, the amino acid in the position corresponding to position X₂₄ in SEQ ID NO:7 is selected from H and Q. In some embodiments, the amino acid in said position corresponding to position X₂₄ in SEQ ID NO:7 is H. In some embodiments, the amino acid in the position corresponding to position X₂₈ in SEQ ID NO:7 is selected from G, H, S and T; such as from G, H and S; such as from G and H. In some embodiments, the amino acid in said position corresponding to position X₂₈ in SEQ ID NO:7 is H. In some embodiments, the polypeptide comprises an amino acid selected from T, S, G, Q, A, E, H, R, P, D, K and N, such as from S, A and E, in a position in the amino acid sequence thereof, which position corresponds to position X₃₃ in SEQ ID NO:7; an amino acid selected from V, E, G, R, D, K, Q, N, H and S, such as from E, G, R, D, K, Q, N, H and S, such as from G, R, E and D, in a position in the amino acid sequence thereof, which position corresponds to position X₄₀ in SEQ ID NO:7; and an amino acid selected from L, V, S, I, R and G, such as from V, L, I and R, in a position in the amino acid sequence thereof, which position corresponds to position X₅₁ in SEQ ID NO:7. In some embodiments, the amino acid in said position corresponding to position X₃₃ in SEQ ID NO:7 is S. In some embodiments, the amino acid in said position corresponding to position X₄₀ in SEQ ID NO:7 is G. In some embodiments, the amino acid in said position corresponding to position X₅₁ in SEQ ID NO:7 is V.

In addition to the above described amino acid substitutions, the inventors have surprisingly found that certain amino acid variants in positions in the amino acid sequence of the polypeptides corresponding to position X₉ and/or X₁₁ in SEQ ID NO:7 contribute to optimal alkali stability of the polypeptides, as e.g. demonstrated in Example 3. In some embodiments, the polypeptide comprises an amino acid selected from E, N, H, R and S, such as from N, E, H and R, in a position in the amino acid sequence thereof, which position corresponds to position X₁₁ in SEQ ID NO:7; and/or an amino acid selected from A and Q in a position in the amino acid sequence thereof, which position corresponds to position X₉ in SEQ ID NO:7; preferably with the proviso that the amino acids in the positions corresponding to X₉X₁₁ in SEQ ID NO:7 are not AE. Thus, in some embodiments, the polypeptide comprises an amino acid selected from E, N, H, R and S, such as from N, E, H and R, in a position in the amino acid sequence thereof, which position corresponds to position X₁₁ in SEQ ID NO:7; and/or an amino acid selected from A and Q in a position in the amino acid sequence thereof, which position corresponds to position X₉ in SEQ ID NO:7. In other embodiments, the polypeptide comprises an amino acid selected from E, N, H, R and S, such as from N, E, H and R, in a position in the amino acid sequence thereof, which position corresponds to position X₁₁ in SEQ ID NO:7; and/or an amino acid selected from A and Q in a position in the amino acid sequence thereof, which position corresponds to position X₉ in SEQ ID NO:7; with the proviso that the amino acids in the positions corresponding to X₉X₁₁ in SEQ ID NO:7 are not AE. As shown in Example 2 and Table 3, such proviso may be surprisingly beneficial for improved binding capacity of the herein disclosed polypeptides and multimers thereof. In some embodiments, the amino acid in said position corresponding to position X₁₁ in SEQ ID NO:7 is selected from H, R and E, such as from R and E. In some embodiments, the amino acid in said position corresponding to position X₁₁ in SEQ ID NO:7 is selected from H, R and N, such as from N and H. In some embodiments, the amino acid in said position corresponding to position X₁₁ in SEQ ID NO:7 is selected from N, R and E, such as from R and E. In some embodiments, the amino acid in said position corresponding to position X₁₁ in SEQ ID NO:7 is selected from N and E. In some embodiments, the amino acid in said position corresponding to position X₁₁ in SEQ ID NO:7 is N. In some embodiments, the amino acid in said position corresponding to position X₁₁ in SEQ ID NO:7 is E. In some embodiments, the amino acid in said position corresponding to position X₉ in SEQ ID NO:7 is A. In some embodiments, the amino acid in said position corresponding to position X₉ in SEQ ID NO:7 is Q. In some embodiments, the amino acid in said position corresponding to position X₉ in SEQ ID NO:7 is Q and the amino acid in said position corresponding to position X₁₁ in SEQ ID NO:7 is E; or the amino acid in said position corresponding to position X₉ in SEQ ID NO:7 is A and the amino acid in said position corresponding to position X₁₁ in SEQ ID NO:7 is N. In some embodiments, the amino acids in the positions corresponding to X₉X₁₁ in SEQ ID NO:7 are not AE. In some embodiments, the polypeptide comprises an amino acid selected from E and H in a position in the amino acid sequence thereof, which position corresponds to position X₁₅ in SEQ ID NO:7. In some particular embodiments, the amino acids in the positions corresponding to X₁₈X₂₄X₂₈ in SEQ ID NO:7 are selected from the group consisting of RGD, KDG, KDT, RES, KQT, KHG, RDK, KHT, RHS, RHH and RHG; such as the group consisting of KDG, KDT, RES, KQT, KHG, RDK, KHT, RHS, RHH and RHG; such as the group consisting of KDT, RES, KQT, KHG, RDK, KHT, RHS, RHH and RHG; such as the group consisting of KQT, KHG, RDK, KHT, RHS, RHH and RHG; such as the group consisting of KHG, RDK, KHT, RHS, RHH and RHG; such as the group consisting of RDK, KHT, RHS, RHH and RHG; such as the group consisting of KHT, RHS, RHH and RHG; such as the group consisting of RHS, RHH and RHG; such as the group consisting of RHH and RHG. In one embodiment, the amino acids in the positions corresponding to X₁₈X₂₄X₂₈ in SEQ ID NO:7 are RHH. In one embodiment, the amino acids in the positions corresponding to X₃₃X₄₀X₅₁ in SEQ ID NO:7 are SGV. In some embodiments, the amino acids in the positions corresponding to X₉X₁₁ in SEQ ID NO:7 are selected from the group consisting of AE, QN, AN, QH, QR and QE; such as the group consisting of QN, AN, QH, QR and QE; such as the group consisting of AN, QH, QR and QE; such as the group consisting of QH, QR and QE; such as the group consisting of QR and QE. In some embodiments, the amino acids in the positions corresponding to X₉X₁₁ in SEQ ID NO:7 are selected from the group consisting of QN, AN, QR and QE; such as the group consisting of AN, QR and QE; such as the group consisting of QR and QE. In some embodiments, the amino acids in the positions corresponding to X₉X₁₁ in SEQ ID NO:7 are selected from the group consisting of QN, AN and QE; such as the group consisting of AN and QE. In one embodiment, the amino acids in the positions corresponding to X₉X₁₁ in SEQ ID NO:7 are QE.

In some embodiments of the first and the second aspects of the present disclosure, the polypeptide comprises Sequence A as defined in any one of the embodiments of the third aspect of the disclosure. It is to be understood that in these embodiments, said Sequence A is comprised in the amino acid sequence of the polypeptide of the first and the second aspects in an amino acid region that corresponds to positions X₁₈ to X₂₈ in SEQ ID NO:7. In some embodiments, the polypeptide comprises Sequence B as defined in any one of the embodiments of the third aspect of the disclosure. It is to be understood that in these embodiments, said Sequence B is comprised in the amino acid sequence of the polypeptide of the first and the second aspects in an amino acid region that corresponds to positions X₁₈ to X₅₁ in SEQ ID NO:7. In some embodiments, the polypeptide comprises Sequence C as defined in any one of the embodiments of the third aspect of the disclosure. It is to be understood that in these embodiments, said Sequence C is comprised in the amino acid sequence of the polypeptide of the first and the second aspects in an amino acid region that corresponds to positions X₉ to X₂₈ in SEQ ID NO:7. In some embodiments, the polypeptide comprises Sequence D as defined in any one of the embodiments of the third aspect of the disclosure. It is to be understood that in these embodiments, said Sequence D is comprised in the amino acid sequence of the polypeptide of the first and the second aspects in an amino acid region that corresponds to positions X₉ to X₅₁ in SEQ ID NO:7.

In a third aspect of the disclosure, there is provided a polypeptide comprising an amino acid sequence comprising Sequence A, which Sequence A consists of an amino acid sequence selected from i), ii) and iii), wherein i), ii) and iii) are defined as follows:
i) X₁₈LPNLTX₂₄EQRX₂₈ (SEQ ID NO:12);
ii) an amino acid sequence which has at least 70% identity to a sequence defined by i);
iii) an amino acid sequence which has at least 70% identity to any sequence selected from the group consisting of: residues 18-28 in SEQ ID NO:6, residues 18-28 in SEQ ID NO:9, residues 18-28 in SEQ ID NO:8, residues 18-28 in SEQ ID NO:1, residues 18-28 in SEQ ID NO:2, residues 18-28 in SEQ ID NO:3, residues 21-31 in SEQ ID NO:4 and residues 16-26 in SEQ ID NO:5;
wherein additionally, in each of i), ii) and iii), independently from each other,
X₁₈ is selected from R and K,
X₂₄ is selected from H, D, Q and E, such as from H and D, and
X₂₈ is selected from H, T, K, S and G, such as from H, K and G, such as
from H and G.

Also encompassed by the present disclosure is a polypeptide comprising Sequence A which consists of an amino acid sequence with at least 75%, such as at least 80%, such as at least 85%, such as at least 90%, such as at least 91%, such as at least 92%, such as at least 93%, such as at least 94%, such as at least 95%, identity to the sequence as defined in i). Also encompassed by the present disclosure is a polypeptide comprising Sequence A which consists of an amino acid sequence with at least 75%, such as at least 80%, such as at least 85%, such as at least 90%, such as at least 91%, such as at least 92%, such as at least 93%, such as at least 94%, such as at least 95%, identity to any sequence selected from the group consisting of: residues 18-28 in SEQ ID NO:6, residues 18-28 in SEQ ID NO:9, residues 18-28 in SEQ ID NO:8, residues 18-28 in SEQ ID NO:1, residues 18-28 in SEQ ID NO:2, residues 18-28 in SEQ ID NO:3, residues 21-31 in SEQ ID NO:4 and residues 16-26 in SEQ ID NO:5. For example, it is possible that an amino acid residue belonging to a certain functional grouping of amino acid residues (e.g. hydrophobic, hydrophilic, polar etc.) could be exchanged for another amino acid residue from the same functional group. In some embodiments, such changes may be made in any position except for X₁₈, X₂₄ and X₂₈ of the sequence of the polypeptide as disclosed herein.

In some embodiments, the polypeptide comprising Sequence A comprises an amino acid selected from T, S, G, Q, A, E, H, R, P, D, K and N, such as from S, A and E, in a position in the amino acid sequence thereof, which position corresponds to position X₃₃ in SEQ ID NO:7; an amino acid selected from V, E, G, R, D, K, Q, N, H and S, such as from E, G, R, D, K, Q, N, H and S, such as from G, R, E and D, in a position in the amino acid sequence thereof, which position corresponds to position X₄₀ in SEQ ID NO:7; and an amino acid selected from L, V, S, I, R and G, such as from V, L, I and R, in a position in the amino acid sequence thereof, which position corresponds to position X₅₁ in SEQ ID NO:7. In one embodiment, the amino acid in said position corresponding to position X₃₃ in SEQ ID NO:7 is S. In one embodiment, the amino acid in said position corresponding to position X₄₀ in SEQ ID NO:7 is G. In one embodiment, the amino acid in said position corresponding to position X₅₁ in SEQ ID NO:7 is V. In one embodiment, X₃₃X₄₀X₅₁ is SGV.

In some embodiments, the amino acid sequence of said polypeptide comprises Sequence B, which Sequence B consists of an amino acid sequence selected from iv), v) and vi), wherein iv), v) and vi) are defined as follows:
iv) X₁₈LPNLTX₂₄EQRX₂₈X₂₉FIQX₃₃LX₃₅DDPSX₄₀SX₄₂X₄₃X₄₄LX₄₆EAX₄₉KX₅₁ (SEQ ID NO:13),
   wherein, independently from each other,
   X₂₉ is selected from A and G,
   X₃₅ is selected from R and K,
   X₄₂ is selected from K, A and T, such as from K and A,
   X₄₃ is selected from N, A and E, such as from N and A,
   X₄₄ is selected from I, L and V, such as from I and L,
   X₄₆ is selected from A and G, and
   X₄₉ is selected from K and Q;
v) an amino acid sequence which has at least 70% identity to a sequence defined by iv);
vi) an amino acid sequence which has at least 70% identity to any sequence selected from the group consisting of: residues 18-51 in SEQ ID NO:6, residues 18-51 in SEQ ID NO:9, residues 18-51 in SEQ ID NO:8, residues 18-51 in SEQ ID NO:1, residues 18-51 in SEQ ID NO:2, residues 18-51 in SEQ ID NO:3, residues 21-54 in SEQ ID NO:4 and residues 16-49 in SEQ ID NO:5;
wherein additionally, in each of iv), v) and vi), independently from each other,
X₁₈ is selected from R and K,
X₂₄ is selected from H, D, Q and E, such as from H and D,
X₂₈ is selected from H, T, K, S and G, such as from H, K and G, such as from H and G,
X₃₃ is selected from T, S, G, Q, A, E, H, R, P, D, K and N, such as from S, A and E,
X₄₀ is selected from V, E, G, R, D, K, Q, N, H and S, such as from E, G, R, D, K, Q, N, H and S, such as from G, R, E and D, and
X₅₁ is selected from L, V, S, I, R and G, such as from V, L, I and R.

It is to be understood that the amino acid sequence from position X₁₈ to position X₂₈ corresponds to Sequence A, as defined in any one of the corresponding embodiments.

Also encompassed by the present disclosure is a polypeptide comprising Sequence B which consists of an amino acid sequence with at least 75%, such as at least 80%, such as at least 85%, such as at least 90%, such as at least 91%, such as at least 92%, such as at least 93%, such as at least 94%, such as at least 95%, identity to the sequence as defined in iv). Also encompassed by the present disclosure is a polypeptide comprising Sequence B which consists of an amino acid sequence with at least 75%, such as at least 80%, such as at least 85%, such as at least 90%, such as at least 91%, such as at least 92%, such as at least 93%, such as at least 94%, such as at least 95%, identity to any sequence selected from the group consisting of: residues 18-51 in SEQ ID NO:6, residues 18-51 in SEQ ID NO:9, residues 18-51 in SEQ ID NO:8, residues 18-51 in SEQ ID NO:1, residues 18-51 in SEQ ID NO:2, residues 18-51 in SEQ ID NO:3, residues 21-54 in SEQ ID NO:4 and residues 16-49 in SEQ ID NO:5. For example, it is possible that an amino acid residue belonging to a certain functional grouping of amino acid residues (e.g. hydrophobic, hydrophilic, polar etc.) could be exchanged for another amino acid residue from the same functional group. In some embodiments, such changes may be made in any position except for X₁₈, X₂₄, X₂₈, X₃₃, X₄₀ and X₅₁ of the sequence of the polypeptide as disclosed herein.

In some embodiments, the polypeptide comprising Sequence A and/or Sequence B comprises an amino acid selected from E, N, H, R and S, such as from N, E, H and R, in a position in the amino acid sequence thereof, which position corresponds to position X₁₁ in SEQ ID NO:7; and/or an amino acid selected from A and Q in a position in the amino acid sequence thereof, which position corresponds to position X₉ in SEQ ID NO:7; preferably with the proviso that the amino acids in the positions corresponding to X₉X₁₁ in SEQ ID NO:7 are not AE. As discussed above in relation to the first and second aspects of the disclosure, and as shown in Example 2 and Table 3, such proviso may be surprisingly beneficial for improved binding capacity of the herein disclosed polypeptides and multimers thereof. In some embodiments, the amino acid in said position corresponding to position X₁₁ in SEQ ID NO:7 is selected from H, R and E, such as from R and E. In some embodiments, the amino acid in said position corresponding to position X₁₁ in SEQ ID NO:7 is selected from H, R and N, such as from N and H. In some embodiments, the amino acid in said position corresponding to position X₁₁ in SEQ ID NO:7 is selected from N, R and E, such as from R and E. In some embodiments, the amino acid in said position corresponding to position X₁₁ in SEQ ID NO:7 is selected from N and E. In one embodiment, the amino acid in said position corresponding to position X₁₁ in SEQ ID NO:7 is N. In one embodiment, the amino acid in said position corresponding to position X₁₁ in SEQ ID NO:7 is E. In one embodiment, the amino acid in said position corresponding to position X₉ in SEQ ID NO:7 is A. In one embodiment, the amino acid in said position corresponding to position X₉ in SEQ ID NO:7 is Q. In some embodiments, the amino acid in said position corresponding to position X₉ in SEQ ID NO:7 is Q and the amino acid in said position corresponding to position X₁₁ in SEQ ID NO:7 is E; or the amino acid in said position corresponding to position X₉ in SEQ ID NO:7 is A and the amino acid in said position corresponding to position X₁₁ in SEQ ID NO:7 is N. In one embodiment, the amino acids in the positions corresponding to X₉X₁₁ in SEQ ID NO:7 are not AE. In some embodiments, the amino acids in the positions corresponding to X₉X₁₁ in SEQ ID NO:7 are selected from the group consisting of AE, QN, AN, QH, QR and QE; such as the group consisting of QN, AN, QH, QR and QE; such as the group consisting of AN, QH, QR and QE; such as the group consisting of QH, QR and QE; such as the group consisting of QR and QE. In some embodiments, the amino acids in the positions corresponding to X₉X₁₁ in SEQ ID NO:7 are selected from the group consisting of QN, AN, QR and QE; such as the group consisting of AN, QR and QE; such as the group consisting of QR and QE. In some embodiments, the amino acids in the positions corresponding to X₉X₁₁ in SEQ ID NO:7 are selected from the group consisting of QN, AN and QE; such as the group consisting of AN and QE. In one embodiment, the amino acids in the positions corresponding to X₉X₁₁ in SEQ ID NO:7 are QE. In some embodiments, the polypeptide comprises an amino acid selected from E and H in a position in the amino acid sequence thereof, which position corresponds to position X₁₅ in SEQ ID NO:7.

In some embodiments, the amino acid sequence of said polypeptide comprises Sequence C, which Sequence C consists of an amino acid sequence selected from vii), viii) and ix), wherein vii), viii) and ix) are defined as follows:
vii) X₉QX₁₁AFYX₁₅X₁₆LX₁₈LPNLTX₂₄EQRX₂₈ (SEQ ID NO:14),
   wherein, independently from each other,
   X₁₆ is selected from I and V;
viii) an amino acid sequence which has at least 70% identity to a sequence defined by vii);
ix) an amino acid sequence which has at least 70% identity to any sequence selected from the group consisting of: residues 9-28 in SEQ ID NO:6, residues 9-28 in SEQ ID NO:9, residues 9-28 in SEQ ID NO:8, residues 9-28 in SEQ ID NO:1, residues 9-28 in SEQ ID NO:2, residues 9-28 in SEQ ID NO:3, residues 12-31 in SEQ ID NO:4 and residues 7-26 in SEQ ID NO:5;
wherein additionally, in each of vii), viii) and ix), independently from each other,
X₉ is selected from A and Q,
X₁₁ is selected from E, N, H, R and S, such as from E, N, H and R;
X₁₅ is selected from E, Q and H, such as from E and H,
X₁₈ is selected from R and K,
X₂₄ is selected from H, D, Q and E, such as from H and D, and
X₂₈ is selected from H, T, K, S and G, such as from H, K and G, such as from H and G,
preferably with the proviso that X₉X₁₁ is not AE.

It is to be understood that the amino acid sequence from position X₁₈ to position X₂₈ corresponds to Sequence A, as defined in any one of the corresponding embodiments.

Also encompassed by the present disclosure is a polypeptide comprising Sequence C which consists of an amino acid sequence with at least 75%, such as at least 80%, such as at least 85%, such as at least 90%, such as at least 91%, such as at least 92%, such as at least 93%, such as at least 94%, such as at least 95%, identity to the sequence as defined in vii). Also encompassed by the present disclosure is a polypeptide comprising Sequence C which consists of an amino acid sequence with at least 75%, such as at least 80%, such as at least 85%, such as at least 90%, such as at least 91%, such as at least 92%, such as at least 93%, such as at least 94%, such as at least 95%, identity to any sequence selected from the group consisting of: residues 9-28 in SEQ ID NO:6, residues 9-28 in SEQ ID NO:9, residues 9-28 in SEQ ID NO:8, residues 9-28 in SEQ ID NO:1, residues 9-28 in SEQ ID NO:2, residues 9-28 in SEQ ID NO:3, residues 12-31 in SEQ ID NO:4 and residues 7-26 in SEQ ID NO:5. For example, it is possible that an amino acid residue belonging to a certain functional grouping of amino acid residues (e.g. hydrophobic, hydrophilic, polar etc.) could be exchanged for another amino acid residue from the same functional group. In some embodiments, such changes may be made in any position except for X₉, X₁₁, X₁₅, X₁₈, X₂₄ and X₂₈ or X₉, X₁₁, X₁₈, X₂₄ and X₂₈ of the sequence of the polypeptide as disclosed herein.

In some embodiments, the polypeptide comprising Sequence C comprises an amino acid selected from T, S, G, Q, A, E, H, R, P, D, K and N, such as from S, A and E, in a position in the amino acid sequence thereof, which position corresponds to position X₃₃ in SEQ ID NO:7; an amino acid selected from V, E, G, R, D, K, Q, N, H and S, such as from E, G, R, D, K, Q, N, H and S, such as from G, R, E and D, in a position in the amino acid sequence thereof, which position corresponds to position X₄₀ in SEQ ID NO:7; and an amino acid selected from L, V, S, I, R and G, such as from V, L, I and R, in a position in the amino acid sequence thereof, which position corresponds to position X₅₁ in SEQ ID NO:7. In one embodiment, the amino acid in said position corresponding to position X₃₃ in SEQ ID NO:7 is S. In one embodiment, the amino acid in said position corresponding to position X₄₀ in SEQ ID NO:7 is G. In one embodiment, the amino acid in said position corresponding to position X₅₁ in SEQ ID NO:7 is V. In one embodiment, the amino acids in the positions corresponding to X₃₃X₄₀X₅₁ in SEQ ID NO:7 are SGV.

In some embodiments, the amino acid sequence of said polypeptide comprises Sequence D, which Sequence D consists of an amino acid sequence selected from x), xi) and xii), wherein x), xi) and xii) are defined as follows:
x) X₉QX₁₁AFYX₁₅X₁₆LX₁₈LPNLTX₂₄EQRX₂₈X₂₉FIQX₃₃LX₃₅DDPSX₄₀SX₄₂X₄₃X₄₄LX₄₆ EAX₄₉KX₅₁ (SEQ ID NO:15),
   wherein, independently from each other,
   X₁₆ is selected from I and V;
   X₂₉ is selected from A and G,
   X₃₅ is selected from R and K,
   X₄₂ is selected from K, A and T, such as from K and A,
   X₄₃ is selected from N, A and E, such as from N and A,
   X₄₄ is selected from I, L and V, such as from I and L,
   X₄₆ is selected from A and G, and
   X₄₉ is selected from K and Q;
xi) an amino acid sequence which has at least 70% identity to a sequence defined by x);
xii) an amino acid sequence which has at least 70% identity to any sequence selected from the group consisting of: residues 9-51 in SEQ ID NO:6, residues 9-51 in SEQ ID NO:9, residues 9-51 in SEQ ID NO:8, residues 9-51 in SEQ ID NO:1, residues 9-51 in SEQ ID NO:2, residues 9-51 in SEQ ID NO:3, residues 12-54 in SEQ ID NO:4 and residues 7-49 in SEQ ID NO:5;
wherein additionally, in each of iv), v) and vi), independently from each other,
   X₉ is selected from A and Q,
   X₁₁ is selected from E, N, H, R and S, such as from E, N, H and R;
   X₁₅ is selected from E, Q and H, such as from E and H,
   X₁₈ is selected from R and K,
   X₂₄ is selected from H, D, Q and E, such as from H and D,
   X₂₈ is selected from H, T, K, S and G, such as from H, K and G, such as from H and G,
   X₃₃ is selected from T, S, G, Q, A, E, H, R, P, D, K and N, such as from S, A and E,
   X₄₀ is selected from V, E, G, R, D, K, Q, N, H and S, such as from E, G, R, D, K, Q, N, H and S, such as from G, R, E and D, and
   X₅₁ is selected from L, V, S, I, R and G, such as from V, L, I and R,
   preferably with the proviso that X₉X₁₁ is not AE.

It is to be understood that the amino acid sequence from position X₁₈ to position X₂₈ corresponds to Sequence A, as defined in any one of the corresponding embodiments; the amino acid sequence from position X₁₈ to position X₅₁ corresponds to Sequence B is as defined in any one of the corresponding embodiments; and the amino acid sequence from position X₉ to position X₂₈ corresponds to Sequence C is as defined in any one of the corresponding embodiments.

Also encompassed by the present disclosure is a polypeptide comprising Sequence D which consists of an amino acid sequence with at least 75%, such as at least 80%, such as at least 85%, such as at least 90%, such as at least 91%, such as at least 92%, such as at least 93%, such as at least 94%, such as at least 95%, identity to the sequence as defined in x). Also encompassed by the present disclosure is a polypeptide comprising Sequence D which consists of an amino acid sequence with at least 75%, such as at least 80%, such as at least 85%, such as at least 90%, such as at least 91%, such as at least 92%, such as at least 93%, such as at least 94%, such as at least 95%, identity to any sequence selected from the group consisting of: residues 9-51 in SEQ ID NO:6, residues 9-51 in SEQ ID NO:9, residues 9-51 in SEQ ID NO:8, residues 9-51 in SEQ ID NO:1, residues 9-51 in SEQ ID NO:2, residues 9-51 in SEQ ID NO:3, residues 12-54 in SEQ ID NO:4 and residues 7-49 in SEQ ID NO:5. For example, it is possible that an amino acid residue belonging to a certain functional grouping of amino acid residues (e.g. hydrophobic, hydrophilic, polar etc.) could be exchanged for another amino acid residue from the same functional group. In some embodiments, such changes may be made in any position except for X₉, X₁₁, X₁₅, X₁₈, X₂₄, X₂₈, X₃₃, X₄₀ and X₅₁ or X₉, X₁₁, X₁₈, X₂₄, X₂₈, X₃₃, X₄₀ and X₅₁ of the sequence of the polypeptide as disclosed herein.

The following embodiments of the third aspect describe amino acid variations and the combinations thereof, which may occur in the respective amino acid positions in Sequence A, Sequence B, Sequence C and/or Sequence D, as listed in the Itemized List of Embodiments below. For the sake of brevity, these are not repeated herein for each individual scaffold separately. In some embodiments, X₁₁ is selected from H, R and E, such as from R and E. In some embodiments, X₁₁ is selected from H, R and N, such as from N and H. In some embodiments, wherein X₁₁ is selected from N, R and E, such as from R and E. In some embodiments, X₁₁ is selected from N and E. In one embodiment, X₁₁ is N. In one embodiment, X₁₁ is E. In one embodiment, X₉ is A. In one embodiment, X₉ is Q. In some embodiments, X₉ is Q and X₁₁ is E; or X₉ is A and X₁₁ is N. In one embodiment, X₉X₁₁ is not AE. In one embodiment, X₁₅ is E. In one embodiment, X₁₆ is I. In one embodiment, X₁₈ is R. In some embodiments, X₂₄ is selected from H and Q. In one embodiment, X₂₄ is H. In some embodiments, X₂₈ is selected from G, H, S and T; such as from G, H and S; such as from G and H. In one embodiment, X₂₈ is H. In one embodiment, X₃₃ is S. In one embodiment, X₄₀ is G. In one embodiment, X₅₁ is V. In one embodiment, X₂₉ is A. In one embodiment, X₃₅ is K. In one embodiment, X₄₂ is K. In one embodiment, X₄₃ is A. In one embodiment, X₄₄ is I. In one embodiment, X₄₆ is A. In one embodiment, X₄₉ is K. In some embodiments, X₉X₁₁ is selected from the group consisting of AE, QN, AN, QH, QR and QE; such as the group consisting of QN, AN, QH, QR and QE; such as the group consisting of AN, QH, QR and QE; such as the group consisting of QH, QR and QE; such as the group consisting of QR and QE. In some embodiments, X₉X₁₁ is selected from the group consisting of QN, AN, QR and QE; such as the group consisting of AN, QR and QE; such as the group consisting of QR and QE. In some embodiments, X₉X₁₁ is selected from the group consisting of QN, AN and QE; such as the group consisting of AN and QE. In one embodiment, X₉X₁₁ is QE.

In some embodiments, X₁₈X₂₄X₂₈ is selected from the group consisting of RGD, KDG, KDT, RES, KQT, KHG, RDK, KHT, RHS, RHH and RHG; such as the group consisting of KDG, KDT, RES, KQT, KHG, RDK, KHT, RHS, RHH and RHG; such as the group consisting of KDT, RES, KQT, KHG, RDK, KHT, RHS, RHH and RHG; such as the group consisting of KQT, KHG, RDK, KHT, RHS, RHH and RHG; such as the group consisting of KHG, RDK, KHT, RHS, RHH and RHG; such as the group consisting of RDK, KHT, RHS, RHH and RHG; such as the group consisting of KHT, RHS, RHH and RHG; such as the group consisting of RHS, RHH and RHG; such as the group consisting of RHH and RHG. In one embodiment, X₃₃X₄₀X₅₁ is SGV.

In some embodiments, the amino acid sequence in i) corresponds to the region from position X₁₈ to position X₂₈ in an amino acid sequence selected from the group consisting of SEQ ID NO:16-33. In some embodiments, the amino acid sequence in i) corresponds to the region from position X₁₈ to position X₂₈ in an amino acid sequence selected from the group consisting of SEQ ID NO:19, 22, 16, 21, 29, 17, 25, 20, 23, 18, 24, 28, 33, 26, 27 and 30; such as the group consisting of SEQ ID NO:22, 16, 21, 29, 17, 25, 20, 23, 18, 24, 28, 33, 26, 27 and 30; such as the group consisting of SEQ ID NO:16, 21, 29, 17, 25, 20, 23, 18, 24, 28, 33, 26, 27 and 30; such as the group consisting of SEQ ID NO:17, 25, 20, 23, 18, 24, 28, 33, 26, 27 and 30; such as the group consisting of SEQ ID NO:25, 20, 23, 18, 24, 28, 33, 26, 27 and 30; such as the group consisting of SEQ ID NO:20, 23, 18, 24, 28, 33, 26, 27 and 30; such as the group consisting of SEQ ID NO:24, 28, 33, 26, 27 and 30; such as the group consisting of SEQ ID NO:28, 33, 26, 27 and 30. In some embodiments, the amino acid sequence in i) corresponds to the region from position X₁₈ to position X₂₈ in an amino acid sequence selected from the group consisting of SEQ ID NO:19, 22, 16, 21, 29, 17, 25, 20, 23, 18, 24, 28, 33, 26 and 27; such as the group consisting of SEQ ID NO:22, 16, 21, 29, 17, 25, 20, 23, 18, 24, 28, 33, 26 and 27; such as the group consisting of SEQ ID NO:16, 21, 29, 17, 25, 20, 23, 18, 24, 28, 33, 26 and 27; such as the group consisting of SEQ ID NO:17, 25, 20, 23, 18, 24, 28, 33, 26 and 27; such as the group consisting of SEQ ID NO:25, 20, 23, 18, 24, 28, 33, 26 and 27; such as the group consisting of SEQ ID NO:20, 23, 18, 24, 28, 33, 26 and 27; such as the group consisting of SEQ ID NO:24, 28, 33, 26 and 27; such as the group consisting of SEQ ID NO:28, 33, 26 and 27. In some embodiments, the amino acid sequence in i) corresponds to the region from position X₁₈ to position X₂₈ in an amino acid sequence selected from the group consisting of SEQ ID NO:18, 26-28, 31 and 32; such as the group consisting of SEQ ID NO:26-28, 31 and 32; such as the group consisting of SEQ ID NO:27, 28, 31 and 32; such as the group consisting of SEQ ID NO:27, 31 and 32; such as the group consisting of SEQ ID NO:31 and 32. In some embodiments, the amino acid sequence in i) corresponds to the region from position X₁₈ to position X₂₈ in an amino acid sequence selected from the group consisting of SEQ ID NO:18, 26-28 and 31; such as the group consisting of SEQ ID NO:26-28 and 31; such as the group consisting of SEQ ID NO:27, 28 and 31; such as the group consisting of SEQ ID NO:27 and 31. In one embodiment, the amino acid sequence in i) corresponds to the region from position X₁₈ to position X₂₈ in the amino acid sequence according to SEQ ID NO:31. In some embodiments, the amino acid sequence in iv) corresponds to the region from position X₁₈ to position X₅₁ in an amino acid sequence selected from the group consisting of SEQ ID NO:16-33. In some embodiments, the amino acid sequence in iv) corresponds to the region from position X₁₈ to position X₅₁ in an amino acid sequence selected from the group consisting of SEQ ID NO:19, 22, 16, 21, 29, 17, 25, 20, 23, 18, 24, 28, 33, 26, 27 and 30; such as the group consisting of SEQ ID NO:22, 16, 21, 29, 17, 25, 20, 23, 18, 24, 28, 33, 26, 27 and 30; such as the group consisting of SEQ ID NO:16, 21, 29, 17, 25, 20, 23, 18, 24, 28, 33, 26, 27 and 30; such as the group consisting of SEQ ID NO:17, 25, 20, 23, 18, 24, 28, 33, 26, 27 and 30; such as the group consisting of SEQ ID NO:25, 20, 23, 18, 24, 28, 33, 26, 27 and 30; such as the group consisting of SEQ ID NO:20, 23, 18, 24, 28, 33, 26, 27 and 30; such as the group consisting of SEQ ID NO:24, 28, 33, 26, 27 and 30; such as the group consisting of SEQ ID NO:28, 33, 26, 27 and 30. In some embodiments, the amino acid sequence in iv) corresponds to the region from position X₁₈ to position X₅₁ in an amino acid sequence selected from the group consisting of SEQ ID NO:19, 22, 16, 21, 29, 17, 25, 20, 23, 18, 24, 28, 33, 26 and 27; such as the group consisting of SEQ ID NO:22, 16, 21, 29, 17, 25, 20, 23, 18, 24, 28, 33, 26 and 27; such as the group consisting of SEQ ID NO:16, 21, 29, 17, 25, 20, 23, 18, 24, 28, 33, 26 and 27; such as the group consisting of SEQ ID NO:17, 25, 20, 23, 18, 24, 28, 33, 26 and 27; such as the group consisting of SEQ ID NO:25, 20, 23, 18, 24, 28, 33, 26 and 27; such as the group consisting of SEQ ID NO:20, 23, 18, 24, 28, 33, 26 and 27; such as the group consisting of SEQ ID NO:24, 28, 33, 26 and 27; such as the group consisting of SEQ ID NO:28, 33, 26 and 27. In some embodiments, the amino acid sequence in iv) corresponds to the region from position X₁₈ to position X₅₁ in an amino acid sequence selected from the group consisting of SEQ ID NO:18, 26-28, 31 and 32; such as the group consisting of SEQ ID NO:26-28, 31 and 32; such as the group consisting of SEQ ID NO:27, 28, 31 and 32; such as the group consisting of SEQ ID NO:27, 31 and 32; such as the group consisting of SEQ ID NO:31 and 32. In some embodiments, the amino acid sequence in iv) corresponds to the region from position X₁₈ to position X₅₁ in an amino acid sequence selected from the group consisting of SEQ ID NO:18, 26-28 and 31; such as the group consisting of SEQ ID NO:26-28 and 31; such as the group consisting of SEQ ID NO:27, 28 and 31; such as the group consisting of SEQ ID NO:27 and 31. In one embodiment, the amino acid sequence in iv) corresponds to the region from position X₁₈ to position X₅₁ in the amino acid sequence according to SEQ ID NO:31. In some embodiments, the amino acid sequence in vii) corresponds to the region from position X₉ to position X₂₈ in an amino acid sequence selected from the group consisting of SEQ ID NO:16-33. In some embodiments, the amino acid sequence in vii) corresponds to the region from position X₉ to position X₂₈ in an amino acid sequence selected from the group consisting of SEQ ID NO:19, 22, 16, 21, 29, 17, 25, 20, 23, 18, 24, 28, 33, 26, 27 and 30; such as the group consisting of SEQ ID NO:22, 16, 21, 29, 17, 25, 20, 23, 18, 24, 28, 33, 26, 27 and 30; such as the group consisting of SEQ ID NO:16, 21, 29, 17, 25, 20, 23, 18, 24, 28, 33, 26, 27 and 30; such as the group consisting of SEQ ID NO:17, 25, 20, 23, 18, 24, 28, 33, 26, 27 and 30; such as the group consisting of SEQ ID NO:25, 20, 23, 18, 24, 28, 33, 26, 27 and 30; such as the group consisting of SEQ ID NO:20, 23, 18, 24, 28, 33, 26, 27 and 30; such as the group consisting of SEQ ID NO:24, 28, 33, 26, 27 and 30; such as the group consisting of SEQ ID NO:28, 33, 26, 27 and 30; such as the group consisting of SEQ ID NO:26, 27 and 30. In one embodiment, the amino acid sequence in vii) corresponds to the region from position X₉ to position X₂₈ in the amino acid sequence according to SEQ ID NO:30. In some embodiments, the amino acid sequence in vii) corresponds to the region from position X₉ to position X₂₈ in an amino acid sequence selected from the group consisting of SEQ ID NO:19, 22, 16, 21, 29, 17, 25, 20, 23, 18, 24, 28, 33, 26 and 27; such as the group consisting of SEQ ID NO:22, 16, 21, 29, 17, 25, 20, 23, 18, 24, 28, 33, 26 and 27; such as the group consisting of SEQ ID NO:16, 21, 29, 17, 25, 20, 23, 18, 24, 28, 33, 26 and 27; such as the group consisting of SEQ ID NO:17, 25, 20, 23, 18, 24, 28, 33, 26 and 27; such as the group consisting of SEQ ID NO:25, 20, 23, 18, 24, 28, 33, 26 and 27; such as the group consisting of SEQ ID NO:20, 23, 18, 24, 28, 33, 26 and 27; such as the group consisting of SEQ ID NO:24, 28, 33, 26 and 27; such as the group consisting of SEQ ID NO:28, 33, 26 and 27; such as the group consisting of SEQ ID NO:26 and 27. In some embodiments, the amino acid sequence in vii) corresponds to the region from position X₉ to position X₂₈ in an amino acid sequence selected from the group consisting of SEQ ID NO:25, 20, 23, 18, 24, 28 and 33; such as the group consisting of SEQ ID NO:20, 23, 18, 24, 28 and 33; such as the group consisting of SEQ ID NO:23, 18, 24, 28 and 33; such as the group consisting of SEQ ID NO:18 and 24. In one embodiment, the amino acid sequence in vii) corresponds to the region from position X₉ to position X₂₈ in the amino acid sequence according to SEQ ID NO:18. In some embodiments, the amino acid sequence in vii) corresponds to the region from position X₉ to position X₂₈ in an amino acid sequence selected from the group consisting of SEQ ID NO:18, 28, 33, 26 and 27; such as the group consisting of SEQ ID NO:28, 33, 26 and 27; such as the group consisting of SEQ ID NO:28, 33 and 27; such as the group consisting of SEQ ID NO:33 and 27. In some embodiments, the amino acid sequence in vii) corresponds to the region from position X₉ to position X₂₈ in an amino acid sequence selected from the group consisting of SEQ ID NO:22, 16, 21, 29, 17, 25, 20, 23, 18, 24, 28, 33, 26, 27, 30, 31 and 32; such as the group consisting of SEQ ID NO:16, 21, 29, 17, 25, 20, 23, 18, 24, 28, 33, 26, 27, 30, 31 and 32; such as the group consisting of SEQ ID NO:17, 25, 20, 23, 18, 24, 28, 33, 26, 27, 30, 31 and 32; such as the group consisting of SEQ ID NO:25, 20, 23, 18, 24, 28, 33, 26, 27, 30, 31 and 32; such as the group consisting of SEQ ID NO:20, 23, 18, 24, 28, 33, 26, 27, 30, 31 and 32; such as the group consisting of SEQ ID NO:23, 18, 24, 28, 33, 26, 27, 30, 31 and 32; such as the group consisting of SEQ ID NO:18, 24, 28, 33, 26, 27, 30, 31 and 32. In some embodiments, the amino acid sequence in vii) corresponds to the region from position X₉ to position X₂₈ in an amino acid sequence selected from the group consisting of SEQ ID NO:19, 22, 16, 21, 29, 17, 25, 20, 23, 18, 24, 28, 33, 26, 27, 31 and 32; such as the group consisting of SEQ ID NO:22, 16, 21, 29, 17, 25, 20, 23, 18, 24, 28, 33, 26, 27, 31 and 32; such as the group consisting of SEQ ID NO:16, 21, 29, 17, 25, 20, 23, 18, 24, 28, 33, 26, 27, 31 and 32; such as the group consisting of SEQ ID NO:17, 25, 20, 23, 18, 24, 28, 33, 26, 27, 31 and 32; such as the group consisting of SEQ ID NO:25, 20, 23, 18, 24, 28, 33, 26, 27, 31 and 32; such as the group consisting of SEQ ID NO:20, 23, 18, 24, 28, 33, 26, 27, 31 and 32; such as the group consisting of SEQ ID NO:23, 18, 24, 28, 33, 26, 27, 31 and 32; such as the group consisting of SEQ ID NO:18, 24, 28, 33, 26, 27, 31 and 32. In some embodiments, the amino acid sequence in vii) corresponds to the region from position X₉ to position X₂₈ in an amino acid sequence selected from the group consisting of SEQ ID NO:25, 20, 23, 18, 24, 28, 31, 32 and 33; such as the group consisting of SEQ ID NO:20, 23, 18, 24, 28, 31, 32 and 33; such as the group consisting of SEQ ID NO:23, 18, 24, 28, 31, 32 and 33; such as the group consisting of SEQ ID NO:18, 31, 32 and 24. In some embodiments, the amino acid sequence in vii) corresponds to the region from position X₉ to position X₂₈ in an amino acid sequence selected from the group consisting of SEQ ID NO:18, 28, 33, 31, 32, 26 and 27; such as the group consisting of SEQ ID NO:28, 33, 31, 32, 26 and 27; such as the group consisting of SEQ ID NO:28, 33, 31, 32 and 27; such as the group consisting of SEQ ID NO:33, 31, 32, and 27. In some embodiments, the amino acid sequence in vii) corresponds to the region from position X₉ to position X₂₈ in an amino acid sequence selected from the group consisting of SEQ ID NO:18, 26-28, 31 and 32; such as the group consisting of SEQ ID NO:26-28, 31 and 32; such as the group consisting of SEQ ID NO:27, 28, 31 and 32; such as the group consisting of SEQ ID NO:27, 31 and 32; such as the group consisting of SEQ ID NO:31 and 32. In some embodiments, the amino acid sequence in vii) corresponds to the region from position X₉ to position X₂₈ in an amino acid sequence selected from the group consisting of SEQ ID NO:18, 26-28 and 31; such as the group consisting of SEQ ID NO:26-28 and 31; such as the group consisting of SEQ ID NO:27, 28 and 31; such as the group consisting of SEQ ID NO:27 and 31. In one embodiment, the amino acid sequence in vii) corresponds to the region from position X₉ to position X₂₈ in the amino acid sequence according to SEQ ID NO:31. In some embodiments, the amino acid sequence in x) corresponds to the region from position X₉ to position X₅₁ in an amino acid sequence selected from the group consisting of SEQ ID NO:16-33. In some embodiments, the amino acid sequence in x) corresponds to the region from position X₉ to position X₅₁ in an amino acid sequence selected from the group consisting of SEQ ID NO:19, 22, 16, 21, 29, 17, 25, 20, 23, 18, 24, 28, 33, 26, 27 and 30; such as the group consisting of SEQ ID NO:22, 16, 21, 29, 17, 25, 20, 23, 18, 24, 28, 33, 26, 27 and 30; such as the group consisting of SEQ ID NO:16, 21, 29, 17, 25, 20, 23, 18, 24, 28, 33, 26, 27 and 30; such as the group consisting of SEQ ID NO:17, 25, 20, 23, 18, 24, 28, 33, 26, 27 and 30; such as the group consisting of SEQ ID NO:25, 20, 23, 18, 24, 28, 33, 26, 27 and 30; such as the group consisting of SEQ ID NO:20, 23, 18, 24, 28, 33, 26, 27 and 30; such as the group consisting of SEQ ID NO:24, 28, 33, 26, 27 and 30; such as the group consisting of SEQ ID NO:28, 33, 26, 27 and 30; such as the group consisting of SEQ ID NO:26, 27 and 30. In one embodiment, the amino acid sequence in x) corresponds to the region from position X₉ to position X₅₁ in the amino acid sequence according to SEQ ID NO:30. In some embodiments, the amino acid sequence in x) corresponds to the region from position X₉ to position X₅₁ in an amino acid sequence selected from the group consisting of SEQ ID NO:19, 22, 16, 21, 29, 17, 25, 20, 23, 18, 24, 28, 33, 26 and 27; such as the group consisting of SEQ ID NO:22, 16, 21, 29, 17, 25, 20, 23, 18, 24, 28, 33, 26 and 27; such as the group consisting of SEQ ID NO:16, 21, 29, 17, 25, 20, 23, 18, 24, 28, 33, 26 and 27; such as the group consisting of SEQ ID NO:17, 25, 20, 23, 18, 24, 28, 33, 26 and 27; such as the group consisting of SEQ ID NO:25, 20, 23, 18, 24, 28, 33, 26 and 27; such as the group consisting of SEQ ID NO:20, 23, 18, 24, 28, 33, 26 and 27; such as the group consisting of SEQ ID NO:24, 28, 33, 26 and 27; such as the group consisting of SEQ ID NO:28, 33, 26 and 27; such as the group consisting of SEQ ID NO:26 and 27. In some embodiments, the amino acid sequence in x) corresponds to the region from position X₉ to position X₅₁ in an amino acid sequence selected from the group consisting of SEQ ID NO:25, 20, 23, 18, 24, 28 and 33; such as the group consisting of SEQ ID NO:20, 23, 18, 24, 28 and 33; such as the group consisting of SEQ ID NO:23, 18, 24, 28 and 33; such as the group consisting of SEQ ID NO:18 and 24. In one embodiment, the amino acid sequence in x) corresponds to the region from position X₉ to position X₅₁ in the amino acid sequence according to SEQ ID NO:18. In some embodiments, the amino acid sequence in x) corresponds to the region from position X₉ to position X₅₁ in an amino acid sequence selected from the group consisting of SEQ ID NO:18, 28, 33, 26 and 27; such as the group consisting of SEQ ID NO:28, 33, 26 and 27; such as the group consisting of SEQ ID NO:28, 33 and 27; such as the group consisting of SEQ ID NO:33 and 27. In some embodiments, the amino acid sequence in x) corresponds to the region from position X₉ to position X₅₁ in an amino acid sequence selected from the group consisting of SEQ ID NO:22, 16, 21, 29, 17, 25, 20, 23, 18, 24, 28, 33, 26, 27, 30, 31 and 32; such as the group consisting of SEQ ID NO:16, 21, 29, 17, 25, 20, 23, 18, 24, 28, 33, 26, 27, 30, 31 and 32; such as the group consisting of SEQ ID NO:17, 25, 20, 23, 18, 24, 28, 33, 26, 27, 30, 31 and 32; such as the group consisting of SEQ ID NO:25, 20, 23, 18, 24, 28, 33, 26, 27, 30, 31 and 32; such as the group consisting of SEQ ID NO:20, 23, 18, 24, 28, 33, 26, 27, 30, 31 and 32; such as the group consisting of SEQ ID NO:23, 18, 24, 28, 33, 26, 27, 30, 31 and 32; such as the group consisting of SEQ ID NO:18, 24, 28, 33, 26, 27, 30, 31 and 32. In some embodiments, the amino acid sequence in x) corresponds to the region from position X₉ to position X₅₁ in an amino acid sequence selected from the group consisting of SEQ ID NO:19, 22, 16, 21, 29, 17, 25, 20, 23, 18, 24, 28, 33, 26, 27, 31 and 32; such as the group consisting of SEQ ID NO:22, 16, 21, 29, 17, 25, 20, 23, 18, 24, 28, 33, 26, 27, 31 and 32; such as the group consisting of SEQ ID NO:16, 21, 29, 17, 25, 20, 23, 18, 24, 28, 33, 26, 27, 31 and 32; such as the group consisting of SEQ ID NO:17, 25, 20, 23, 18, 24, 28, 33, 26, 27, 31 and 32; such as the group consisting of SEQ ID NO:25, 20, 23, 18, 24, 28, 33, 26, 27, 31 and 32; such as the group consisting of SEQ ID NO:20, 23, 18, 24, 28, 33, 26, 27, 31 and 32; such as the group consisting of SEQ ID NO:23, 18, 24, 28, 33, 26, 27, 31 and 32; such as the group consisting of SEQ ID NO:18, 24, 28, 33, 26, 27, 31 and 32. In some embodiments, the amino acid sequence in x) corresponds to the region from position X₉ to position X₅₁ in an amino acid sequence selected from the group consisting of SEQ ID NO:25, 20, 23, 18, 24, 28, 31, 32 and 33; such as the group consisting of SEQ ID NO:20, 23, 18, 24, 28, 31, 32 and 33; such as the group consisting of SEQ ID NO:23, 18, 24, 28, 31, 32 and 33; such as the group consisting of SEQ ID NO:18, 31, 32 and 24. In some embodiments, the amino acid sequence in x) corresponds to the region from position X₉ to position X₅₁ in an amino acid sequence selected from the group consisting of SEQ ID NO:18, 28, 33, 31, 32, 26 and 27; such as the group consisting of SEQ ID NO:28, 33, 31, 32, 26 and 27; such as the group consisting of SEQ ID NO:28, 33, 31, 32 and 27; such as the group consisting of SEQ ID NO:33, 31, 32, and 27. In some embodiments, the amino acid sequence in x) corresponds to the region from position X₉ to position X₅₁ in an amino acid sequence selected from the group consisting of SEQ ID NO:18, 26-28, 31 and 32; such as the group consisting of SEQ ID NO:26-28, 31 and 32; such as the group consisting of SEQ ID NO:27, 28, 31 and 32; such as the group consisting of SEQ ID NO:27, 31 and 32; such as the group consisting of SEQ ID NO:31 and 32. In some embodiments, the amino acid sequence in x) corresponds to the region from position X₉ to position X₅₁ in an amino acid sequence selected from the group consisting of SEQ ID NO:18, 26-28 and 31; such as the group consisting of SEQ ID NO:26-28 and 31; such as the group consisting of SEQ ID NO:27, 28 and 31; such as the group consisting of SEQ ID NO:27 and 31. In one embodiment, the amino acid sequence in x) corresponds to the region from position X₉ to position X₅₁ in the amino acid sequence according to SEQ ID NO:31.

It is to be understood that embodiments discussed above in the first and the second aspect of the disclosure in relation to the features of the herein disclosed polypeptides are equally relevant for the third aspect. For the sake of brevity, these are not repeated herein or just briefly mentioned: In some embodiments, the polypeptide comprises an amino acid sequence that has at least 70% identity to a sequence selected from the group consisting of SEQ ID NO:1-9, such as the group consisting of SEQ ID NO:1-8. For the purpose of determining said sequence identity, positions in the amino acid sequence corresponding to positions X₁₈, X₂₄, X₂₈; positions X₁₈, X₂₄, X₂₈, X₃₃, X₄₀, X₅₁; positions X₉, X₁₁, X₁₈, X₂₄, X₂₈; positions X₉, X₁₁, X₁₅, X₁₈, X₂₄, X₂₈; positions X₉, X₁₁, X₁₈, X₂₄, X₂₈, X₃₃, X₄₀, X₅₁; and/or positions X₉, X₁₁, X₁₅, X₁₈, X₂₄, X₂₈, X₃₃, X₄₀, X₅₁; in SEQ ID NO:7 are, in some embodiments, omitted. In some embodiments, the polypeptide has binding affinity to an Fc region of an immunoglobulin, such as binding affinity to said Fc region of the immunoglobulin at pH 7.0. In some embodiments, the polypeptide has binding affinity to said Fc region of the immunoglobulin at pH 7.0, which binding affinity for said Fc region is essentially lost at and below pH 4.4. In some embodiments, the binding affinity for said Fc region is essentially lost at and below pH 4.4, or at and below pH 4.5, or at and below pH 4.6, or at and below pH 4.7, or at and below pH 4.8, or at and below pH 4.9, or at and below pH 5.0, or at and below pH 5.5, or at and below pH 6.0. In some embodiments, the binding affinity for said Fc region is essentially lost at and below pH 4.5, or at and below pH 4.6, or at and below pH 4.7, or at and below pH 4.8, or at and below pH 4.9, or at and below pH 5.0, or at and below pH 5.5, or at and below pH 6.0. Said binding affinity, in some embodiments, is essentially lost at and below pH 4.6, or at and below pH 4.7, or at and below pH 4.8, or at and below pH 4.9, or at and below pH 5.0, or at and below pH 5.5, or at and below pH 6.0. In other embodiments, said binding affinity is essentially lost at and below pH 4.7, or at and below pH 4.8, or at and below pH 4.9, or at and below pH 5.0, or at and below pH 5.5, or at and below pH 6.0. In some embodiments, said binding affinity is essentially lost at and below pH 4.8, or at and below pH 4.9, or at and below pH 5.0, or at and below pH 5.5, or at and below pH 6.0. In some embodiments, the binding affinity is essentially lost at and below pH 4.9, or at and below pH 5.0, or at and below pH 5.5, or at and below pH 6.0. In some embodiments, the binding affinity is essentially lost at and below pH 5.0, or at and below pH 5.5, or at and below pH 6.0. In some embodiments, the binding affinity is essentially lost at and below pH 5.5, or at and below pH 6.0. In some embodiments, the binding affinity is essentially lost at and below pH 6.0. In particular embodiments, the binding affinity is essentially lost at and below pH 5.0, or at and below pH 5.5. In one embodiment, the binding affinity is essentially lost at and below pH 4.5. In one embodiment, the binding affinity is essentially lost at and below pH 4.6. In one embodiment, the binding affinity is essentially lost at and below pH 4.7. In one embodiment, the binding affinity is essentially lost at and below pH 4.8. In one embodiment, the binding affinity is essentially lost at and below pH 4.9. In one embodiment, the binding affinity is essentially lost at and below pH 5.0. In one embodiment, the binding affinity is essentially lost at and below pH 5.5. In one embodiment, the binding affinity is essentially lost at and below pH 5.6. In one embodiment, the binding affinity is essentially lost at and below pH 5.8. In particular embodiments, the binding affinity for the Fc region is essentially lost at and below a pH range of pH 4.4-6.0, such as a pH range of pH 4.5-6.0, such as a pH range of pH 4.7-6.0, such as a pH range of pH 4.8-6.0, such as a pH range of pH 4.9-6.0, such as a pH range of pH 5.0-6.0, such as a pH range of pH 5.1-6.0, such as a pH range of pH 5.2-6.0, such as a pH range of pH 5.3-6.0, such as a pH range of pH 5.4-6.0, such as a pH range of pH 5.5-6.0, such as a pH range of pH 5.6-6.0, such as a pH range of pH 5.7-6.0, such as a pH range of pH 5.8-6.0, such as a pH range of pH 5.9-6.0. In particular embodiments, the binding affinity for the Fc region is essentially lost at and below a pH range of pH 4.4-6.0, such as a pH range of pH 4.4-5.9, such as a pH range of pH 4.4-5.8, such as a pH range of pH 4.4-5.7, such as a pH range of pH 4.4-5.6, such as a pH range of pH 4.4-5.5, such as a pH range of pH 4.4-5.4, such as a pH range of pH 4.4-5.3, such as a pH range of pH 4.4-5.2, such as a pH range of pH 4.4-5.1, such as a pH range of pH 4.5-5.0, such as a pH range of pH 4.6-4.9, such as a pH range of pH 4.7-4.9. The loss of said binding affinity for the Fc region may be induced by electrostatic repulsion, such as electrostatic repulsion mediated by at least one amino acid selected from the group consisting of amino acids in positions in the amino acid sequence of the polypeptide corresponding to positions X₁₈, X₂₄ and X₂₈ in SEQ ID NO:7. In some embodiments, the immunoglobulin is IgG, such as IgG selected from the group consisting of IgG1, IgG2 and IgG4. The IgG, in particular embodiments, is IgG1, such as trastuzumab and/or adalimumab. In some embodiments, the polypeptide has a higher binding affinity than SEQ ID NO:10 for adalimumab. In some embodiments, the polypeptide has lower binding affinity for a VH3 region of trastuzumab compared to the binding affinity of SEQ ID NO:6 and/or SEQ ID NO:7 for the same VH3 region. The binding affinity for the VH3 region of trastuzumab may be measured at pH 7.0.

In line with that some of the most important positively charged amino acids in known SpA-derived molecules are thought to be Arginine (R) in X₂₇ and Lysine (K) in X₃₅ (corresponding to positions X₂₇ and X₃₅ in SEQ ID NO:7), in some embodiments, the polypeptide according to any embodiment of the first, the second and the third aspects of the present disclosure comprises a R in a position in the amino sequence thereof, which position corresponds to position X₂₇ in SEQ ID NO:7; and/or a K or R (such as a K) in a position in the amino sequence thereof, which position corresponds to position X₃₅ in SEQ ID NO:7.

As it is demonstrated by the appended Examples and is apparent from the above description, in some embodiments of the first, the second and the third aspects of the disclosure, the amino acid sequence of the polypeptide comprises an amino acid sequence selected from the group consisting of SEQ ID NO:16-33. In some embodiments of the first, the second and the third aspects of the disclosure, the amino acid sequence of the polypeptide comprises an amino acid sequence selected from the group consisting of SEQ ID NO:19, 22, 16, 21, 29, 17, 25, 20, 23, 18, 24, 28, 33, 26, 27 and 30; such as the group consisting of SEQ ID NO:22, 16, 21, 29, 17, 25, 20, 23, 18, 24, 28, 33, 26, 27 and 30; such as the group consisting of SEQ ID NO:16, 21, 29, 17, 25, 20, 23, 18, 24, 28, 33, 26, 27 and 30; such as the group consisting of SEQ ID NO:17, 25, 20, 23, 18, 24, 28, 33, 26, 27 and 30; such as the group consisting of SEQ ID NO:25, 20, 23, 18, 24, 28, 33, 26, 27 and 30; such as the group consisting of SEQ ID NO:20, 23, 18, 24, 28, 33, 26, 27 and 30; such as the group consisting of SEQ ID NO:24, 28, 33, 26, 27 and 30; such as the group consisting of SEQ ID NO:28, 33, 26, 27 and 30; such as the group consisting of SEQ ID NO:26, 27 and 30. In one embodiment, the amino acid sequence of the polypeptide comprises an amino acid sequence according to SEQ ID NO:30. In some embodiments of the first, the second and the third aspects of the disclosure, the amino acid sequence of the polypeptide comprises an amino acid sequence selected from the group consisting of SEQ ID NO:19, 22, 16, 21, 29, 17, 25, 20, 23, 18, 24, 28, 33, 26 and 27; such as the group consisting of SEQ ID NO:22, 16, 21, 29, 17, 25, 20, 23, 18, 24, 28, 33, 26 and 27; such as the group consisting of SEQ ID NO:16, 21, 29, 17, 25, 20, 23, 18, 24, 28, 33, 26 and 27; such as the group consisting of SEQ ID NO:17, 25, 20, 23, 18, 24, 28, 33, 26 and 27; such as the group consisting of SEQ ID NO:25, 20, 23, 18, 24, 28, 33, 26 and 27; such as the group consisting of SEQ ID NO:20, 23, 18, 24, 28, 33, 26 and 27; such as the group consisting of SEQ ID NO:24, 28, 33, 26 and 27; such as the group consisting of SEQ ID NO:28, 33, 26 and 27; such as the group consisting of SEQ ID NO:26 and 27. In some embodiments of the first, the second and the third aspects of the disclosure, the amino acid sequence of the polypeptide comprises an amino acid sequence selected from the group consisting of SEQ ID NO:25, 20, 23, 18, 24, 28 and 33; such as the group consisting of SEQ ID NO:20, 23, 18, 24, 28 and 33; such as the group consisting of SEQ ID NO:23, 18, 24, 28 and 33; such as the group consisting of SEQ ID NO:18 and 24. In one embodiment, the amino acid sequence of the polypeptide comprises an amino acid sequence according to SEQ ID NO:18. In some embodiments of the first, the second and the third aspects of the disclosure, the amino acid sequence of the polypeptide comprises an amino acid sequence selected from the group consisting of SEQ ID NO:18, 28, 33, 26 and 27; such as the group consisting of SEQ ID NO:28, 33, 26 and 27; such as the group consisting of SEQ ID NO:28, 33 and 27; such as the group consisting of SEQ ID NO:33 and 27. In some embodiments of the first, the second and the third aspects of the disclosure, the amino acid sequence of the polypeptide comprises an amino acid sequence selected from the group consisting of SEQ ID NO:22, 16, 21, 29, 17, 25, 20, 23, 18, 24, 28, 33, 26, 27, 30, 31 and 32; such as the group consisting of SEQ ID NO:16, 21, 29, 17, 25, 20, 23, 18, 24, 28, 33, 26, 27, 30, 31 and 32; such as the group consisting of SEQ ID NO:17, 25, 20, 23, 18, 24, 28, 33, 26, 27, 30, 31 and 32; such as the group consisting of SEQ ID NO:25, 20, 23, 18, 24, 28, 33, 26, 27, 30, 31 and 32; such as the group consisting of SEQ ID NO:20, 23, 18, 24, 28, 33, 26, 27, 30, 31 and 32; such as the group consisting of SEQ ID NO:23, 18, 24, 28, 33, 26, 27, 30, 31 and 32; such as the group consisting of SEQ ID NO:18, 24, 28, 33, 26, 27, 30, 31 and 32. In some embodiments of the first, the second and the third aspects of the disclosure, the amino acid sequence of the polypeptide comprises an amino acid sequence selected from the group consisting of SEQ ID NO:19, 22, 16, 21, 29, 17, 25, 20, 23, 18, 24, 28, 33, 26, 27, 31 and 32; such as the group consisting of SEQ ID NO:22, 16, 21, 29, 17, 25, 20, 23, 18, 24, 28, 33, 26, 27, 31 and 32; such as the group consisting of SEQ ID NO:16, 21, 29, 17, 25, 20, 23, 18, 24, 28, 33, 26, 27, 31 and 32; such as the group consisting of SEQ ID NO:17, 25, 20, 23, 18, 24, 28, 33, 26, 27, 31 and 32; such as the group consisting of SEQ ID NO:25, 20, 23, 18, 24, 28, 33, 26, 27, 31 and 32; such as the group consisting of SEQ ID NO:20, 23, 18, 24, 28, 33, 26, 27, 31 and 32; such as the group consisting of SEQ ID NO:23, 18, 24, 28, 33, 26, 27, 31 and 32; such as the group consisting of SEQ ID NO:18, 24, 28, 33, 26, 27, 31 and 32. In some embodiments of the first, the second and the third aspects of the disclosure, the amino acid sequence of the polypeptide comprises an amino acid sequence selected from the group consisting of SEQ ID NO:25, 20, 23, 18, 24, 28, 31, 32 and 33; such as the group consisting of SEQ ID NO:20, 23, 18, 24, 28, 31, 32 and 33; such as the group consisting of SEQ ID NO:23, 18, 24, 28, 31, 32 and 33; such as the group consisting of SEQ ID NO:18, 31, 32 and 24. In some embodiments of the first, the second and the third aspects of the disclosure, the amino acid sequence of the polypeptide comprises an amino acid sequence selected from the group consisting of SEQ ID NO:18, 28, 33, 31, 32, 26 and 27; such as the group consisting of SEQ ID NO:28, 33, 31, 32, 26 and 27; such as the group consisting of SEQ ID NO:28, 33, 31, 32 and 27; such as the group consisting of SEQ ID NO:33, 31, 32, and 27. In some embodiments of the first, the second and the third aspects of the disclosure, the amino acid sequence of the polypeptide comprises an amino acid sequence selected from the group consisting of SEQ ID NO:18, 26-28, 31 and 32; such as the group consisting of SEQ ID NO:26-28, 31 and 32; such as the group consisting of SEQ ID NO:27, 28, 31 and 32; such as the group consisting of SEQ ID NO:27, 31 and 32; such as the group consisting of SEQ ID NO:31 and 32. In some embodiments of the first, the second and the third aspects of the disclosure, the amino acid sequence of the polypeptide comprises an amino acid sequence selected from the group consisting of SEQ ID NO:18, 26-28 and 31; such as the group consisting of SEQ ID NO:26-28 and 31; such as the group consisting of SEQ ID NO:27, 28 and 31; such as the group consisting of SEQ ID NO:27 and 31. In one embodiment, the amino acid sequence of the polypeptide comprises an amino acid sequence according to SEQ ID NO:31.

In some embodiments of the first, the second or the third aspect of the present disclosure, the polypeptide derived from SpA or any domain thereof, said Sequence A, said Sequence B, said Sequence C or said Sequence D as defined above "forms part of" a three-helix bundle protein domain. This is understood to mean that the sequence of the SpA derived polypeptide, said Sequence A, said Sequence B or said Sequence C is "inserted" into or "grafted" onto the sequence of the original three-helix bundle domain, such that the grafted sequence replaces a similar structural motif in the original domain. As the skilled person will realize, the replacement has to be performed so as not to affect the basic structure of the polypeptide. That is, the overall folding of the backbone of the polypeptide according to this embodiment of the invention is substantially the same as that of the three-helix bundle protein domain of which it forms a part, e.g. having the same elements of secondary structure in the same order etc. Thus, for example, a Sequence A according to the disclosure "forms part" of a three-helix bundle domain if the polypeptide according to this embodiment has the same fold as the original domain, implying that the basic structural properties are shared, those properties e.g. resulting in similar CD spectra. The skilled person is aware of other parameters that are relevant.

Thus, in some embodiments of the first, the second or the third aspect of the present disclosure, said polypeptide derived from SpA or any domain thereof, said Sequence A, said Sequence B, said Sequence C or said Sequence D forms part of a three-helix bundle protein domain. In some embodiments, said three-helix bundle protein domain is selected from bacterial receptor domains. In some embodiments, said three-helix bundle protein domain is selected from domains of protein A from Staphylococcus aureus (SpA) or derivatives thereof.

In a fourth aspect of the present disclosure, there is provided a polypeptide multimer, wherein each monomer of the multimer comprises a polypeptide which is independently selected from any polypeptide defined in any one the embodiments of the first, second and third aspects. In some embodiments, the polypeptide multimer further comprises at least one linker. The term "linker" herein means an element linking two polypeptide units, monomers or domains to each other in a multimer. The linker may comprise up to 15 amino acid residues.

In some embodiments, the multimer is selected from the group consisting of dimer, trimer, tetramer, pentamer, hexamer, heptamer, octamer, nonamer and decamer. In some embodiments, the multimer is selected from the group consisting of tetramer and hexamer. In one embodiment, said multimer is a hexamer. In one embodiment, the multimer is a homomer. In some embodiments, the multimer is a multimer, such as a hexamer, of an amino acid sequence selected from the group consisting of SEQ ID NO:19, 22, 16, 21, 29, 17, 25, 20, 23, 18, 24, 28, 33, 26, 27 and 30; such as the group consisting of SEQ ID NO:22, 16, 21, 29, 17, 25, 20, 23, 18, 24, 28, 33, 26, 27 and 30; such as the group consisting of SEQ ID NO:16, 21, 29, 17, 25, 20, 23, 18, 24, 28, 33, 26, 27 and 30; such as the group consisting of SEQ ID NO:17, 25, 20, 23, 18, 24, 28, 33, 26, 27 and 30; such as the group consisting of SEQ ID NO:25, 20, 23, 18, 24, 28, 33, 26, 27 and 30; such as the group consisting of SEQ ID NO:20, 23, 18, 24, 28, 33, 26, 27 and 30; such as the group consisting of SEQ ID NO:24, 28, 33, 26, 27 and 30; such as the group consisting of SEQ ID NO:28, 33, 26, 27 and 30; such as the group consisting of SEQ ID NO:26, 27 and 30. In one embodiment, the multimer is a multimer, such as a hexamer, of the amino acid sequence according to SEQ ID NO:30. In some embodiments, the multimer is a multimer, such as a hexamer, of an amino acid sequence selected from the group consisting of SEQ ID NO:19, 22, 16, 21, 29, 17, 25, 20, 23, 18, 24, 28, 33, 26 and 27; such as the group consisting of SEQ ID NO:22, 16, 21, 29, 17, 25, 20, 23, 18, 24, 28, 33, 26 and 27; such as the group consisting of SEQ ID NO:16, 21, 29, 17, 25, 20, 23, 18, 24, 28, 33, 26 and 27; such as the group consisting of SEQ ID NO:17, 25, 20, 23, 18, 24, 28, 33, 26 and 27; such as the group consisting of SEQ ID NO:25, 20, 23, 18, 24, 28, 33, 26 and 27; such as the group consisting of SEQ ID NO:20, 23, 18, 24, 28, 33, 26 and 27; such as the group consisting of SEQ ID NO:24, 28, 33, 26 and 27; such as the group consisting of SEQ ID NO:28, 33, 26 and 27; such as the group consisting of SEQ ID NO:26 and 27. In some embodiments, the multimer is a multimer, such as a hexamer, of an amino acid sequence selected from the group consisting of SEQ ID NO:25, 20, 23, 18, 24, 28 and 33; such as the group consisting of SEQ ID NO:20, 23, 18, 24, 28 and 33; such as the group consisting of SEQ ID NO:23, 18, 24, 28 and 33; such as the group consisting of SEQ ID NO:18 and 24. In one embodiment, the multimer is a multimer, such as a hexamer, of the amino acid sequence according to SEQ ID NO:18. In some embodiments, the multimer is a multimer, such as a hexamer, of an amino acid sequence selected from the group consisting of SEQ ID NO:18, 28, 33, 26 and 27; such as the group consisting of SEQ ID NO:28, 33, 26 and 27; such as the group consisting of SEQ ID NO:28, 33 and 27; such as the group consisting of SEQ ID NO:33 and 27. In some embodiments, the multimer is a multimer, such as a hexamer, of an amino acid sequence selected from the group consisting of SEQ ID NO:22, 16, 21, 29, 17, 25, 20, 23, 18, 24, 28, 33, 26, 27, 30, 31 and 32; such as the group consisting of SEQ ID NO:16, 21, 29, 17, 25, 20, 23, 18, 24, 28, 33, 26, 27, 30, 31 and 32; such as the group consisting of SEQ ID NO:17, 25, 20, 23, 18, 24, 28, 33, 26, 27, 30, 31 and 32; such as the group consisting of SEQ ID NO:25, 20, 23, 18, 24, 28, 33, 26, 27, 30, 31 and 32; such as the group consisting of SEQ ID NO:20, 23, 18, 24, 28, 33, 26, 27, 30, 31 and 32; such as the group consisting of SEQ ID NO:23, 18, 24, 28, 33, 26, 27, 30, 31 and 32; such as the group consisting of SEQ ID NO:18, 24, 28, 33, 26, 27, 30, 31 and 32. In some embodiments, the multimer is a multimer, such as a hexamer, of an amino acid sequence selected from the group consisting of SEQ ID NO:19, 22, 16, 21, 29, 17, 25, 20, 23, 18, 24, 28, 33, 26, 27, 31 and 32; such as the group consisting of SEQ ID NO:22, 16, 21, 29, 17, 25, 20, 23, 18, 24, 28, 33, 26, 27, 31 and 32; such as the group consisting of SEQ ID NO:16, 21, 29, 17, 25, 20, 23, 18, 24, 28, 33, 26, 27, 31 and 32; such as the group consisting of SEQ ID NO:17, 25, 20, 23, 18, 24, 28, 33, 26, 27, 31 and 32; such as the group consisting of SEQ ID NO:25, 20, 23, 18, 24, 28, 33, 26, 27, 31 and 32; such as the group consisting of SEQ ID NO:20, 23, 18, 24, 28, 33, 26, 27, 31 and 32; such as the group consisting of SEQ ID NO:23, 18, 24, 28, 33, 26, 27, 31 and 32; such as the group consisting of SEQ ID NO:18, 24, 28, 33, 26, 27, 31 and 32. In some embodiments, the multimer is a multimer, such as a hexamer, of an amino acid sequence selected from the group consisting of SEQ ID NO:25, 20, 23, 18, 24, 28, 31, 32 and 33; such as the group consisting of SEQ ID NO:20, 23, 18, 24, 28, 31, 32 and 33; such as the group consisting of SEQ ID NO:23, 18, 24, 28, 31, 32 and 33; such as the group consisting of SEQ ID NO:18, 31, 32 and 24. In some embodiments, the multimer is a multimer, such as a hexamer, of an amino acid sequence selected from the group consisting of SEQ ID NO:18, 28, 33, 31, 32, 26 and 27; such as the group consisting of SEQ ID NO:28, 33, 31, 32, 26 and 27; such as the group consisting of SEQ ID NO:28, 33, 31, 32 and 27; such as the group consisting of SEQ ID NO:33, 31, 32, and 27. In some embodiments, the multimer is a multimer, such as a hexamer, of an amino acid sequence selected from the group consisting of SEQ ID NO:18, 26-28, 31 and 32; such as the group consisting of SEQ ID NO:26-28, 31 and 32; such as the group consisting of SEQ ID NO:27, 28, 31 and 32; such as the group consisting of SEQ ID NO:27, 31 and 32; such as the group consisting of SEQ ID NO:31 and 32. In some embodiments, the multimer is a multimer, such as a hexamer, of an amino acid sequence selected from the group consisting of SEQ ID NO:18, 26-28 and 31; such as the group consisting of SEQ ID NO:26-28 and 31; such as the group consisting of SEQ ID NO:27, 28 and 31; such as the group consisting of SEQ ID NO:27 and 31. In one embodiment, the multimer is a multimer, such as a hexamer, of the amino acid sequence according to SEQ ID NO 31. It is to be understood that the above multimers of said amino acid sequences may comprise further amino acids, such as e.g. a linker in between the monomers, and/or amino acid sequences at the N-terminal and/or C-terminal of the multimers. In some embodiments, the above multimers of said amino acid sequences do not comprise a linker in between the monomers but comprise amino acid sequences at the N-terminal and/or C-terminal of the multimers. Thus, in some embodiments, the multimer comprises an amino acid sequence selected from the group consisting of SEQ ID NO:50-54; such as the group consisting of SEQ ID NO:50-52 and 54; such as the group consisting of SEQ ID NO:50, 52 and 54; such as the group consisting of SEQ ID NO:50 and 54. In one embodiment, the multimer comprises the amino acid sequence according to SEQ ID NO:50. In particular embodiments, the multimer comprises an amino acid sequence selected from the group consisting of SEQ ID NO:45-49; such as the group consisting of SEQ ID NO:45-47 and 49; such as the group consisting of SEQ ID NO:45, 47 and 49; such as the group consisting of SEQ ID NO:45 and 49. In one embodiment, the multimer comprises the amino acid sequence according to SEQ ID NO:45.

In some embodiments, the polypeptide multimer has binding affinity to an Fc region of an immunoglobulin at pH 7.0, which binding affinity for said Fc region is essentially lost at and below pH 4.4. In some embodiments, the binding affinity for said Fc region is essentially lost at and below pH 4.4, or at and below pH 4.5, or at and below pH 4.6, or at and below pH 4.7, or at and below pH 4.8, or at and below pH 4.9, or at and below pH 5.0, or at and below pH 5.5, or at and below pH 6.0. In some embodiments, the binding affinity for said Fc region is essentially lost at and below pH 4.5, or at and below pH 4.6, or at and below pH 4.7, or at and below pH 4.8, or at and below pH 4.9, or at and below pH 5.0, or at and below pH 5.5, or at and below pH 6.0. Said binding affinity, in some embodiments, is essentially lost at and below pH 4.6, or at and below pH 4.7, or at and below pH 4.8, or at and below pH 4.9, or at and below pH 5.0, or at and below pH 5.5, or at and below pH 6.0. In other embodiments, said binding affinity is essentially lost at and below pH 4.7, or at and below pH 4.8, or at and below pH 4.9, or at and below pH 5.0, or at and below pH 5.5, or at and below pH 6.0. In some embodiments, said binding affinity is essentially lost at and below pH 4.8, or at and below pH 4.9, or at and below pH 5.0, or at and below pH 5.5, or at and below pH 6.0. In some embodiments, the binding affinity is essentially lost at and below pH 4.9, or at and below pH 5.0, or at and below pH 5.5, or at and below pH 6.0. In some embodiments, the binding affinity is essentially lost at and below pH 5.0, or at and below pH 5.5, or at and below pH 6.0. In some embodiments, the binding affinity is essentially lost at and below pH 5.5, or at and below pH 6.0. In some embodiments, the binding affinity is essentially lost at and below pH 6.0. In particular embodiments, the binding affinity is essentially lost at and below pH 5.0, or at and below pH 5.5. In one embodiment, the binding affinity is essentially lost at and below pH 4.5. In one embodiment, the binding affinity is essentially lost at and below pH 4.6. In one embodiment, the binding affinity is essentially lost at and below pH 4.7. In one embodiment, the binding affinity is essentially lost at and below pH 4.8. In one embodiment, the binding affinity is essentially lost at and below pH 4.9. In one embodiment, the binding affinity is essentially lost at and below pH 5.5. In one embodiment, the binding affinity is essentially lost at and below pH 5.6. In one embodiment, the binding affinity is essentially lost at and below pH 5.8. As demonstrated in Examples 2, 4 and 5, the binding affinity for the Fc region is essentially lost at and below pH 4.6, or at and below pH 4.7, or at and below pH 4.8, or at and below pH 4.9, or at and below pH 5.0, in certain embodiments. In particular embodiments, the binding affinity for the Fc region is essentially lost at and below a pH range of pH 4.5-5.5, such as a pH range of pH 4.6-5.5, such as a pH range of pH 4.6-5.0, such as a pH range of pH 4.8-5.0. In other particular embodiments, the binding affinity for the Fc region is essentially lost at and below a pH range of pH 4.5-5.0, such as a pH range of pH 4.5-4.9, such as a pH range of pH 4.5-4.8, such as a pH range of pH 4.5-4.7, such as a pH range of pH 4.5-4.6. In other particular embodiments, the binding affinity for the Fc region is essentially lost at and below pH 5.0. As discussed above in more detail, the loss of said binding affinity for the Fc region may be induced by electrostatic repulsion, such as electrostatic repulsion mediated by at least one amino acid selected from the group consisting of amino acids in positions in the amino acid sequences of the monomers of the polypeptide multimer corresponding to positions X₁₈, X₂₄ and X₂₈ in SEQ ID NO:7. In some embodiments, the immunoglobulin is IgG, such as IgG selected from the group consisting of IgG1, IgG2 and IgG4. In some embodiments, the IgG is IgG1, such as trastuzumab and/or adalimumab. It is to be understood that embodiments discussed above in the first, the second and the third aspects of the disclosure in relation to the features of the herein disclosed polypeptides are also relevant for the fourth aspect. For the sake of brevity, these are not repeated herein or just briefly mentioned.

Polypeptides and polypeptide multimers according to the present disclosure exhibit optimal alkali stability. In embodiments where the polypeptide or the polypeptide multimer is used for separation or isolation of immunoglobulins (such as immunoglobulins having an IgG isotype), high alkali stability will allow for use of highly alkaline conditions during cleaning, essential for long-term repeated use in a bioprocess separation setting. Alkali stability can be tested for example in Biacore using 10 min incubation cycles in 0.5 M NaOH up to 100 cycles. The incubation cycle may for example be at 22 +/- 2°C. As demonstrated in Example 3, 100 cleaning cycles with 0.5 M NaOH can be indicative of alkali stability by measuring retained binding capacity for the analyte, such as trastuzumab. As shown in this Example, polypeptide multimers of the present disclosure may exhibit at least 40%, such as at least 45%, such as at least 50%, such as at least 55%, such as at least 60%, such as at least 65%, such as at least 70%, such as at least 75%, residual binding capacity after about 100 cleaning cycles with 0.5 M NaOH.

The skilled person will understand that various modifications and/or additions can be made to the polypeptide or to the polypeptide multimer according to any aspect disclosed herein without departing from the scope of the present disclosure in order to tailor the polypeptide or the polypeptide multimer to a specific application. For example, in one embodiment there is provided the polypeptide or the polypeptide multimer that has been extended by and/or comprises additional amino acids at the C terminus and/or N terminus. Such polypeptide or such polypeptide multimer has one or more additional amino acid residues at the very first and/or the very last position(s) in the polypeptide chain, i.e. at the N- and/or C-terminus of the polypeptide or the polypeptide multimer. In some embodiments of the first, the second, the third and the fourth aspects of the disclosure, thus, the polypeptide or the polypeptide multimer comprises additional amino acids at the C-terminal and/or N-terminal end thereof. For example, the polypeptide or the polypeptide multimer may further comprise at the N-terminal end a plurality of amino acid residues originating from the cloning process or constituting a residue from a cleaved off signaling sequence. The number of additional amino acid residues at the N- and/or C-terminus of the polypeptide or the polypeptide multimer may e.g. be 15 or less, such as 10 or less or 5 or less. The number of additional amino acid residues may for example be one, two, three, four, five, six, seven, eight, nine, ten or more. Said additional amino acid(s) at the C-terminal and/or N-terminal end of the polypeptide or the polypeptide multimer may improve production, purification, stabilization and/or *in vitro* coupling of the polypeptide or the polypeptide multimer. Non-limiting exemplary N-terminal amino acid sequences for improved production yields are SEQ ID NO:42 and 43. Moreover, *in vitro* coupling may be performed using a C-terminal amino acid sequence according to SEQ ID NO:44, as shown and explained in Example 1 below. In some embodiments, said additional amino acid(s) improve(s) coupling of the polypeptide or the polypeptide multimer and are selected from the group consisting of one or more cysteine residues, a plurality of Lysine residues and a plurality of Histidine residues. The coupling element may e.g. be a single cysteine at the C-terminal end or a plurality of Histidine residues. An advantage of having a C-terminal cysteine is that endpoint coupling of the protein can be achieved through reaction of the cysteine thiol with an electrophilic group on a support. This provides excellent mobility of the coupled protein which is important for binding capacity. A plurality of Histidine residues may be six Histidine residues, i.e. a His6-tag.

In a fifth aspect of the present disclosure, there is provided a polynucleotide encoding the polypeptide as defined in any one of the embodiments of the first, second and third aspects of the disclosure, or the polypeptide multimer as defined in any one of the embodiments of the fourth aspect of the disclosure.

In a sixth aspect, there is provided an expression vector comprising the polynucleotide as defined in the fifth aspect of the present disclosure.

In a seventh aspect, there is provided a host cell comprising the expression vector as defined in the sixth aspect of the present disclosure.

In an eighth aspect, there is provided an adsorbent material comprising the polypeptide as defined in any one of the embodiments of the first, second and third aspects of the disclosure, or the polypeptide multimer as defined in any one of the embodiments of the fourth aspect of the disclosure, coupled to a solid support. As the skilled person will understand, the expressed polypeptide, in monomeric or multimeric form, should be purified to an appropriate extent before being immobilized to a support. Such purification methods are well known in the field, and the immobilization of protein-based ligands to supports is easily carried out using standard methods. Suitable methods and supports will be discussed below in more detail and are disclosed e.g. in WO16079033 which is incorporated herein by reference in its entirety.

The support may be a solid support and may optionally be a porous material. The support may be or comprise a surface onto which the polypeptide or the polypeptide multimer is coupled. Examples of such support materials include conventional protein-binding support and surfaces such as a chip, a plate, a well, and a sheet. The support may optionally be provided in other forms such as a fiber, a membrane, a fibrous matrix, a filter, a porous monolith, a particle or a bead, such as a gel bead as used in chromatography resins. In some embodiments, the solid support material may be selected from a particle, a bead, a fiber, a fibrous membrane, a filter, a sheet, a porous monolith, a chip, a plate and a well. Particles or beads can be porous or non-porous. Particles or beads may include magnetic beads. Supports in the form of beads or particles can be used as a packed bed or in a suspended form. Suspended forms include those known as expanded beds and pure suspensions, in which the particles or beads are free to move. In case of monoliths, packed bed and expanded beds, a separation procedure commonly follows conventional chromatography with a concentration gradient. In case of pure suspension, batch-wise mode will be used.

The support may be made of any suitable material, as outlined in more detail below. As a non-limiting example, a conventional affinity separation matrix is often of organic nature and based on polymers that expose a hydrophilic surface to the aqueous media used, i.e. expose hydroxy (-OH), carboxy (-COOH), carboxamido (CONH₂, possibly in N-substituted forms), amino (-NH₂, possibly in substituted form), oligo- or polyethylenoxy groups on their external surface and, if present, also on internal surfaces. With regard to specific materials, the support may comprise a polymeric material. Polymeric materials include materials of natural or synthetic polymers, and combinations thereof. For example, the support may comprise a polyhydroxy polymer, such as a polysaccharide. Examples of polysaccharides include e.g. dextran, starch, cellulose, pullulan, agar, agarose etc., including derivatives thereof. Polysaccharides are inherently hydrophilic with low degrees of nonspecific interactions, they provide a high content of reactive (activatable) hydroxyl groups and they are generally stable towards alkaline cleaning solutions used in bioprocessing. The support may comprise agar or agarose, such as crosslinked agarose. Such supports used in the present invention can easily be prepared according to standard methods, such as inverse suspension gelation (S Hjerten, Biochim Biophys Acta, 79(2), 393-398 (1964)). Alternatively, the base matrices are commercially available products, such as crosslinked agarose beads sold under the name of SEPHAROSE^{™} FF (Cytiva). In an embodiment, which is especially advantageous for large-scale separations, the support has been adapted to increase its rigidity using the methods described in US6602990 or US7396467, which are hereby incorporated by reference in their entirety, and hence renders the matrix more suitable for high flow rates. Synthetic polymers useful as material for the support include polyvinyl alcohol, polyhydroxyalkyl acrylates, polyhydroxyalkyl methacrylates, polyacrylamides, polymethacrylamides, etc. In case of hydrophobic polymers, such as matrices based on divinyl and monovinyl-substituted benzenes, the surface of the matrix can be hydrophilized to expose hydrophilic groups as defined above to a surrounding aqueous liquid. Such polymers are easily produced according to standard methods. As an alternative, a commercially available product, such as SOURCE^{™} (Cytiva) may be used. Alternatively, the solid support according to the invention comprises a support of inorganic nature, e.g. silica, zirconium oxide, etc.

The polypeptide or the polypeptide multimer may be attached to the support via known coupling techniques utilizing e.g. thiol, amino and/or carboxy groups present in the polypeptide or the polypeptide multimer. Bisepoxides, epichlorohydrin, CNBr, N-hydroxysuccinimide (NHS) etc. are well-known coupling reagents. Between the support and the polypeptide or the polypeptide multimer, a spacer molecule can be introduced, which improves the availability of the first polypeptide moiety and/or facilitates the chemical coupling of the polypeptide or the polypeptide multimer to the support. The term "spacer" herein means an element connecting a polypeptide or a polypeptide multimer to a support. Depending on the nature of the polypeptide or the polypeptide multimer and the coupling conditions, the coupling may be a random or multipoint coupling (e.g. via a plurality of Lysines or Histidines) or a single point coupling (e.g. via a single cysteine). In embodiments, to increase coupling control, it may be preferable that the polypeptide or the polypeptide multimer variant as such does not comprise any Histidine residues. However also in such cases, the polypeptide or the polypeptide multimer as a whole may comprise a Histidine tag, such as a His6 tag. Alternatively, the polypeptide or the polypeptide multimer may be attached to the support by non-covalent bonding, such as physical adsorption or biospecific adsorption. The polypeptide or the polypeptide multimer may be coupled to the support via thioether bonds. Methods for performing such coupling are well-known in this field and easily performed by the skilled person in this field using standard techniques and equipment. Thioether bonds are flexible and stabile and generally suited for use in affinity chromatography. In particular when the thioether bond is via a terminal or near-terminal cysteine residue on the polypeptide or polypeptide multimer, the mobility of the coupled polypeptide or polypeptide multimer is enhanced which provides improved binding capacity and binding kinetics. In some embodiments the polypeptide or polypeptide multimer is coupled via a C-terminal cysteine provided on the polypeptide or the polypeptide multimer, as described above. This allows for efficient coupling of the cysteine thiol to electrophilic groups, e.g. epoxide groups, halohydrin groups etc. on a support, resulting in a thioether bridge coupling.

In embodiments, the polypeptide or the polypeptide multimer may be coupled to a support which is a convection-based chromatography matrix. Such convection-based chromatography matrix may comprise a porous polymer membrane, a filter, a fibrous matrix, or a porous monolith. Examples of a porous polymer membrane include MustangTM membranes (Cytiva) and SartobindTM membranes (Sartorius). A fibrous support may be based on electrospun polymeric fibers or cellulose fibers, optionally non-woven fibers. A fibrous matrix may thus be a non-woven fibrous matrix. The fibers may have a cross-sectional diameter of 10-1000 nm, such as 200-800 nm, 200-400 nm or 300-400 nm. Such a fibrous support can be found in a HiTrap Fibro^{™} unit (Cytiva). Alternative fibrous supports are disclosed in e.g. WO2019/137869 and WO2018/011600.

An adsorbent material as described herein may be used for the same purposes as mentioned above for the polypeptide or the polypeptide multimer. Thus, in one embodiment, the present disclosure provides a chromatography material comprising a separation matrix coupled to polypeptides or multimers as disclosed herein, as well as chromatography columns or devices incorporating such separation matrix. In a particular embodiment, the solid support is thus a separation matrix. In one embodiment, the adsorbent material may be a sensor surface intended for use in a sensor or other detection or quantification devices.

In a ninth aspect of the present disclosure, there is provided a method of isolating an immunoglobulin or a fragment thereof, said method comprising a) contacting a liquid sample comprising said immunoglobulin or a fragment thereof with the adsorbent material as defined in any one of the embodiments of the eighth aspect above. The immunoglobulin may be IgG, such as IgG selected from the group consisting of IgG1, IgG2 and IgG4. In some embodiments, the IgG is IgG1. As discussed above in relation to the first aspect of the disclosure, the fragment comprises the Fc fragment of the immunoglobulin. Moreover, it is to be understood that any polypeptide construct, such as a fusion protein, which comprises at least said Fc region and has maintained its binding property to the polypeptide or the polypeptide multimer of the present disclosure can be isolated according to the ninth aspect.

It is to be understood, that the contacting is performed under conditions under which the target entity can bind to the polypeptide or the polypeptide multimer moiety. The method may furthermore comprise washing the adsorbent material with a washing liquid, eluting the target entity from the adsorbent material with an elution buffer, and/or optionally cleaning the adsorbent material with a cleaning liquid. The cleaning liquid can alternatively be called a cleaning-in-place (CIP) liquid. As shown in Examples 4 and 5, a method according to the ninth aspect of the present disclosure may comprise eluting the immunoglobulin or the fragment thereof from the adsorbent material with an elution buffer which has a pH within a pH range of pH 4.5-5.5, such as a pH range of pH 4.6-5.5, such as a pH range of pH 4.6-5.0, such as a pH range of pH 4.8-5.0. In some embodiments, the elution buffer has a pH within a pH range of pH 4.5-5.5, such as a pH range of pH 4.6-5.4, such as a pH range of pH 4.7-5.3, such as a pH range of pH 4.8-5.2, such as a pH range of pH 4.9-5.1. In a particular embodiment, the pH of the elution buffer is pH 5.0. The cleaning liquid typically is an alkaline solution, such as comprising NaOH or KOH of at least 0.05 M, such as 0.05-1 M, such as 0.05-0.5 M, such as at least 0.1 M, such as 0.1-0.5 M, e.g. 0.3 M or 0.5 M. The contact (incubation) time may be at least 10 min. The binding, eluting and cleaning steps may advantageously be repeated at least 10 times, such as at least 20 times.

The skilled person will understand that the liquid sample to be purified may be any sample comprising the target entity in an environment from which it is to be purified or separated. The sample may be obtained from a cell culture, such as a clarified cell culture harvest, and may have been subjected to one or more conventional steps of filtration, concentration, dilution and/or buffer exchange, and optionally one of more initial chromatography steps, in particular a step which is not based on affinity chromatography, such as ion exchange chromatography, hydrophobic interaction chromatography, multimodal chromatography, or size exclusion chromatography. For example, the sample may be a clarified and filtered cell culture harvest. In some cases the sample may have been subjected to one or more steps of filtration by tangential flow filtration (TFF). Prior to contacting with the present absorbent material, liquid sample contains the target entity and at least one impurity, such as host cell protein (HCP) or host cell nucleic acids. The present adsorbent material is useful for separating the target entity from such impurities, and after performing the above process, the eluate containing the target entity has a reduced level of at least one such impurity.

Optionally, after performing a separation based on affinity capture as described herein, the eluate containing purified target entity may be subjected to one or more steps of filtration, concentration, dilution or buffer exchange, or chromatography step(s) not based on affinity chromatography, such as ion exchange chromatography, hydrophobic interaction chromatography, multimodal chromatography, or size exclusion chromatography. A further chromatography step performed after the affinity separation of the present disclosure may be referred to as a polishing step.

While the invention has been described with reference to various exemplary aspects and embodiments, it will be understood by those skilled in the art that various changes may be made and equivalents may be substituted for elements thereof without departing from the scope of the invention. In addition, many modifications may be made to adapt a particular situation or molecule to the teachings of the invention without departing from the essential scope thereof. Therefore, it is intended that the invention not be limited to any particular embodiment contemplated, but that the invention will include all embodiments falling within the scope of the appended claims.

### Brief description of the drawings

Figure 1A and 1B show representative sensorgrams of association and dissociation profiles of seven polypeptides according to the present disclosure (SEQ ID NO:35-41) and a reference polypeptide (SEQ ID NO:34), as measured in Biacore using trastuzumab and adalimumab as analyte.
Figure 2A and 2B show representative sensorgrams of association and dissociation profiles of seven polypeptides according to the present disclosure (SEQ ID NO:35-41) and a reference polypeptide (SEQ ID NO:34) at various elution pH, as measured in Biacore using trastuzumab as analyte.
Figure 3A, 3B and 3C show representative chromatograms of step elution at pH 4.6, pH 4.8 and pH 5.0, respectively, of adalimumab on columns packed with resin coupled to hexameric constructs of either SEQ ID NO:50, 52 or 54 in comparison to a reference resin.
Figure 4 shows representative chromatograms of step elution at pH 4.6, 4.8 and 5.0 of adalimumab on column packed with resin coupled to the hexameric construct SEQ ID NO:50.

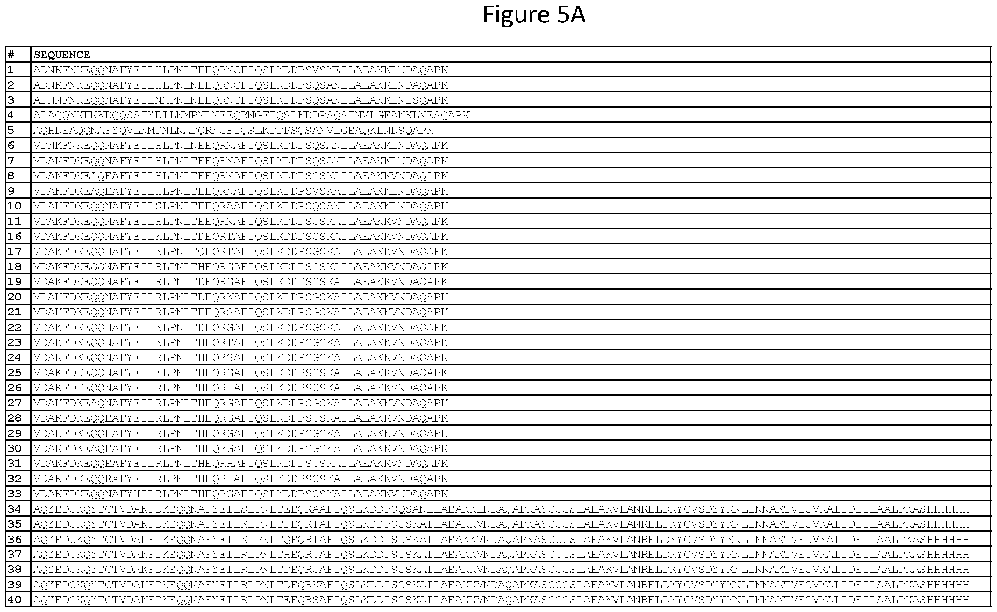

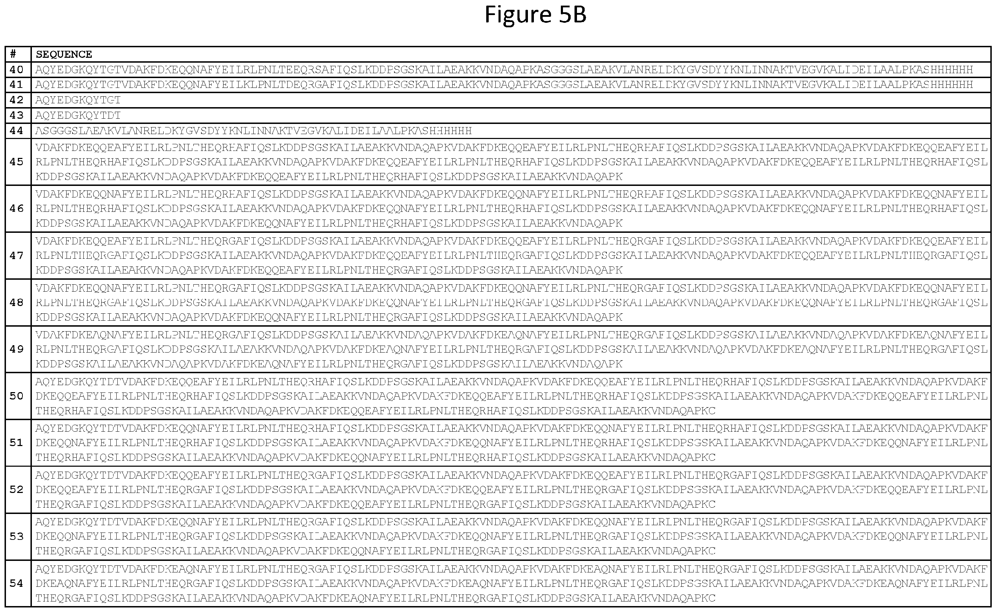

Figure 5A and 5B is a listing of amino acid sequences and corresponding SEQ ID NOs (#) of examples of polypeptides and polypeptide multimers according to the present disclosure as well as reference sequences as described in the Examples and amino acid sequences of domain A, B, C, D and E of SpA and derivatives thereof.

### EXAMPLES

This project aimed for providing protein ligands, i.e. novel polypeptides and multimers thereof, that enable mild elution of their bound target molecules, which bind to these polypeptides and multimers at pH 7.0. As discussed above in detail, mild conditions for elution are advantageous for preventing or decreasing aggregation of the bound target molecules. These conditions include elution at and below a pH range of pH 4.4-6.0, such as a pH range of pH 4.5-6.0, such as a pH range of pH 5.0-6.0, such as a pH range of pH 5.5-6.0. The inventors have found three amino acid positions (corresponding to position X₁₈, X₂₄ and X₂₈ in SEQ ID NO:7) in polypeptides deriving from SpA domains that are surprisingly advantageous for generating protein ligands enabling mild elution. Certain amino acid variants and the combination thereof in these positions were found particularly beneficial (Table 1):

**Table 1: Amino acid variants in positions beneficial for mild elution**

| | | |
|---|---|---|
| 18 | 24 | 28 |
| K | D | T |
| R | Q | G |
| | H | K |
| | E | S |
| | | H |

The inventors have found that using these amino acid positions, and in particular, the listed amino acid variants, milder elution pH can be achieved by increasing the effect of electrostatic repulsion between the novel polypeptides (and multimers thereof) and the target molecule. In particular, by introducing at least one of these variants on SpA-derived polypeptides, a stronger net repulsion can be created as these will be positioned opposite to Histidine (H) residues on the target molecule.

In the following non-limiting examples, the inventors demonstrate the suitability of the herein disclosed novel polypeptides and multimers thereof for mild elution as well as present further amino acid substitutions that may be introduced for additional beneficial properties.

### Example 1

### Biacore evaluation of novel polypeptide monomers for mild elution

Based on the above amino acid variants, seven polypeptides (SEQ ID NO:35-41) comprising SEQ ID NO:16-22, respectively, were constructed and characterized as described below.

The polypeptides (SEQ ID NO:35-41) were analysed in Biacore using trastuzumab and adalimumab as analytes and a reference polypeptide (SEQ ID NO:34) comprising SEQ ID NO:10 as control. Affinity, elution profiles at pH 5.0, pH 5.5 and pH 6.0, and alkaline stability were evaluated.

### Material and Methods

### Generation of the novel and reference polypeptides with ABD-His-tag

Nucleic acid sequences encoding SEQ ID NO:16-22 and SEQ ID NO:10 were cloned into pAM183 expression vector for production of the seven novel and the one reference polypeptides as ABD-His-tagged constructs (SEQ ID NO:34-41). Each ABD-His-tagged construct comprised a leader sequence (SEQ ID NO:42) at the N-terminal of the amino acid sequence selected from SEQ ID NO:16-22 and SEQ ID NO:10, and SEQ ID NO:44 (comprising a linker-ABD-tag-linker-His-tag motif) at the C-terminal thereof. For the avoidance of doubt, each construct consisted of SEQ ID NO:42-SEQ ID NO:X-SEQ ID NO:44, wherein SEQ ID NO:X corresponds to a sequence selected from SEQ ID NO:16-22 and SEQ ID NO:10. For protein production, materials and equipment used were as follows: glycerol stocks for the polypeptides; 2YT culture medium, Carbenicillin, IPTG, 100 ml baffled glass shake flasks, Infors HT Shaking incubator, His-GraviTrap and kit (Cytiva), PD10 GraviTraps (Cytiva).

Protein expression culture medium (2YT medium supplemented with 200 µg/ml Carbenicillin) was prepared and added to baffled shake flasks (20 ml per flask). Each flask was inoculated with the corresponding glycerol stock and incubated at 37°C and 150 rpm until OD600 reached approximately 0.7, then IPTG was added to a final concentration of 1 mM. Thereafter, flasks were incubated for 20 hours at 27°C and 150 rpm shaking. For harvest, the cultures were pelleted and re-suspended in 5 ml PBS in Falcon tubes and incubated in 80°C water bath for 1 hour followed by pelleting of cell debris by centrifugation at 12000xg for 30 min. Imidazole was added to the lysates to achieve the concentration recommended by the manufacturer and the samples were purified using His GraviTraps kit according to kit instructions. Buffer exchange into PBS was performed using PD10 Gravitraps and kit instructions. Concentration of buffer exchanged samples was measured using NanoDrop (Thermo Scientific) according to manufact'rer's instructions. Following purification, samples were kept in refrigerator until all measurements performed and were then transferred to Eppendorf tubes and kept in freezer (-20 C).

### Evaluation of binding to trastuzumab and adalimumab using Biacore

Materials and equipment used were as follows: CM5 sensor chips (Cytiva), Biacore NHS coupling kit (Cytiva), polypeptide variants from above, Biacore 8K+ (Cytiva); 500 nM trastuzumab and 500 nM adalimumab as analytes, diluted in PBS+P (P=0.05% Tween 20).

Immobilization was performed using a standard method in Biacore software with coupling of the polypeptide variants in FC2 and activation/inactivation in FC1 on a CM5 sensor chip. The polypeptide variants were diluted in 10 mM acetate buffer (pH 5.0) at approximately 10 µg/ml. The immobilization levels did vary somewhat between different polypeptide variants (1500 to 1700 RU).

Channel 1 to channel 8 were immobilized with the polypeptides. The same chips were used for binding analyses, elution pH testing and alkali stability studies. Injections for each channel (cycles) were as follows: buffer, 500 nM trastuzumab and 500 mM adalimumab.

### Biacore method:

Running buffer: PBS+P
Flow rate: 10 µl/min
Sample injection: 600s over both Flow Cells (FC1 and FC2)
Dissociation time: 1200s
Regeneration: 10 mM Glycine-HCl pH 1.5, 30 µl/min, 2x30s

### Evaluation of elution pH using trastuzumab as analyte in Biacore

For testing at which pH trastuzumab binding is lost, the method below was repeated three times using different elution buffers (50 mM citrate buffer with pH 6.0, pH 5.5 and pH 5.0).

Materials and equipment used were as follows: the same CM5 sensor chips with the polypeptide variants from above, Biacore 8K+ (Cytiva); 500 nM trastuzumab, 50 mM citrate buffer with pH 6.0, pH 5.5 and pH 5.0.

### Biacore method:

Running buffer: PBS+P
Flow rate: 10 µl/min
Sample injection 1 (500 nM trastuzumab): 300s over both Flow Cells
Dissociation time 1: 300s
Sample injection 2 (50 mM citrate buffer, different pH each round): 300s over both Flow Cells
Regeneration: 10 mM Glycine-HCl pH 1.5, 30 µl/min, 2x30s

### Alkali stability (0.5 M NaOH) evaluation using Biacore

After binding analyses and elution pH testing were done, alkali stability tests were performed on the same CM5 sensor chips with the immobilized polypeptide variants from above.

Materials and equipment used were as follows: the same CM5 sensor chips with the polypeptide variants from above, Biacore 8K+(Cytiva); 500 nM trastuzumab as above, 0.5 M NaOH.

### Biacore method (cycle):

Running buffer: PBS+P
Flow rate: 10 µl/min
Sample injection 1 (500 nM trastuzumab): 200s over both Flow Cells
Dissociation time 1: 120s
Sample injection 2 (0.5 M NaOH): 600s over both Flow Cells
Regeneration: 10 mM Glycine-HCl pH 1.5, 30 µl/min, 2x30s
This cycle was repeated 100 times to follow stability of trastuzumab response values.

### Results and Conclusions

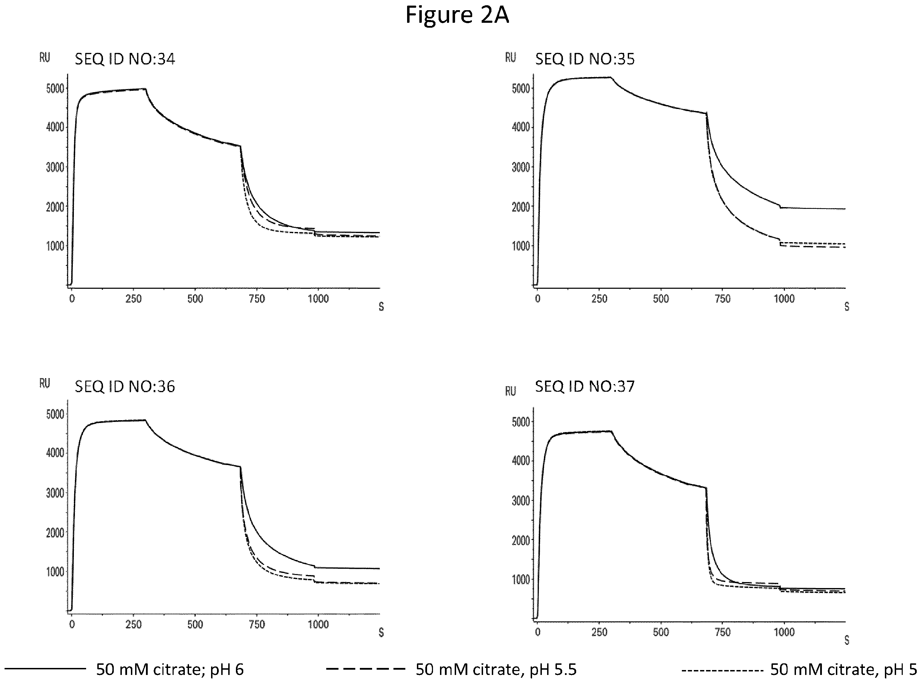

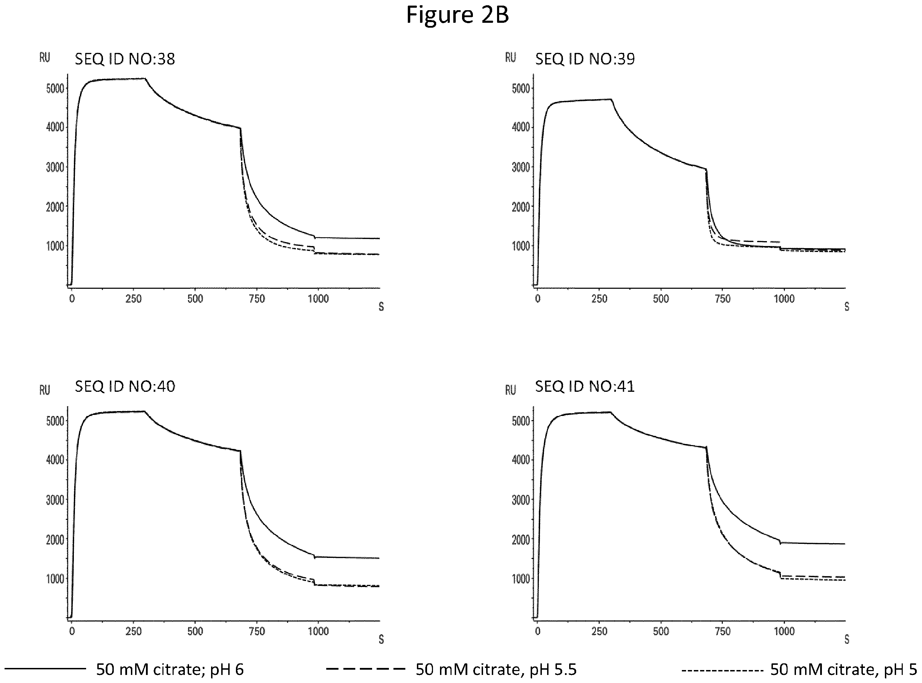

Representative sensorgrams of the binding evaluation and elution pH analysis of the seven tested polypeptides (SEQ ID NO:35-41) are shown in Fig. 1A and B and Fig. 2A and B together with the reference polypeptide (SEQ ID NO:34). All sensorgrams were generated as reference subtracted and the responses as the difference between baseline before injection and signal just before end of injection. In Table 2 below, results of the alkali stability test are presented.

**Table 2: Evaluation of alkali stability of SEQ ID NO:34-41 in Biacore. Retained binding capacity after 100 cycles using 0.5 M NaOH is shown (as % of the initial capacity). (SEQ ID NOs in parenthesis are sequence IDs of the corresponding sequences without the N- and C-terminal tags described above.)**

| SEQ ID NO | % of binding capacity after 100 cycles |
|---|---|
| 34 (10) | 31 |
| 35 (16) | 37 |
| 36 (17) | 39 |
| 37 (18) | 29 |
| 38 (19) | 34 |
| 39 (20) | 30 |
| 40 (21) | 37 |
| 41 (22) | 37 |

The binding analysis (Fig. 1A and B) demonstrates that each novel polypeptide (SEQ ID NO:35-41 comprising SEQ ID NO:16-22, respectively) had higher binding affinity for adalimumab than the binding affinity of the reference polypeptide (SEQ ID NO:34 comprising SEQ ID NO:10). The novel polypeptides (SEQ ID NO:35-41) also showed higher maximum RU values for adalimumab, which is considered indicative of higher binding capacity. Binding profiles of the novel polypeptides (SEQ ID NO:35-41) were similar for the two IgG types (trastuzumab and adalimumab). This demonstrates that the hereby shown advantageous binding properties are equally applicable for a diversity of IgG target molecules.

For testing elution pH, the novel and reference polypeptides (SEQ ID NO:34-41) were immobilized to the Biacore surface and trastuzumab was stepwise eluted off the surface by lowering pH to 6.0, 5.5 and 5.0 with a 50 mM citrate buffer. As shown in Fig. 2A and B, mild elution was detected for each tested novel polypeptide in Biacore, at least at pH 5.0 and pH 5.5. The mildest elution profiles, including elution at pH 6.0, were detected for SEQ ID NO:37 and 39 (comprising SEQ ID NO:18 and 20, respectively).

As shown in Table 2, alkali stability was comparable and acceptable among the tested polypeptides (SEQ ID NO:34-41).

In conclusion, the inventors have found that the above presented amino acid positions and selected variants are surprisingly beneficial for generating protein ligands that enable mild elution with improved binding affinity to IgG target molecules, with acceptable alkali stability.

### Example 2

### Evaluation of novel polypeptide monomers and multimers for mild elution on resin - dynamic binding capacity (DBC) and elution pH

This example illustrates DBC and elution pH characteristic for various polypeptide monomers and multimers according to the disclosure as measured by Biacore using trastuzumab as analyte.

### Material and Methods

### Generation of novel polypeptide monomers and multimers

Monomeric, tetrameric and hexameric constructs of SEQ ID NO:16-33 were ordered from ATUM (Newark, CA, USA) in pJ401 plasmid backbone with an N-terminal signal peptide, an N-terminal leader sequence (SEQ ID NO:43) and a C-terminal cysteine (C). Hexameric constructs of SEQ ID NO:18, 26-28 and 31 were produced without a linker in between the monomers, thus comprised SEQ ID NO:48, 46, 49, 47 and 45, respectively. Together with the N-terminal leader sequence (SEQ ID NO:43) and C-terminal cysteine, these hexameric constructs thus comprised SEQ ID NO:53, 51, 54, 52 and 50, respectively. For the sake of clarity, the monomeric constructs comprised SEQ ID NO:43-SEQ ID NO:X-C, wherein SEQ ID NO:X was selected from SEQ ID NO:16-33. The plasmids were transformed into chemically competent E. coli K12-017 cells and research cell-banks were made by inoculating 10 mL LB-broth with a single colony and growth for 5 h in 37°C. 1 L fermentation was performed for protein production, and the products of the fermentations were purified using IgG Sepharose 6FF (Cytiva) for capture, followed by a polishing step using Source 15Q (Cytiva). The purified protein was concentrated to approx. 50 g/L and stored frozen.

### Coupling and column packing

The monomeric and multimeric constructs were coupled to HFA25 base matrix (Cytiva) using epoxy chemistry. Ligand density of the coupled prototypes was measured with amino acid analysis.

To estimate slurry concentration, a resin slurry with 50 % concentration was prepared using a Falcon tube, settled overnight. A cube with a prefilter was filled with the slurry, connected to a pump, and subjected to vacuum. After drying, the cube was disassembled, and 0.2 M NaCl in 20% ethanol was added. The resin was then packed into Tricorn columns using a 0.2 M NaCl in 20% ethanol packing solution at various flow rates. The gel height was measured after the packing procedure. Ligand density of the coupled prototypes was measured with amino acid analysis (AAA).

### Dynamic binding capacity (DBC) measurement

For determining a 100% absorbance signal, PBS buffer was run through the bypass position until a stable baseline was reached. Followed by auto zeroing the system, trastuzumab was applied through bypass to obtain a stable 100% signal at 6 minutes RT and at a flow rate of 0.167 ml/min. The absorbance value was noted. Afterwards, PBS buffer was run through the bypass position until a stable baseline was reached.

### Buffers:

Adsorption/equilibration buffer: Phosphate buffer 20 mM + 0.15 M NaCl, pH 7.2 Elution buffer: Citrate buffer 50 mM, pH 2.5

### CIP: 0.1 M NaOH

Breakthrough capacity was calculated using the Extensions-DBC Calculations-Analyze tool in UNICORN 7.7 (Cytiva) according to manufacturer's instructions. Accordingly, breakthrough capacity at 10% was calculated, which equals to the amount of trastuzumab that is loaded onto the column until the concentration of trastuzumab in the column effluent is 10% of the trastuzumab concentration in the feed.

### pH elution gradient

pH elution gradients were performed in Tricorn 5/50 with 1 mL cubed resin at an approximate bed-height of 5 cm. Elution pH was determined by applying a small fraction of trastuzumab (approximately 5 mg) and record the pH of the elution peak in a citrate pH-gradient according to the following method.

| | |
|---|---|
| Buffer A1: | PBS |
| Buffer A2: | 50 mM sodium citrate pH=6.5 |
| Buffer B1: | 50 mM sodium citrate pH=3.5 |

| | |
|---|---|
| Equilibration: | 12 CV Buffer A1, flow; 1 ml/min |
| Sample applic.: | 2 ml trastuzumab at 1 ml/min |
| Wash: | 10 CV Buffer A2 at 1 mL/min |
| Elution: | 0-100% Buffer A2 to Buffer B1 in 20 CV at 1 mL/min |
| Re-equilib.: | 18 CV Buffer A1 at 1 mL/min |

### Results and Conclusions

Results for monomeric and multimeric constructs of SEQ ID NO:16-18, 20-31 and 33 coupled to HFA25 are presented below in Table 3:

**Table 3: Ligand density (LD), DBC (g/l) using trastuzumab of 6 min RT and 10% breakthrough, elution peak pH and pH of the pooled eluted fractions for monomeric and multimeric constructs of SEQ ID NO:16-18, 20-31 and 33 (N/A: not available). (SEQ ID NOs in parenthesis are sequence IDs of the corresponding hexamers with the N- and C-terminal tags described above.)**

| SEQ ID NO | Multimerization state | LD | DBC | pH of apex | pH in pool |
|---|---|---|---|---|---|
| 16 | monomer | 9.4 | 28 | 4.48 | 4.44 |
| 17 | monomer | 10.0 | 28 | 4.60 | 4.64 |
| 18 | monomer | 10.6 | 25 | 4.81 | 4.90 |
| 18 (53) | hexamer | 13.2 | 66.0 | 4.80 | N/A |
| 20 | monomer | 8.6 | 24 | 4.76 | N/A |
| 21 | monomer | 9.7 | 28 | 4.49 | 4.57 |
| 22 | monomer | 8.6 | 29 | 4.42 | 4.54 |
| 23 | monomer | 10.5 | 27,5 | 4.79 | N/A |
| 24 | monomer | 10.4 | 23,8 | 4.90 | N/A |
| 25 | monomer | 10.5 | 29,5 | 4.71 | N/A |
| 26 | monomer | 10.7 | 20 | 5.57 | N/A |
| 26 (51) | hexamer | 12.6 | 57.0 | 4.69 | N/A |
| 27 | monomer | 10.1 | 13 | 5.63 | N/A |
| 27 (54) | hexamer | 14.2 | 50.8 | 5.04 | N/A |
| 28 | monomer | 9.6 | 19 | 5.01 | N/A |
| 28 (52) | hexamer | 14.8 | 56.6 | 4.73 | N/A |
| 29 | monomer | 11.8 | 25 | 4.49 | N/A |
| 30 | monomer | 10.7 | 7 | 5.85 | N/A |
| 31 (50) | hexamer | 11.8 | 50.3 | 4.59 | N/A |
| 33 | monomer | 12.1 | 20 | 5.01 | N/A |

The data demonstrate suitability of the herein disclosed polypeptide monomers for mild elution on resin with at least pH 4.42 at the elution peak and at least pH 4.44 in the pool. Some monomers, such as monomeric constructs of SEQ ID NO:26, 27 and 30, showed exceptional performance with an elution pH of 5.57 or higher. The data also indicate (e.g. for the monomeric construct of SEQ ID NO:33) that certain amino acid variants in the amino acid position corresponding to position X₁₅ in SEQ ID NO:7 in polypeptides deriving from SpA domains may also be beneficial for generating protein ligands enabling mild elution.

The results for hexameric constructs in comparison to their respective monomers indicate that dynamic binding capacity increased with the ligand length (multimerization state of the polypeptide monomer). At the same time, increasing ligand length led to lower elution pH. The data show that elution pH for the tested multimeric constructs is at or above pH 4.59. The data also reveal that the tested hexamers performed with at least 50.3 g/l in DBC.

### Example 3

### Biacore evaluation of novel polypeptide monomers and multimers for mild elution - alkali stability

This example presents alkali stability of various polypeptide monomers and multimers according to the disclosure as measured by Biacore using trastuzumab as analyte. Alkali stability is assessed by retained binding capacity (% of initial capacity) after 100 wash cycles using 0.5 M NaOH.

### Material and Methods

Monomeric and multimeric constructs of SEQ ID NO:16-18, 20-31 and 33 (as listed below in Table 4) were produced and coupled to resin essentially as described in Example 2.

Alkali stability runs were carried out on Biacore 8K using trastuzumab as analyte and 10 min incubation cycles in 0.5 M NaOH up to 100 cycles, essentially as described in Example 1. Ligands were reduced and thereafter coupled on CM5 with ligand thiol coupling to mediate a C-terminal directed coupling (C-terminal cysteine).

### Results and Conclusions

Results of the alkali stability study for monomeric and hexameric constructs of SEQ ID NO:16-18, 20-31 and 33 is shown in Table 4:

**Table 4: Evaluation of alkali stability for monomeric and multimeric constructs of SEQ ID NO:16-18 and 21-33. Retained binding capacity after 100 cycles using 0.5 M NaOH is shown (in RU and also as % of the initial capacity; N/A: not available). (SEQ ID NOs in parenthesis are sequence IDs of the corresponding hexamers with the N- and C-terminal tags described above.)**

| SEQ ID NO | Multimerization state | RU | % of binding capacity after 100 cycles |
|---|---|---|---|
| 16 | monomer | 1384 | 34 |
| 17 | monomer | 1546 | 37 |
| 18 | monomer | 1119 | 20 |
| 20 | monomer | 1304 | 23 |
| 21 | monomer | 1235 | 30 |
| 22 | monomer | 1393 | 33 |
| 23 | monomer | 1453 | 22 |
| 24 | monomer | 1318 | 20 |
| 25 | monomer | 1127 | 23 |
| 26 | monomer | 1201 | 14 |
| 26 (51) | hexamer | N/A | 42 |
| 27 | monomer | 1355 | 24 |
| 27 (54) | hexamer | N/A | 65 |
| 28 | monomer | 1371 | 32 |
| 28 (52) | hexamer | N/A | 76 |
| 29 | monomer | 1445 | 35 |
| 30 | monomer | 1667 | 38 |
| 31 (50) | hexamer | N/A | 72 |
| 33 | monomer | 1713 | 17 |

The above Biacore results demonstrate that monomeric constructs of the herein disclosed novel polypeptides for mild elution retain at least 17% of initial binding capacity after 100 cycles using 0.5 M NaOH, while the tested multimeric constructs retain at least 42% of initial binding capacity after 100 cycles using 0.5 M NaOH. The data indicate that certain amino acid substitutions in positions corresponding to position X₉ and X₁₁ in SEQ ID NO:7 are particularly advantageous for improved alkali stability of the hereby disclosed novel polypeptide monomers and multimers for mild elution (for example as shown for monomeric constructs of SEQ ID NO:27-30) These amino acid variants include Alanine (A) in the position corresponding to position X₉ in SEQ ID NO:7. Glutamic acid (E) and Histidine (H) are also considered advantageous for improved alkali stability in the position corresponding to position X₁₁ in SEQ ID NO:7. As indicated in Table 4, certain combinations of amino acid variants in these positions corresponding to position X₉ and X₁₁ in SEQ ID NO:7 can be beneficial, however the inventors have surprisingly found that A in the position corresponding to position X₉ in SEQ ID NO:7 in combination with E in the position corresponding to position X₁₁ in SEQ ID NO:7 leads to reduced binding capacity to IgG (as shown in Table 3 for the monomeric construct of SEQ ID NO:30).

### Example 4

### Step elution of adalimumab on resin using hexameric constructs of SEQ ID NO:50, 52 and 54

A test with step elution at pH 4.6, 4.8 and 5.0 was performed on columns packed with resin coupled to hexamer constructs SEQ ID NO:50, 52 or 54 in comparison to a reference resin comprising a construct according to SEQ ID NO:51 in WO2022013272 (referred to herein as "reference" or "reference resin")..

### Material and Methods

Resins coupled to hexamer constructs SEQ ID NO:50, 52 or 54 were produced essentially as described in Example 2.

### Step elution

Resins coupled to hexamer constructs SEQ ID NO:50, 52 or 54 were packed in Tricorn 5/50 columns to a cubed volume of 1 ml resin. Equilibration was carried out using a buffer of 20 mM Phosphate and 150 mM NaCl, pH 7.2. The load was approximately 60-80% of Qb10, with 30-40 mg adalimumab. The column was washed with 50 mM acetate, pH 6.0. The elution was performed with 50 mM acetate, pH 4.6, 4.8 or 5.0 at 0.25 ml/min with a UV setting of 150 to 100 mAU and a max collection of 10 CV. All elutions with less than 4 CV were considered successful.

### Results and Conclusions

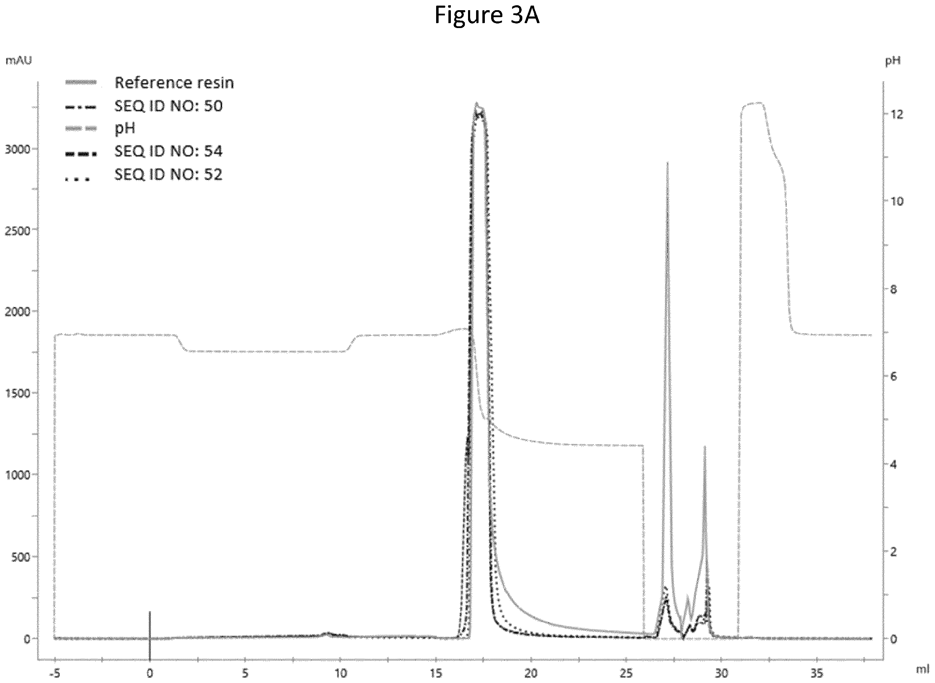

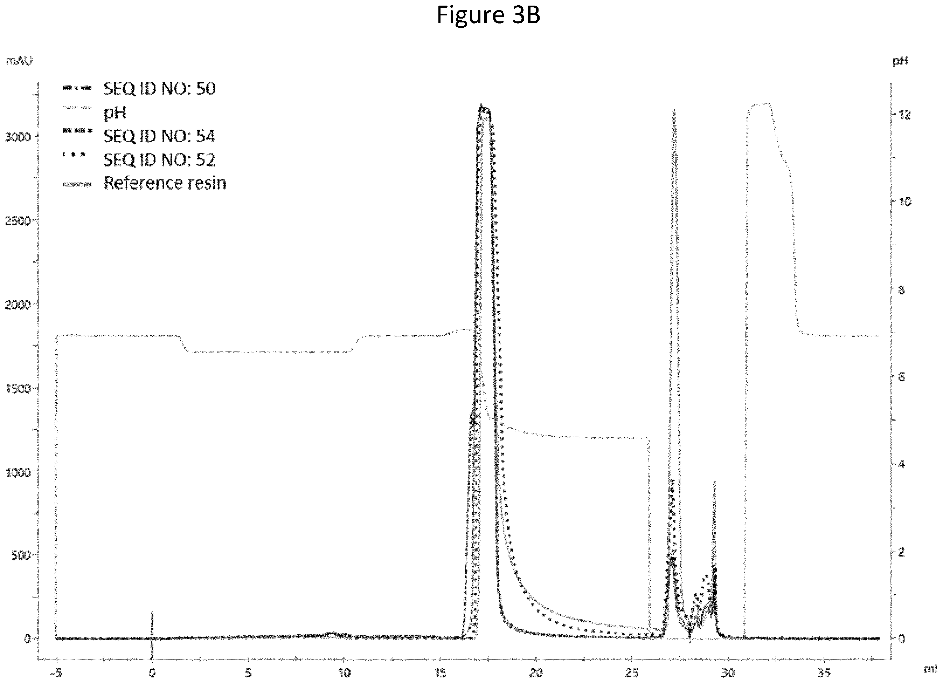

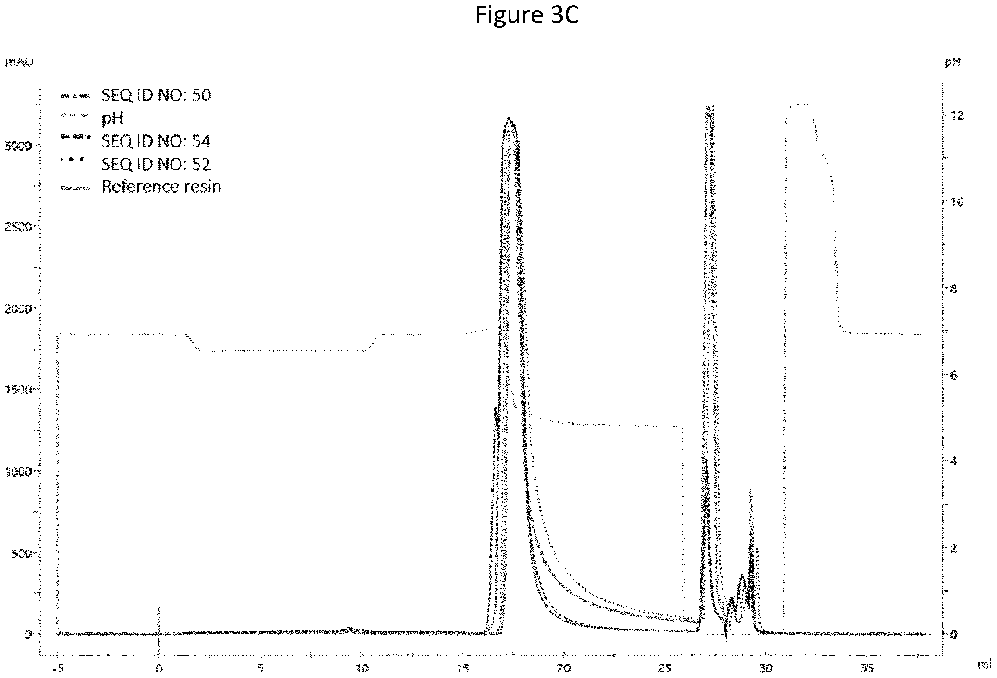

Results of the step elution study are presented in Fig. 3A, 3B and 3C together with Table 5 below:

**Table 5: Elution pool volumes in column volumes (CV) of different pH for resins coupled to hexamer constructs SEQ ID NO:50, 52 or 54 in comparison to the reference resin.**

| pH | SEQ ID NO:54 (CV) | SEQ ID NO:52 (CV) | SEQ ID NO:50 (CV) | Reference (CV) |
|---|---|---|---|---|
| 4.6 | 1.8 | 1.8 | 1.5 | 3.8 |
| 4.8 | 2.2 | 4 | 1.7 | 5.7 |
| 5.0 | 4.0 | 8.7 | 3.0 | 7.6 |

Step elution at pH 4.6 (Fig. 3A) shows that each novel polypeptide multimer could be fully eluted at pH 4.6. The reference resin did not completely elute which led to a high peak of 3000 mAU in the strip, following the elution peak.

Step elution at pH 4.8 (Fig. 3B) showed that the reference resin had a large pool volume and high peak in strip elution. In this chromatogram, it can also be seen that the elution profile which has been obtained using the resin coupled to the hexamer construct SEQ ID NO:52 had a larger pool volume (4 CV, Table 5) in comparison to those obtained by the other two resins coupled to hexamer constructs SEQ ID NO:50 or 54. It is demonstrated by peak broadening, the so-called tailing.

Step elution at pH 5.0. (Fig. 3C) showed that the elution profile which has been obtained using the reference resin and the resin coupled to the hexamer construct SEQ ID NO:52 did not reach the base-line and thus, a significant amount of the protein was eluted in the strip. The other two resins coupled to hexamer construct SEQ ID NO:50 or 54 reached base-line and the resin coupled to the hexamer construct SEQ ID NO:50 had the smallest pool volume (3 CV, Table 5).

The results showed that all three multimeric constructs performed better compared to the reference resin. The resin coupled to the hexamer construct SEQ ID NO:52 could be eluted up to pH 4.8, with a pool volume less or equal to 4 CV. The other two resins coupled to hexamer constructs SEQ ID NO:50 or 54 had a successful elution profile up to pH 5.0 with a pool volume less or equal to 4 CV.

### Example 5

### Step elution of adalimumab on resin using hexamer construct SEQ ID NO:50

Step elution at pH 4.6, 4.8 and 5.0 was performed on column packed with resin coupled to the hexamer construct SEQ ID NO:50.

### Material and Methods

Resin coupled to the hexamer construct SEQ ID NO:50 was produced essentially as described in Example 2.

### Step elution

Adalimumab cultivation in CHO cells was harvested with centrifugation and depth filtration. The resulting clarified cell culture feed (CCCF) contained 1.7 g/l adalimumab with ~3% aggregate. Resin coupled to the hexamer construct SEQ ID NO:50 was packed in Tricorn 5/100 columns to a cubed volume of 2 ml resin. The load was 70% of QB10, 37 mg antibody/ml resin. Followed by a purification protocol with, 20 mM Na-phosphate, 500 mM NaCl for high salt wash, 50 mM Acetate pH 6.0 for a second wash and elution with 50 mM Acetate pH 5.0 / 4.8 / 4.6, elution was collected from 150 mAU to 100 mAU.

### Size Exclusion Chromatography (SEC)

SEC was performed on a Superdex 200 Increase 10/300 GL column (Cytiva) using a 30-minute isocratic separation with PBS-buffer, pH 7.4. The column was mounted on an Agilent 1260 Infinity II Bio-LC system with a DAD-detector. Sample purity was evaluated at 214 nm. Purity was determined by manual integration of the high molecular weight (HMW) species, the main peak, and the low molecular weight (LMW) species in the chromatogram, where purity is given as the main peak area %.

### Results and Conclusions

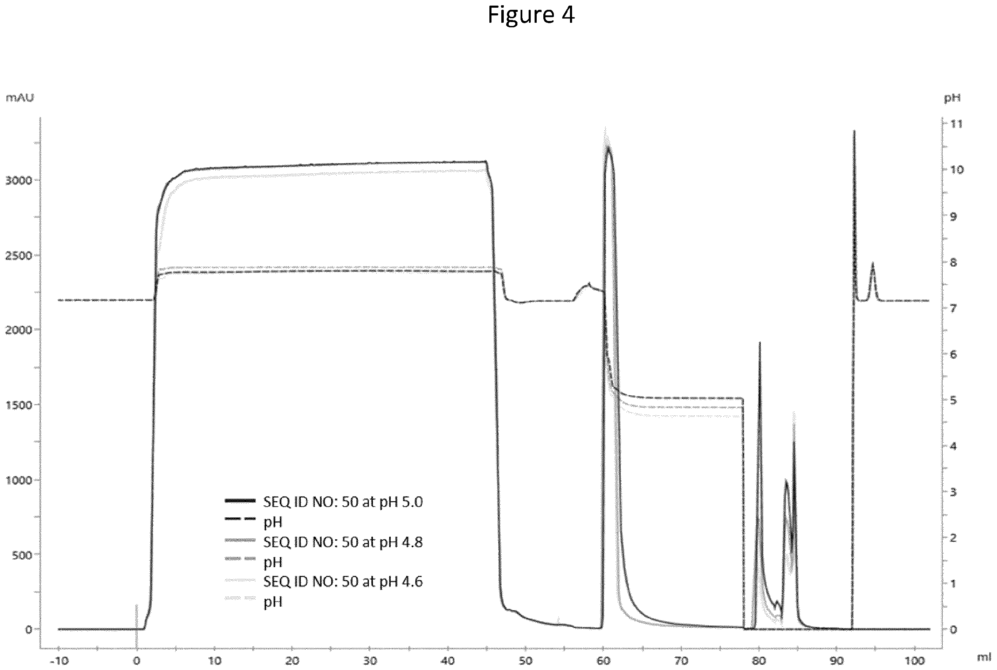

Results of the step elution study are presented in Fig. 4 together with Table 6 below:

**Table 6: Summary of data from SEC analysis, elution pool pH, volume and aggreates %. Concentration of the adalimumab monomer in eluates was calculated based on adalimumab standard curve. The yield was calculated based on the initial concentration (1.7 g/l) in the start material.**

| pH | Eluate conc. (g/l) | Pool volume (CV) | Elution pool (pH) | Yield (%) | Aggregate (%) |
|---|---|---|---|---|---|
| 5.0 | 13.7 | 2.5 | 5.1 | 93 | 3.2 |
| 4.8 | 23.4 | 1.5 | 5.0 | 95 | 5.8 |
| 4.6 | 22.2 | 1.6 | 4.8 | 93 | 6.3 |

Fig. 4 shows the purification of adalimumab on resin coupled to the hexamer construct SEQ ID NO:50. A sample of 44 mL clarified CCCF (about 1.7 g/l) was applied on Tricorn 5/100 columns at 6 min RT. Column wash was performed with 20 mM Na-phosphate, 500 mM NaCl, pH 7.4 followed by wash with 50 mM Na-acetate pH 6.0 followed by step elution with 50 mM Na-acetate, pH 5.0 (black), pH 4.8 (dark grey) and pH 4.6 (light grey). The results show that adalimumab elutes in a narrow elution peak at pH 4.6 and 4.8. Elution at pH 5.0 gives a larger elution pool of 2.5 CV (Table 6), hence a broader elution peak (Fig. 4). More protein elutes in the strip peak at pH 5.0, however the aggregate clearance is better (Table 6). The yield and elution pool volume are within specified limits of >90 % yield and <4 CV.

### ITEMIZED LIST OF EMBODIMENTS

1. A polypeptide derived from a Staphylococcus Protein A (SpA) or any domain thereof, wherein said polypeptide has binding affinity to an Fc region of an immunoglobulin at pH 7.0, which binding affinity for said Fc region is essentially lost at and below pH 4.4, or at and below pH 4.5, or at and below pH 4.6, or at and below pH 4.7, or at and below pH 4.8, or at and below pH 4.9, or at and below pH 5.0, or at and below pH 5.5, or at and below pH 6.0.
2. The polypeptide according to item 1, wherein said polypeptide has a higher binding affinity than SEQ ID NO:10 for adalimumab.
3. The polypeptide according to item 1 or 2, wherein said immunoglobulin is immunoglobulin G (IgG), such as IgG selected from the group consisting of IgG1, IgG2 and IgG4.
4. The polypeptide according to item 3, wherein said IgG is IgG1, such as trastuzumab and/or adalimumab.
5. The polypeptide according to any one of items 1-4, wherein said polypeptide has lower binding affinity for a VH3 region of trastuzumab compared to the binding affinity of SEQ ID NO:6 and/or SEQ ID NO:7 for the same VH3 region.
6. The polypeptide according to item 5, wherein said binding affinity for the VH3 region of trastuzumab is measured at pH 7.0.
7. The polypeptide according to any one of items 1-7, wherein said polypeptide comprises an amino acid sequence that has at least 70% identity to a sequence selected from the group consisting of SEQ ID NO:1-9, such as the group consisting of SEQ ID NO:1-8.
8. The polypeptide according to item 7, wherein for the purpose of determining said sequence identity, positions in the amino acid sequence corresponding to positions X₁₈, X₂₄, X₂₈; positions X₁₈, X₂₄, X₂₈, X₃₃, X₄₀, X₅₁; positions X₉, X₁₁, X₁₈, X₂₄, X₂₈; positions X₉, X₁₁, X₁₅, X₁₈, X₂₄, X₂₈, positions X₉, X₁₁, X₁₈, X₂₄, X₂₈, X₃₃, X₄₀, X₅₁, and/or positions X₉, X₁₁, X₁₅, X₁₈, X₂₄, X₂₈, X₃₃, X₄₀, X₅₁; in SEQ ID NO:7 are omitted.
9. The polypeptide according to any one of items 1-8, wherein the loss of said binding affinity for the Fc region is induced by electrostatic repulsion.
10. The polypeptide according to item 9, wherein the electrostatic repulsion is mediated by at least one amino acid selected from the group consisting of amino acids in positions in the amino acid sequence of the polypeptide corresponding to positions X₁₈, X₂₄ and X₂₈ in SEQ ID NO:7.
11. The polypeptide according to any one of items 1-10, wherein the polypeptide comprises an amino acid selected from R and K in a position in the amino acid sequence thereof, which position corresponds to position X₁₈ in SEQ ID NO:7.
12. The polypeptide according to any one of items 1-11, wherein the polypeptide comprises an amino acid selected from H, D, Q and E, such as from H and D, in a position in the amino acid sequence thereof, which position corresponds to position X₂₄ in SEQ ID NO:7.
13. The polypeptide according to any one of items 1-12, wherein the polypeptide comprises an amino acid selected from H, T, K, S and G, such as from H, K and G, such as from H and G, in a position in the amino acid sequence thereof, which position corresponds to position X₂₈ in SEQ ID NO:7.
14. A polypeptide derived from a Staphylococcus Protein A (SpA) or any domain thereof comprising an amino acid sequence, wherein said polypeptide comprises an amino acid selected from R and K in a position in the amino acid sequence thereof, which position corresponds to position X₁₈ in SEQ ID NO:7;
   an amino acid selected from H, D, Q and E, such as from H and D, in a position in said amino acid sequence, which position corresponds to position X₂₄ in SEQ ID NO:7; and
   an amino acid selected from H, T, K, S and G, such as from H, K and G, such as from H and G, in a position in said amino acid sequence, which position corresponds to position X₂₈ in SEQ ID NO:7.
15. The polypeptide according to item 14, wherein said polypeptide has binding affinity to an Fc region of an immunoglobulin at pH 7.0, which binding affinity for said Fc region is essentially lost at and below pH 4.4, or at and below pH 4.5, or at and below pH 4.6, or at and below pH 4.7, or at and below pH 4.8, or at and below pH 4.9, or at and below pH 5.0, or at and below pH 5.5, or at and below pH 6.0.
16. The polypeptide according to item 15, wherein the loss of said binding affinity for the Fc region is induced by electrostatic repulsion.
17. The polypeptide according to item 15, wherein the electrostatic repulsion is mediated by at least one amino acid selected from the group consisting of amino acids in positions in the amino acid sequence of the polypeptide corresponding to positions X₁₈, X₂₄ and X₂₈ in SEQ ID NO:7.
18. The polypeptide according to any one of items 14-17, wherein said polypeptide has a higher binding affinity than SEQ ID NO:10 for adalimumab.
19. The polypeptide according to any one of items 15-18, wherein said immunoglobulin is immunoglobulin G (IgG), such as IgG selected from the group consisting of IgG1, IgG2 and IgG4.
20. The polypeptide according to item 19, wherein said IgG is IgG1, such as trastuzumab and/or adalimumab.
21. The polypeptide according to any one of items 14-20, wherein said polypeptide has lower binding affinity for a VH3 region of trastuzumab compared to the binding affinity of SEQ ID NO:6 and/or SEQ ID NO:7 for the same VH3 region.
22. The polypeptide according to item 21, wherein said binding affinity for the VH3 region of trastuzumab is measured at pH 7.0.
23. The polypeptide according to any one of items 14-22, wherein the amino acid sequence of the polypeptide has at least 70% identity to a sequence selected from the group consisting of SEQ ID NO:1-9, such as the group consisting of SEQ ID NO:1-8.
24. The polypeptide according to item 23, wherein for the purpose of determining said sequence identity, positions in the amino acid sequence corresponding to positions X₁₈, X₂₄, X₂₈; positions X₁₈, X₂₄, X₂₈, X₃₃, X₄₀, X₅₁; positions X₉, X₁₁, X₁₈, X₂₄, X₂₈; positions X₉, X₁₁, X₁₅, X₁₈, X₂₄, X₂₈; positions X₉, X₁₁, X₁₈, X₂₄, X₂₈, X₃₃, X₄₀, X₅₁, and/or positions X₉, X₁₁, X₁₅, X₁₈, X₂₄, X₂₈, X₃₃, X₄₀, X₅₁; in SEQ ID NO:7 are omitted.
25. The polypeptide according to any one of items 11-24, wherein the amino acid in said position corresponding to position X₁₈ in SEQ ID NO:7 is R.
26. The polypeptide according to any one of items 11-25, wherein the amino acid in said position corresponding to position X₂₄ in SEQ ID NO:7 is H.
27. The polypeptide according to any one of items 11-26, wherein the amino acid in said position corresponding to position X₂₈ in SEQ ID NO:7 is H.
28. The polypeptide according to any one of items 1-27, wherein the polypeptide comprises
   an amino acid selected from T, S, G, Q, A, E, H, R, P, D, K and N, such as from S, A and E, in a position in the amino acid sequence thereof, which position corresponds to position X₃₃ in SEQ ID NO:7;
   an amino acid selected from V, E, G, R, D, K, Q, N, H and S, such as from E, G, R, D, K, Q, N, H and S, such as from G, R, E and D, in a position in the amino acid sequence thereof, which position corresponds to position X₄₀ in SEQ ID NO:7; and
   an amino acid selected from L, V, S, I, R and G, such as from V, L, I and R, in a position in the amino acid sequence thereof, which position corresponds to position X₅₁ in SEQ ID NO:7.
29. The polypeptide according to item 28, wherein the amino acid in said position corresponding to position X₃₃ in SEQ ID NO:7 is S.
30. The polypeptide according to item 28 or 29, wherein the amino acid in said position corresponding to position X₄₀ in SEQ ID NO:7 is G.
31. The polypeptide according to any one of items 28-30, wherein the amino acid in said position corresponding to position X₅₁ in SEQ ID NO:7 is V.
32. The polypeptide according to any one of items 1-31, wherein the polypeptide comprises
   an amino acid selected from E, N, H, R and S, such as from N, E, H and R, in a position in the amino acid sequence thereof, which position corresponds to position X₁₁ in SEQ ID NO:7; and/or
   an amino acid selected from A and Q in a position in the amino acid sequence thereof, which position corresponds to position X₉ in SEQ ID NO:7;
   preferably with the proviso that the amino acids in the positions corresponding to X₉X₁₁ in SEQ ID NO:7 are not AE.
33. The polypeptide according to item 32, wherein the amino acid in said position corresponding to position X₁₁ in SEQ ID NO:7 is selected from H, R and E, such as from Rand E.
34. The polypeptide according to item 32, wherein the amino acid in said position corresponding to position X₁₁ in SEQ ID NO:7 is selected from H, R and N, such as from N and H.
35. The polypeptide according to item 32, wherein the amino acid in said position corresponding to position X₁₁ in SEQ ID NO:7 is selected from N, Rand E, such as from Rand E.
36. The polypeptide according to item 32 or 35, wherein the amino acid in said position corresponding to position X₁₁ in SEQ ID NO:7 is selected from N and E.
37. The polypeptide according to any one of items 32 and 34-36, wherein the amino acid in said position corresponding to position X₁₁ in SEQ ID NO:7 is N.
38. The polypeptide according to any one of items 32, 33, 35 and 36, wherein the amino acid in said position corresponding to position X₁₁ in SEQ ID NO:7 is E.
39. The polypeptide according to any one of items 32-37, wherein the amino acid in said position corresponding to position X₉ in SEQ ID NO:7 is A.
40. The polypeptide according to any one of items 32-38, wherein the amino acid in said position corresponding to position X₉ in SEQ ID NO:7 is Q.
41. The polypeptide according to any one of items 32-40, wherein
   the amino acid in said position corresponding to position X₉ in SEQ ID NO:7 is Q and
   the amino acid in said position corresponding to position X₁₁ in SEQ ID NO:7 is E; or
   the amino acid in said position corresponding to position X₉ in SEQ ID NO:7 is A and
   the amino acid in said position corresponding to position X₁₁ in SEQ ID NO:7 is N.
41. The polypeptide according to any one of items 32-41, wherein the amino acids in the positions corresponding to X₉X₁₁ in SEQ ID NO:7 are not AE.
42. The polypeptide according to any one of items 1-41, wherein the polypeptide comprises an amino acid selected from E and H in a position in the amino acid sequence thereof, which position corresponds to position X₁₅ in SEQ ID NO:7.
43. The polypeptide according to any one of items 11-42, wherein the amino acids in the positions corresponding to X₁₈X₂₄X₂₈ in SEQ ID NO:7 are selected from the group consisting of RGD, KDG, KDT, RES, KQT, KHG, RDK, KHT, RHS, RHH and RHG; such as the group consisting of KDG, KDT, RES, KQT, KHG, RDK, KHT, RHS, RHH and RHG; such as the group consisting of KDT, RES, KQT, KHG, RDK, KHT, RHS, RHH and RHG; such as the group consisting of KQT, KHG, RDK, KHT, RHS, RHH and RHG; such as the group consisting of KHG, RDK, KHT, RHS, RHH and RHG; such as the group consisting of RDK, KHT, RHS, RHH and RHG; such as the group consisting of KHT, RHS, RHH and RHG; such as the group consisting of RHS, RHH and RHG; such as the group consisting of RHH and RHG.
44. The polypeptide according to any one of items 11-43, wherein the amino acids in the positions corresponding to X₁₈X₂₄X₂₈ in SEQ ID NO:7 are RHH.
45. The polypeptide according to any one of items 28-44, wherein the amino acids in the positions corresponding to X₃₃X₄₀X₅₁ in SEQ ID NO:7 are SGV.
46. The polypeptide according to any one of items 32-45, wherein the amino acids in the positions corresponding to X₉X₁₁ in SEQ ID NO:7 are selected from the group consisting of AE, QN, AN, QH, QR and QE; such as the group consisting of QN, AN, QH, QR and QE; such as the group consisting of AN, QH, QR and QE; such as the group consisting of QH, QR and QE; such as the group consisting of QR and QE.
47. The polypeptide according to any one of items 32-46, wherein the amino acids in the positions corresponding to X₉X₁₁ in SEQ ID NO:7 are QE.
48. A polypeptide comprising an amino acid sequence comprising Sequence A, which Sequence A consists of an amino acid sequence selected from i), ii) and iii), wherein i), ii) and iii) are defined as follows:
   i) X₁₈LPNLTX₂₄EQRX₂₈ (SEQ ID NO:12);
   ii) an amino acid sequence which has at least 70% identity to a sequence defined by i);
   iii) an amino acid sequence which has at least 70% identity to any sequence selected from the group consisting of: residues 18-28 in SEQ ID NO:6, residues 18-28 in SEQ ID NO:9, residues 18-28 in SEQ ID NO:8, residues 18-28 in SEQ ID NO:1, residues 18-28 in SEQ ID NO:2, residues 18-28 in SEQ ID NO:3, residues 21-31 in SEQ ID NO:4 and residues 16-26 in SEQ ID NO:5;
   wherein additionally, in each of i), ii) and iii), independently from each other,
   X₁₈ is selected from R and K,
   X₂₄ is selected from H, D, Q and E, such as from H and D, and
   X₂₈ is selected from H, T, K, S and G, such as from H, K and G, such as from H and G.
49. The polypeptide according to item 48, wherein X₁₈ is R.
50. The polypeptide according to item 48 or 49, wherein X₂₄ is H.
51. The polypeptide according to any one of items 48-50, wherein X₂₈ is H.
52. The polypeptide according to any one of items 48-51, wherein X₁₈X₂₄X₂₈ is selected from the group consisting of RGD, KDG, KDT, RES, KQT, KHG, RDK, KHT, RHS, RHH and RHG; such as the group consisting of KDG, KDT, RES, KQT, KHG, RDK, KHT, RHS, RHH and RHG; such as the group consisting of KDT, RES, KQT, KHG, RDK, KHT, RHS, RHH and RHG; such as the group consisting of KQT, KHG, RDK, KHT, RHS, RHH and RHG; such as the group consisting of KHG, RDK, KHT, RHS, RHH and RHG; such as the group consisting of RDK, KHT, RHS, RHH and RHG; such as the group consisting of KHT, RHS, RHH and RHG; such as the group consisting of RHS, RHH and RHG; such as the group consisting of RHH and RHG.
53. The polypeptide according to any one of items 48-52, wherein X₁₈X₂₄X₂₈ is RHH.
54. The polypeptide according to any one of items 48-53, wherein the amino acid sequence in i) corresponds to the region from position X₁₈ to position X₂₈ in an amino acid sequence selected from the group consisting of SEQ ID NO:16-33, such as the amino acid sequence according to SEQ ID NO:31.
55. The polypeptide according to any one of items 1-47, wherein the polypeptide comprises Sequence A as defined in any one of items 48-54.
56. The polypeptide according to any one of items 48-55, wherein the polypeptide comprises
   an amino acid selected from T, S, G, Q, A, E, H, R, P, D, K and N, such as from S, A and E, in a position in the amino acid sequence thereof, which position corresponds to position X₃₃ in SEQ ID NO:7;
   an amino acid selected from V, E, G, R, D, K, Q, N, H and S, such as from E, G, R, D, K, Q, N, H and S, such as from G, R, E and D, in a position in the amino acid sequence thereof, which position corresponds to position X₄₀ in SEQ ID NO:7; and
   an amino acid selected from L, V, S, I, R and G, such as from V, L, I and R, in a position in the amino acid sequence thereof, which position corresponds to position X₅₁ in SEQ ID NO:7.
57. The polypeptide according to item 56, wherein the amino acid in said position corresponding to position X₃₃ in SEQ ID NO:7 is S.
58. The polypeptide according to item 56 or 57, wherein the amino acid in said position corresponding to position X₄₀ in SEQ ID NO:7 is G.
59. The polypeptide according to any one of items 56-58, wherein the amino acid in said position corresponding to position X₅₁ in SEQ ID NO:7 is V.
60. The polypeptide according to any one of items 56-59, wherein X₃₃X₄₀X₅₁ is SGV.
61. The polypeptide according to any one of items 48-60, wherein the amino acid sequence of said polypeptide comprises Sequence B, which Sequence B consists of an amino acid sequence selected from iv), v) and vi), wherein iv), v) and vi) are defined as follows:
   iv) X₁₈LPNLTX₂₄EQRX₂₈X₂₉FIQX₃₃LX₃₅DDPSX₄₀SX₄₂X₄₃X₄₄LX₄₆EAX₄₉KX₅₁ (SEQ ID NO:13),
      wherein, independently from each other,
      X₂₉ is selected from A and G,
      X₃₅ is selected from R and K,
      X₄₂ is selected from K, A and T, such as from K and A,
      X₄₃ is selected from N, A and E, such as from N and A,
      X₄₄ is selected from I, L and V, such as from I and L,
      X₄₆ is selected from A and G, and
      X₄₉ is selected from K and Q;
   v) an amino acid sequence which has at least 70% identity to a sequence defined by iv);
   vi) an amino acid sequence which has at least 70% identity to any sequence selected from the group consisting of: residues 18-51 in SEQ ID NO:6, residues 18-51 in SEQ ID NO:9, residues 18-51 in SEQ ID NO:8, residues 18-51 in SEQ ID NO:1, residues 18-51 in SEQ ID NO:2, residues 18-51 in SEQ ID NO:3, residues 21-54 in SEQ ID NO:4 and residues 16-49 in SEQ ID NO:5;
   wherein additionally, in each of iv), v) and vi), independently from each other,
   X₁₈ is selected from R and K,
   X₂₄ is selected from H, D, Q and E, such as from H and D,
   X₂₈ is selected from H, T, K, S and G, such as from H, K and G, such as from H and G,
   X₃₃ is selected from T, S, G, Q, A, E, H, R, P, D, K and N, such as from S, A and E,
   X₄₀ is selected from V, E, G, R, D, K, Q, N, H and S, such as from E, G, R, D, K, Q, N, H and S, such as from G, R, E and D, and
   X₅₁ is selected from L, V, S, I, R and G, such as from V, L, I and R.
62. The polypeptide according to item 61, wherein X₁₈ is R.
63. The polypeptide according to item 61 or 62, wherein X₂₄ is H.
64. The polypeptide according to any one of items 61-63, wherein X₂₈ is H.
65. The polypeptide according to any one of items 61-64, wherein X₃₃ is S.
66. The polypeptide according to any one of items 61-65, wherein X₄₀ is G.
67. The polypeptide according to any one of items 61-66, wherein X₅₁ is V.
68. The polypeptide according to any one of items 61-67, wherein X₂₉ is A.
69. The polypeptide according to any one of items 61-68, wherein X₃₅ is K.
70. The polypeptide according to any one of items 61-69, wherein X₄₂ is K.
71. The polypeptide according to any one of items 61-70, wherein X₄₃ is A.
72. The polypeptide according to any one of items 61-71, wherein X₄₄ is I.
73. The polypeptide according to any one of items 61-72, wherein X₄₆ is A.
74. The polypeptide according to any one of items 61-73, wherein X₄₉ is K.
75. The polypeptide according to any one of items 61-74, wherein X₁₈X₂₄X₂₈ is selected from the group consisting of RGD, KDG, KDT, RES, KQT, KHG, RDK, KHT, RHS, RHH and RHG; such as the group consisting of KDG, KDT, RES, KQT, KHG, RDK, KHT, RHS, RHH and RHG; such as the group consisting of KDT, RES, KQT, KHG, RDK, KHT, RHS, RHH and RHG; such as the group consisting of KQT, KHG, RDK, KHT, RHS, RHH and RHG; such as the group consisting of KHG, RDK, KHT, RHS, RHH and RHG; such as the group consisting of RDK, KHT, RHS, RHH and RHG; such as the group consisting of KHT, RHS, RHH and RHG; such as the group consisting of RHS, RHH and RHG; such as the group consisting of RHH and RHG.
76. The polypeptide according to any one of items 61-75, wherein X₁₈X₂₄X₂₈ is RHH.
77. The polypeptide according to any one of items 61-76, wherein X₃₃X₄₀X₅₁ is SGV.
78. The polypeptide according to any one of items 61-77, wherein the amino acid sequence in iv) corresponds to the region from position X₁₈ to position X₅₁ in an amino acid sequence selected from the group consisting of SEQ ID NO:16-33, such as the amino acid sequence according to SEQ ID NO:31.
79. The polypeptide according to any one of items 1-47 and 55, wherein the polypeptide comprises Sequence B as defined in any one of items 61-78.
80. The polypeptide according to any one of items 48-79, wherein the polypeptide comprises
   an amino acid selected from E, N, H, R and S, such as from N, E, H and R, in a position in the amino acid sequence thereof, which position corresponds to position X₁₁ in SEQ ID NO:7; and/or
   an amino acid selected from A and Q in a position in the amino acid sequence thereof, which position corresponds to position X₉ in SEQ ID NO:7;
   preferably with the proviso that the amino acids in the positions corresponding to X₉X₁₁ in SEQ ID NO:7 are not AE.
81. The polypeptide according to item 80, wherein the amino acid in said position corresponding to position X₁₁ in SEQ ID NO:7 is selected from H, R and E, such as from Rand E.
82. The polypeptide according to item 80, wherein the amino acid in said position corresponding to position X₁₁ in SEQ ID NO:7 is selected from H, R and N, such as from N and H.
83. The polypeptide according to item 80, wherein the amino acid in said position corresponding to position X₁₁ in SEQ ID NO:7 is selected from N, Rand E, such as from Rand E.
84. The polypeptide according to item 80 or 83, wherein the amino acid in said position corresponding to position X₁₁ in SEQ ID NO:7 is selected from N and E.
85. The polypeptide according to any one of items 80 and 82-84, wherein the amino acid in said position corresponding to position X₁₁ in SEQ ID NO:7 is N.
86. The polypeptide according to any one of items 80, 81, 83 and 84, wherein the amino acid in said position corresponding to position X₁₁ in SEQ ID NO:7 is E.
87. The polypeptide according to any one of items 80-85, wherein the amino acid in said position corresponding to position X₉ in SEQ ID NO:7 is A.
88. The polypeptide according to any one of items 80-86, wherein the amino acid in said position corresponding to position X₉ in SEQ ID NO:7 is Q.
89. The polypeptide according to any one of items 80-88, wherein
   the amino acid in said position corresponding to position X₉ in SEQ ID NO:7 is Q and
   the amino acid in said position corresponding to position X₁₁ in SEQ ID NO:7 is E; or
   the amino acid in said position corresponding to position X₉ in SEQ ID NO:7 is A and
   the amino acid in said position corresponding to position X₁₁ in SEQ ID NO:7 is N.
90. The polypeptide according to any one of items 80-89, wherein the amino acids in the positions corresponding to X₉X₁₁ in SEQ ID NO:7 are not AE.
91. The polypeptide according to any one of items 80-90, wherein the amino acids in the positions corresponding to X₉X₁₁ in SEQ ID NO:7 are selected from the group consisting of AE, QN, AN, QH, QR and QE; such as the group consisting of QN, AN, QH, QR and QE; such as the group consisting of AN, QH, QR and QE; such as the group consisting of QH, QR and QE; such as the group consisting of QR and QE.
92. The polypeptide according to any one of items 80-91, wherein the amino acids in the positions corresponding to X₉X₁₁ in SEQ ID NO:7 are QE.
93. The polypeptide according to any one of items 48-92, wherein the polypeptide comprises an amino acid selected from E and H in a position in the amino acid sequence thereof, which position corresponds to position X₁₅ in SEQ ID NO:7.
94. The polypeptide according to any one of items 48-93, wherein the amino acid sequence of said polypeptide comprises Sequence C, which Sequence C consists of an amino acid sequence selected from vii), viii) and ix), wherein vii), viii) and ix) are defined as follows:
   vii) X₉QX₁₁AFYX₁₅X₁₆LX₁₈LPNLTX₂₄EQRX₂₈ (SEQ ID NO:14),
      wherein, independently from each other,
      X₁₆ is selected from I and V;
   viii) an amino acid sequence which has at least 70% identity to a sequence defined by vii);
   ix) an amino acid sequence which has at least 70% identity to any sequence selected from the group consisting of: residues 9-28 in SEQ ID NO:6, residues 9-28 in SEQ ID NO:9, residues 9-28 in SEQ ID NO:8, residues 9-28 in SEQ ID NO:1, residues 9-28 in SEQ ID NO:2, residues 9-28 in SEQ ID NO:3, residues 12-31 in SEQ ID NO:4 and residues 7-26 in SEQ ID NO:5;
   wherein additionally, in each of vii), viii) and ix), independently from each other,
   X₉ is selected from A and Q,
   X₁₁ is selected from E, N, H, R and S, such as from E, N, H and R;
   X₁₅ is selected from E, Q and H, such as from E and H,
   X₁₈ is selected from R and K,
   X₂₄ is selected from H, D, Q and E, such as from H and D, and
   X₂₈ is selected from H, T, K, S and G, such as from H, K and G, such as from H and G,
   preferably with the proviso that X₉X₁₁ is not AE.
95. The polypeptide according to item 94, wherein X₁₁ is selected from H, R and E, such as from Rand E.
96. The polypeptide according to item 94, wherein X₁₁ is selected from H, R and N, such as from N and H.
97. The polypeptide according to item 94, wherein X₁₁ is selected from N, Rand E, such as from Rand E.
98. The polypeptide according to item 94 or 97, wherein X₁₁ is selected from N and E.
99. The polypeptide according to any one of items 94 and 96-98, wherein X₁₁ is N.
100. The polypeptide according to any one of items 94, 95, 97 and 98, wherein X₁₁ is E.
101. The polypeptide according to any one of items 94-99, wherein X₉ is A.
102. The polypeptide according to any one of items 94-100, wherein X₉ is Q.
103. The polypeptide according to any one of items 94-102, wherein X₉ is Q and X₁₁ is E; or X₉ is A and X₁₁ is N.
104. The polypeptide according to any one of items 94-103, wherein X₉X₁₁ is not AE.
105. The polypeptide according to any one of items 94-104, wherein X₁₅ is E.
106. The polypeptide according to any one of items 94-105, wherein X₁₆ is I.
107. The polypeptide according to any one of items 94-106, wherein X₁₈ is R.
108. The polypeptide according to any one of items 94-107, wherein X₂₄ is H.
109. The polypeptide according to any one of items 94-108, wherein X₂₈ is H.
110. The polypeptide according to any one of items 94-109, wherein X₉X₁₁ is selected from the group consisting of AE, QN, AN, QH, QR and QE; such as the group consisting of QN, AN, QH, QR and QE; such as the group consisting of AN, QH, QR and QE; such as the group consisting of QH, QR and QE; such as the group consisting of QR and QE.
111. The polypeptide according to any one of items 94-110, wherein X₉X₁₁ is QE.
112. The polypeptide according to any one of items 94-111, wherein X₁₈X₂₄X₂₈ is selected from the group consisting of RGD, KDG, KDT, RES, KQT, KHG, RDK, KHT, RHS, RHH and RHG; such as the group consisting of KDG, KDT, RES, KQT, KHG, RDK, KHT, RHS, RHH and RHG; such as the group consisting of KDT, RES, KQT, KHG, RDK, KHT, RHS, RHH and RHG; such as the group consisting of KQT, KHG, RDK, KHT, RHS, RHH and RHG; such as the group consisting of KHG, RDK, KHT, RHS, RHH and RHG; such as the group consisting of RDK, KHT, RHS, RHH and RHG; such as the group consisting of KHT, RHS, RHH and RHG; such as the group consisting of RHS, RHH and RHG; such as the group consisting of RHH and RHG.
113. The polypeptide according to any one of items 94-112, wherein X₁₈X₂₄X₂₈ is RHH.
114. The polypeptide according to any one of items 94-113, wherein the amino acid sequence in vii) corresponds to the region from position X₉ to position X₂₈ in an amino acid sequence selected from the group consisting of SEQ ID NO:16-33, such as the amino acid sequence according to SEQ ID NO:31.
115. The polypeptide according to any one of items 1-47, 55 and 79, wherein the polypeptide comprises Sequence C as defined in any one of items 94-114.
116. The polypeptide according to any one of items 94-115, wherein the polypeptide comprises
   an amino acid selected from T, S, G, Q, A, E, H, R, P, D, K and N, such as from S, A and E, in a position in the amino acid sequence thereof, which position corresponds to position X₃₃ in SEQ ID NO:7;
   an amino acid selected from V, E, G, R, D, K, Q, N, H and S, such as from E, G, R, D, K, Q, N, H and S, such as from G, R, E and D, in a position in the amino acid sequence thereof, which position corresponds to position X₄₀ in SEQ ID NO:7; and
   an amino acid selected from L, V, S, I, R and G, such as from V, L, I and R, in a position in the amino acid sequence thereof, which position corresponds to position X₅₁ in SEQ ID NO:7.
117. The polypeptide according to item 116, wherein the amino acid in said position corresponding to position X₃₃ in SEQ ID NO:7 is S.
118. The polypeptide according to item 116 or 117, wherein the amino acid in said position corresponding to position X₄₀ in SEQ ID NO:7 is G.
119. The polypeptide according to any one of items 116-118, wherein the amino acid in said position corresponding to position X₅₁ in SEQ ID NO:7 is V.
120. The polypeptide according to any one of items 116-119, wherein the amino acids in the positions corresponding to X₃₃X₄₀X₅₁ in SEQ ID NO:7 are SGV.
121. The polypeptide according to any one of items 48-120, wherein the amino acid sequence of said polypeptide comprises Sequence D, which Sequence D consists of an amino acid sequence selected from x), xi) and xii), wherein x), xi) and xii) are defined as follows:
   x) X₉QX₁₁AFYX₁₅X₁₆LX₁₈LPNLTX₂₄EQRX₂₈X₂₉FIQX₃₃LX₃₅DDPSX₄₀SX₄₂X₄₃X₄₄LX₄₆ EAX₄₉KX₅₁ (SEQ ID NO:15),
      wherein, independently from each other,
      X₁₆ is selected from I and V;
      X₂₉ is selected from A and G,
      X₃₅ is selected from R and K,
      X₄₂ is selected from K, A and T, such as from K and A,
      X₄₃ is selected from N, A and E, such as from N and A,
      X₄₄ is selected from I, L and V, such as from I and L,
      X₄₆ is selected from A and G, and
      X₄₉ is selected from K and Q;
   xi) an amino acid sequence which has at least 70% identity to a sequence defined by x);
   xii) an amino acid sequence which has at least 70% identity to any sequence selected from the group consisting of: residues 9-51 in SEQ ID NO:6, residues 9-51 in SEQ ID NO:9, residues 9-51 in SEQ ID NO:8, residues 9-51 in SEQ ID NO:1, residues 9-51 in SEQ ID NO:2, residues 9-51 in SEQ ID NO:3, residues 12-54 in SEQ ID NO:4 and residues 7-49 in SEQ ID NO:5;
   wherein additionally, in each of iv), v) and vi), independently from each other, X₉ is selected from A and Q,
      X₁₁ is selected from E, N, H, R and S, such as from E, N, H and R;
      X₁₅ is selected from E, Q and H, such as from E and H,
      X₁₈ is selected from R and K,
      X₂₄ is selected from H, D, Q and E, such as from H and D,
      X₂₈ is selected from H, T, K, S and G, such as from H, K and G, such as from H and G,
      X₃₃ is selected from T, S, G, Q, A, E, H, R, P, D, K and N, such as from S, A and E,
      X₄₀ is selected from V, E, G, R, D, K, Q, N, H and S, such as from E, G, R, D, K, Q, N, H and S, such as from G, R, E and D, and
      X₅₁ is selected from L, V, S, I, R and G, such as from V, L, I and R,
      preferably with the proviso that X₉X₁₁ is not AE.
122. The polypeptide according to item 121, wherein X₁₁ is selected from H, R and E, such as from Rand E.
123. The polypeptide according to item 121, wherein X₁₁ is selected from H, R and N, such as from N and H.
124. The polypeptide according to item 121, wherein X₁₁ is selected from N, Rand E, such as from Rand E.
125. The polypeptide according to item 121 or 124, wherein X₁₁ is selected from N and E.
126. The polypeptide according to any one of items 121 and 123-125, wherein X₁₁ is N.
127. The polypeptide according to any one of items 121, 122, 124 and 125, wherein X₁₁ is E.
128. The polypeptide according to any one of items 121-126, wherein X₉ is A.
129. The polypeptide according to any one of items 121-127, wherein X₉ is Q.
130. The polypeptide according to any one of items 121-129, wherein X₉ is Q and X₁₁ is E; or X₉ is A and X₁₁ is N.
131. The polypeptide according to any one of items 121-130, wherein X₉X₁₁ is not AE.
132. The polypeptide according to any one of items 121-131, wherein X₁₅ is E.
133. The polypeptide according to any one of items 121-132, wherein X₁₆ is I.
134. The polypeptide according to any one of items 121-133, wherein X₁₈ is R.
135. The polypeptide according to any one of items 121-134, wherein X₂₄ is H.
136. The polypeptide according to any one of items 121-135, wherein X₂₈ is H.
137. The polypeptide according to any one of items 121-136, wherein X₃₃ is S.
138. The polypeptide according to any one of items 121-137, wherein X₄₀ is G.
139. The polypeptide according to any one of items 121-138, wherein X₅₁ is V.
140. The polypeptide according to any one of items 121-139, wherein X₂₉ is A.
141. The polypeptide according to any one of items 121-140, wherein X₃₅ is K.
142. The polypeptide according to any one of items 121-141, wherein X₄₂ is K.
143. The polypeptide according to any one of items 121-142, wherein X₄₃ is A.
144. The polypeptide according to any one of items 121-143, wherein X₄₄ is I.
145. The polypeptide according to any one of items 121-144, wherein X₄₆ is A.
146. The polypeptide according to any one of items 121-145, wherein X₄₉ is K.
147. The polypeptide according to any one of items 121-146, wherein the amino acid sequence in x) corresponds to the region from position X₉ to position X₅₁ in an amino acid sequence selected from the group consisting of SEQ ID NO:16-33, such as the amino acid sequence according to SEQ ID NO:31.
148. The polypeptide according to any one of items 1-47, 55, 79 and 115, wherein the polypeptide comprises Sequence D as defined in any one of items 121-147.
149. The polypeptide according to any one of items 48-148, wherein said polypeptide comprises an amino acid sequence that has at least 70% identity to a sequence selected from the group consisting of SEQ ID NO:1-9, such as the group consisting of SEQ ID NO:1-8.
150. The polypeptide according to item 149, wherein for the purpose of determining said sequence identity, positions in the amino acid sequence corresponding to positions X₁₈, X₂₄, X₂₈; positions X₁₈, X₂₄, X₂₈, X₃₃, X₄₀, X₅₁; positions X₉, X₁₁, X₁₈, X₂₄, X₂₈; positions X₉, X₁₁, X₁₅, X₁₈, X₂₄, X₂₈; positions X₉, X₁₁, X₁₈, X₂₄, X₂₈, X₃₃, X₄₀, X₅₁, and/or positions X₉, X₁₁, X₁₅, X₁₈, X₂₄, X₂₈, X₃₃, X₄₀, X₅₁; in SEQ ID NO:7 are omitted.
151. The polypeptide according to any one of items 48-150, wherein the amino acid sequence of said polypeptide comprises an amino acid sequence selected from the group consisting of SEQ ID NO:16-33, such as the amino acid sequence according to SEQ ID NO:31.
152. The polypeptide according to any one of items 48-151, wherein said polypeptide has binding affinity to an Fc region of an immunoglobulin at pH 7.0, which binding affinity for said Fc region is essentially lost at and below pH 4.4, or at and below pH 4.5, or at and below pH 4.6, or at and below pH 4.7, or at and below pH 4.8, or at and below pH 4.9, or at and below pH 5.0, or at and below pH 5.5, or at and below pH 6.0.
153. The polypeptide according item 152, wherein the loss of said binding affinity for the Fc region is induced by electrostatic repulsion.
154. The polypeptide according item 153, wherein the electrostatic repulsion is mediated by at least one amino acid selected from the group consisting of amino acids in positions in the amino acid sequence of the polypeptide corresponding to positions X₁₈, X₂₄ and X₂₈ in SEQ ID NO:7.
155. The polypeptide according to any one of items 48-154, wherein said polypeptide has a higher binding affinity than SEQ ID NO:10 for adalimumab.
156. The polypeptide according to any one of items 152-155, wherein said immunoglobulin is immunoglobulin G (IgG), such as IgG selected from the group consisting of IgG1, IgG2 and IgG4.
157. The polypeptide according item 156, wherein said IgG is IgG1, such as trastuzumab and/or adalimumab.
158. The polypeptide according to any one of items 48-157, wherein said polypeptide has lower binding affinity for a VH3 region of trastuzumab compared to the binding affinity of SEQ ID NO:6 and/or SEQ ID NO:7 for the same VH3 region.
159. The polypeptide according item 158, wherein said binding affinity for the VH3 region of trastuzumab is measured at pH 7.0.
160. A polypeptide multimer, wherein each monomer of the multimer comprises a polypeptide which is independently selected from any polypeptide defined in any one of items 1-159.
161. The polypeptide multimer according to item 160, wherein said multimer is selected from the group consisting of dimer, trimer, tetramer, pentamer, hexamer, heptamer, octamer, nonamer and decamer.
162. The polypeptide multimer according to item 160 or 161, wherein said multimer is selected from the group consisting of tetramer and hexamer.
163. The polypeptide multimer according to any one of items 160-162, wherein said multimer is a hexamer.
164. The polypeptide multimer according to any one of items 160-163, wherein said multimer is a homomer.
165. The polypeptide multimer according to any one of items 160-164, wherein said polypeptide multimer has binding affinity to an Fc region of an immunoglobulin at pH 7.0, which binding affinity for said Fc region is essentially lost at and below pH 4.4 or at and below pH 4.5, or at and below pH 4.6, or at and below pH 4.7, or at and below pH 4.8, or at and below pH 4.9, or at and below pH 5.0, or at and below pH 5.5, or at and below pH 6.0.
166. The polypeptide multimer according item 165, wherein the loss of said binding affinity for the Fc region is induced by electrostatic repulsion.
167. The polypeptide multimer according item 166, wherein the electrostatic repulsion is mediated by at least one amino acid selected from the group consisting of amino acids in positions in the amino acid sequences of the monomers of the polypeptide multimer corresponding to positions X₁₈, X₂₄ and X₂₈ in SEQ ID NO:7.
167. The polypeptide multimer according to any one of items 165-167, wherein said immunoglobulin is immunoglobulin G (IgG), such as IgG selected from the group consisting of IgG1, IgG2 and IgG4.
168. The polypeptide multimer according item 167, wherein said IgG is IgG1, such as trastuzumab and/or adalimumab.
169. The polypeptide multimer according to any one of items 160-168, wherein the polypeptide multimer exhibits at least 40%, such as at least 45%, such as at least 50%, such as at least 55%, such as at least 60%, such as at least 65%, such as at least 70%, such as at least 75%, residual binding capacity after about 100 cleaning cycles with 0.5 M NaOH.
170. The polypeptide multimer according to any one of items 160-169, wherein said multimer comprises an amino acid sequence selected from the group consisting of SEQ ID NO:45-49; such as the group consisting of SEQ ID NO:45-47 and 49; such as the group consisting of SEQ ID NO:45, 47 and 49; such as the group consisting of SEQ ID NO:45 and 49. In one embodiment, the multimer comprises the amino acid sequence according to SEQ ID NO:45.
171. The polypeptide multimer according to any one of items 160-170, further comprising at least one linker.
172. The polypeptide multimer according to any one of items 160-171, wherein said linker comprises up to 15 amino acid residues.
173. The polypeptide according to any one of items 1-159 or the polypeptide multimer according to any one of items 160-172, which comprises additional amino acids at the C-terminal and/or N-terminal end thereof.
174. The polypeptide or the polypeptide multimer according to item 173, wherein said additional amino acid(s) improve(s) production, purification, stabilization and/or *in vitro* coupling of the polypeptide or the polypeptide multimer.
175. The polypeptide or the polypeptide multimer according to item 174, wherein said additional amino acid(s) improve(s) coupling of the polypeptide or the polypeptide multimer and are selected from the group consisting of one or more cysteine residues, a plurality of Lysine residues and a plurality of Histidine residues.
176. A polynucleotide encoding the polypeptide as defined in any one of items 1-159 and 173-175, or the polypeptide multimer as defined in any one of items 160-175.
177. An expression vector comprising the polynucleotide as defined in item 176.
178. A host cell comprising the expression vector as defined in item 177.
179. An adsorbent material comprising the polypeptide as defined in any one of items 1-159 and 173-175, or the polypeptide multimer as defined in any one of items 160-175, coupled to a solid support.
180. The adsorbent material according to item 179, wherein the solid support is selected from a particle, a bead, a fiber, a fibrous membrane, a filter, a sheet, a porous monolith, a chip, a plate, and a well.
181. The adsorbent material according to item 179 or 180, wherein the solid support is a separation matrix.
182. A method of isolating an immunoglobulin or a fragment thereof, said method comprising
   a) contacting a liquid sample comprising said immunoglobulin or a fragment thereof with the adsorbent material as defined in any one of items 179-181.
183. The method according to item 182, wherein said immunoglobulin is immunoglobulin G (IgG), such as IgG selected from the group consisting of IgG1, IgG2 and IgG4.
184. The method according to item 183, wherein said IgG is IgG1.
185. The method according to any one of items 182-184, wherein said method comprises eluting the immunoglobulin or the fragment thereof from the adsorbent material with an elution buffer.
186. The method according to item 185, wherein said elution buffer has a pH within a pH range of pH 4.5-5.5, such as a pH range of pH 4.6-5.5, such as a pH range of pH 4.6-5.0, such as a pH range of pH 4.8-5.0.

## Claims

1. A polypeptide derived from a Staphylococcus Protein A (SpA) or any domain thereof, wherein said polypeptide has binding affinity to an Fc region of an immunoglobulin, such as immunoglobulin G, at pH 7.0, which binding affinity for said Fc region is essentially lost at and below pH 4.4, or at and below pH 4.5, or at and below pH 4.6, or at and below pH 4.7, or at and below pH 4.8, or at and below pH 4.9, or at and below pH 5.0, or at and below pH 5.5, or at and below pH 6.0; and wherein said polypeptide has a higher binding affinity than SEQ ID NO:10 for adalimumab.

2. The polypeptide according to claim 1, wherein the polypeptide comprises an amino acid selected from R and K in a position in the amino acid sequence thereof, which position corresponds to position X₁₈ in SEQ ID NO:7;
an amino acid selected from H, D, Q and E, such as from H and D, in a position in the amino acid sequence thereof, which position corresponds to position X₂₄ in SEQ ID NO:7; and/or
an amino acid selected from H, T, K, S and G, such as from H, K and G, such as from H and G, in a position in the amino acid sequence thereof, which position corresponds to position X₂₈ in SEQ ID NO:7.

3. A polypeptide derived from a Staphylococcus Protein A (SpA) or any domain thereof comprising an amino acid sequence, wherein said polypeptide comprises an amino acid selected from R and K in a position in the amino acid sequence thereof, which position corresponds to position X₁₈ in SEQ ID NO:7;
an amino acid selected from H, D, Q and E, such as from H and D, in a position in said amino acid sequence, which position corresponds to position X₂₄ in SEQ ID NO:7; and
an amino acid selected from H, T, K, S and G, such as from H, K and G, such as from H and G, in a position in said amino acid sequence, which position corresponds to position X₂₈ in SEQ ID NO:7.

4. A polypeptide comprising an amino acid sequence comprising Sequence A, which Sequence A consists of an amino acid sequence selected from i), ii) and iii), wherein i), ii) and iii) are defined as follows:
i) X₁₈LPNLTX₂₄EQRX₂₈ (SEQ ID NO:12);
ii) an amino acid sequence which has at least 70% identity to a sequence defined by i);
iii) an amino acid sequence which has at least 70% identity to any sequence selected from the group consisting of: residues 18-28 in SEQ ID NO:6, residues 18-28 in SEQ ID NO:9, residues 18-28 in SEQ ID NO:8, residues 18-28 in SEQ ID NO:1, residues 18-28 in SEQ ID NO:2, residues 18-28 in SEQ ID NO:3, residues 21-31 in SEQ ID NO:4 and residues 16-26 in SEQ ID NO:5;
wherein additionally, in each of i), ii) and iii), independently from each other,
X₁₈ is selected from R and K,
X₂₄ is selected from H, D, Q and E, such as from H and D, and
X₂₈ is selected from H, T, K, S and G, such as from H, K and G, such as from H and G.

5. The polypeptide according to claim 4, wherein the amino acid sequence in i) corresponds to the region from position X₁₈ to position X₂₈ in an amino acid sequence selected from the group consisting of SEQ ID NO:16-33, such as the amino acid sequence according to SEQ ID NO:31.

6. The polypeptide according to any one claims 1-5, wherein said polypeptide comprises an amino acid sequence that has at least 70% identity to a sequence selected from the group consisting of SEQ ID NO:1-9, such as the group consisting of SEQ ID NO:1-8.

7. The polypeptide according to any one of claims 3-6, wherein the polypeptide comprises
an amino acid selected from T, S, G, Q, A, E, H, R, P, D, K and N, such as from S, A and E, in a position in the amino acid sequence thereof, which position corresponds to position X₃₃ in SEQ ID NO:7;
an amino acid selected from V, E, G, R, D, K, Q, N, H and S, such as from E, G, R, D, K, Q, N, H and S, such as from G, R, E and D, in a position in the amino acid sequence thereof, which position corresponds to position X₄₀ in SEQ ID NO:7; and
an amino acid selected from L, V, S, I, R and G, such as from V, L, I and R, in a position in the amino acid sequence thereof, which position corresponds to position X₅₁ in SEQ ID NO:7.

8. The polypeptide according to any one of claims 4-7, wherein the amino acid sequence of said polypeptide comprises Sequence B, which Sequence B consists of an amino acid sequence selected from iv), v) and vi), wherein iv), v) and vi) are defined as follows:
iv) X₁₈LPNLTX₂₄EQRX₂₈X₂₉FIQX₃₃LX₃₅DDPSX₄₀SX₄₂X₄₃X₄₄LX₄₆EAX₄₉KX₅₁ (SEQ ID NO:13),
wherein, independently from each other,
X₂₉ is selected from A and G,
X₃₅ is selected from R and K,
X₄₂ is selected from K, A and T, such as from K and A,
X₄₃ is selected from N, A and E, such as from N and A,
X₄₄ is selected from I, L and V, such as from I and L,
X₄₆ is selected from A and G, and
X₄₉ is selected from K and Q;
v) an amino acid sequence which has at least 70% identity to a sequence defined by iv);
vi) an amino acid sequence which has at least 70% identity to any sequence selected from the group consisting of: residues 18-51 in SEQ ID NO:6, residues 18-51 in SEQ ID NO:9, residues 18-51 in SEQ ID NO:8, residues 18-51 in SEQ ID NO:1, residues 18-51 in SEQ ID NO:2, residues 18-51 in SEQ ID NO:3, residues 21-54 in SEQ ID NO:4 and residues 16-49 in SEQ ID NO:5;
wherein additionally, in each of iv), v) and vi), independently from each other,
X₁₈ is selected from R and K,
X₂₄ is selected from H, D, Q and E, such as from H and D,
X₂₈ is selected from H, T, K, S and G, such as from H, K and G, such as from H and G,
X₃₃ is selected from T, S, G, Q, A, E, H, R, P, D, K and N, such as from S, A and E,
X₄₀ is selected from V, E, G, R, D, K, Q, N, H and S, such as from E, G, R, D, K, Q, N, H and S, such as from G, R, E and D, and
X₅₁ is selected from L, V, S, I, R and G, such as from V, L, I and R.

9. The polypeptide according to claim 8, wherein the amino acid sequence in iv) corresponds to the region from position X₁₈ to position X₅₁ in an amino acid sequence selected from the group consisting of SEQ ID NO:16-33, such as the amino acid sequence according to SEQ ID NO:31.

10. The polypeptide according to any one of claims 3-9, wherein the polypeptide comprises
an amino acid selected from E, N, H, R and S, such as from N, E, H and R, in a position in the amino acid sequence thereof, which position corresponds to position X₁₁ in SEQ ID NO:7; and/or
an amino acid selected from A and Q in a position in the amino acid sequence thereof, which position corresponds to position X₉ in SEQ ID NO:7;
preferably with the proviso that the amino acids in the positions corresponding to X₉X₁₁ in SEQ ID NO:7 are not AE.

11. The polypeptide according to any one of claims 4-10, wherein the amino acid sequence of said polypeptide comprises Sequence C, which Sequence C consists of an amino acid sequence selected from vii), viii) and ix), wherein vii), viii) and ix) are defined as follows:
vii) X₉QX₁₁AFYX₁₅X₁₆LX₁₈LPNLTX₂₄EQRX₂₈ (SEQ ID NO:14),
wherein, independently from each other,
X₁₆ is selected from I and V;
viii) an amino acid sequence which has at least 70% identity to a sequence defined by vii);
ix) an amino acid sequence which has at least 70% identity to any sequence selected from the group consisting of: residues 9-28 in SEQ ID NO:6, residues 9-28 in SEQ ID NO:9, residues 9-28 in SEQ ID NO:8, residues 9-28 in SEQ ID NO:1, residues 9-28 in SEQ ID NO:2, residues 9-28 in SEQ ID NO:3, residues 12-31 in SEQ ID NO:4 and residues 7-26 in SEQ ID NO:5;
wherein additionally, in each of vii), viii) and ix), independently from each other,
X₉ is selected from A and Q,
X₁₁ is selected from E, N, H, R and S, such as from E, N, H and R;
X₁₅ is selected from E, Q and H, such as from E and H,
X₁₈ is selected from R and K,
X₂₄ is selected from H, D, Q and E, such as from H and D, and
X₂₈ is selected from H, T, K, S and G, such as from H, K and G, such as from H and G,
preferably with the proviso that X₉X₁₁ is not AE.

12. The polypeptide according to claim 11, wherein the amino acid sequence in vii) corresponds to the region from position X₉ to position X₂₈ in an amino acid sequence selected from the group consisting of SEQ ID NO:16-33, such as the amino acid sequence according to SEQ ID NO:31.

13. The polypeptide according to claim 11 or 12, wherein the polypeptide comprises an amino acid selected from T, S, G, Q, A, E, H, R, P, D, K and N, such as from S, A and E, in a position in the amino acid sequence thereof, which position corresponds to position X₃₃ in SEQ ID NO:7;
an amino acid selected from V, E, G, R, D, K, Q, N, H and S, such as from E, G, R, D, K, Q, N, H and S, such as from G, R, E and D, in a position in the amino acid sequence thereof, which position corresponds to position X₄₀ in SEQ ID NO:7; and
an amino acid selected from L, V, S, I, R and G, such as from V, L, I and R, in a position in the amino acid sequence thereof, which position corresponds to position X₅₁ in SEQ ID NO:7.

14. The polypeptide according to any one of claims 4-13, wherein the amino acid sequence of said polypeptide comprises Sequence D, which Sequence D consists of an amino acid sequence selected from x), xi) and xii), wherein x), xi) and xii) are defined as follows:
x) X₉QX₁₁AFYX₁₅X₁₆LX₁₈LPNLTX₂₄EQRX₂₈X₂₉FIQX₃₃LX₃₅DDPSX₄₀SX₄₂X₄₃X₄₄LX₄₆ EAX₄₉KX₅₁ (SEQ ID NO:15),
wherein, independently from each other,
X₁₆ is selected from I and V;
X₂₉ is selected from A and G,
X₃₅ is selected from R and K,
X₄₂ is selected from K, A and T, such as from K and A,
X₄₃ is selected from N, A and E, such as from N and A,
X₄₄ is selected from I, L and V, such as from I and L,
X₄₆ is selected from A and G, and
X₄₉ is selected from K and Q;
xi) an amino acid sequence which has at least 70% identity to a sequence defined by x);
xii) an amino acid sequence which has at least 70% identity to any sequence selected from the group consisting of: residues 9-51 in SEQ ID NO:6, residues 9-51 in SEQ ID NO:9, residues 9-51 in SEQ ID NO:8, residues 9-51 in SEQ ID NO:1, residues 9-51 in SEQ ID NO:2, residues 9-51 in SEQ ID NO:3, residues 12-54 in SEQ ID NO:4 and residues 7-49 in SEQ ID NO:5;
wherein additionally, in each of iv), v) and vi), independently from each other,
X₉ is selected from A and Q,
X₁₁ is selected from E, N, H, R and S, such as from E, N, H and R;
X₁₅ is selected from E, Q and H, such as from E and H,
X₁₈ is selected from R and K,
X₂₄ is selected from H, D, Q and E, such as from H and D,
X₂₈ is selected from H, T, K, S and G, such as from H, K and G, such as from H and G,
X₃₃ is selected from T, S, G, Q, A, E, H, R, P, D, K and N, such as from S, A and E,
X₄₀ is selected from V, E, G, R, D, K, Q, N, H and S, such as from E, G, R, D, K, Q, N, H and S, such as from G, R, E and D, and
X₅₁ is selected from L, V, S, I, R and G, such as from V, L, I and R, preferably with the proviso that X₉X₁₁ is not AE.

15. The polypeptide according to claim 14, wherein the amino acid sequence in x) corresponds to the region from position X₉ to position X₅₁ in an amino acid sequence selected from the group consisting of SEQ ID NO:16-33, such as the amino acid sequence according to SEQ ID NO:31.

16. The polypeptide according to any one of claims 3-15, wherein said polypeptide has binding affinity to an Fc region of an immunoglobulin at pH 7.0, which binding affinity for said Fc region is essentially lost at and below pH 4.4, or at and below pH 4.5, or at and below pH 4.6, or at and below pH 4.7, or at and below pH 4.8, or at and below pH 4.9, or at and below pH 5.0, or at and below pH 5.5, or at and below pH 6.0.

17. The polypeptide according to any one of claims 3-16, wherein said polypeptide has a higher binding affinity than SEQ ID NO:10 for adalimumab.

18. The polypeptide according to any one of claims 1-17, wherein said polypeptide has lower binding affinity for a VH3 region of trastuzumab compared to the binding affinity of SEQ ID NO:6 and/or SEQ ID NO:7 for the same VH3 region.

19. A polypeptide multimer, wherein each monomer of the multimer comprises a polypeptide which is independently selected from any polypeptide defined in any one of claims 1-18.

20. An adsorbent material comprising the polypeptide as defined in any one of claims 1-18, or the polypeptide multimer as defined in claim 19.

21. A method of isolating an immunoglobulin or a fragment thereof, said method comprising
a) contacting a liquid sample comprising said immunoglobulin or a fragment thereof with the adsorbent material as defined in claim 20.
